# EUROPÄISCHE PATENTSCHRIFT

(11) **EP 0 986 545 B1**
(45) Veröffentlichungstag und Bekanntmachung des Hinweises auf die Patenterteilung: **29.12.2004**
(21) Anmeldenummer: 98928338.7
(22) Anmeldetag: 02.06.1998
(51) Int. Cl.: C07D 249/18, A61K 31/41, C07D 271/12, C07D 285/10, C07C 235/26, A61K 31/16, C07D 307/88, A61K 31/365, C07D 333/72, A61K 31/38, C07D 311/76, C07D 265/02, A61K 31/535, C07D 237/32, A61K 31/50

(54) **NICHTSTEROIDALE (HETERO)ZYKLISCH-SUBSTITUIERTE ACYLANILIDE MIT GEMISCHTER GESTAGENER UND ANDROGENER WIRKSAMKEIT**
NON-STEROIDAL (HETERO) CYCLICALLY SUBSTITUTED ACYLANILIDES WITH MIXED GESTAGEN AND ANDROGEN ACTIVITY
ACYLANILIDES NON STEROIDIQUES HETEROCYCLIQUEMENT SUBSTITUES A EFFET MIXTE GESTAGENE ET ANDROGENE

(30) Priorität: 30.05.1997 DE 19723722
(43) Veröffentlichungstag der Anmeldung: 22.03.2000
(73) Patentinhaber: SCHERING AKTIENGESELLSCHAFT, 13353 Berlin (DE)
(72) Erfinder: LEHMANN, Manfred, D-12305 Berlin (DE); SCHÖLLKOPF, Klaus, D-14129 Berlin (DE); STREHLKE, Peter, D-10629 Berlin (DE); HEINRICH, Nikolaus, D-12159 Berlin (DE); FRITZEMEIER, Karl-Heinrich, D-13467 Berlin (DE); MUHN, Hans-Peter, D-13465 Berlin (DE); KRATTENMACHER, Rolf, D-13467 Berlin (DE)
(86) Internationale Anmeldenummer: PCT/EP1998/003242
(87) Internationale Veröffentlichungsnummer: WO 1998/054159

(56) Entgegenhaltungen:
- EP-A- 0 002 892
- EP-A- 0 040 932
- EP-A- 0 173 516
- EP-A- 0 253 500
- EP-A- 0 253 503

## Beschreibung

Die vorliegende Erfindung betrifft nichtsteroidale Verbindungen, die eine hohe gestagene Aktivität aufweisen.

Neben einer großen Zahl von Steroidverbindungen mit gestagener Wirkung sind auch Gestagene bekannt, die keine Steroide sind (beispielsweise aus der EP 0 253 500 B1 und der WO 94/01412, vgl. J. Med. Chem. 38 (1995) 4878).

Die vorliegende Erfindung beschreibt die Verbindungen der allgemeinen Formel I 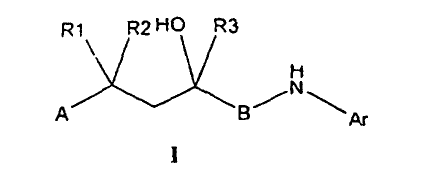 worin
- R¹ und R²: gleich oder verschieden sind und für ein Wasserstoffatom, eine C₁-C₅-Alkylgruppe oder ein Halogenatom. ferner gemeinsam mit dem C-Atom der Kette für einen Ring mit insgesamt 3-7 Gliedern,
- R³: für eine C₁-C₅ Alkylgruppe oder eine teilweise oder vollständig fluorierte C₁-C₅ Alkylgruppe,
- A: für einen gegebenenfalls durch einen oder mehrere Reste, ausgewählt aus Halogenatomen, C₁-C₅-Alkylgruppen, C₂-C₅-Alkenylgruppen -CR⁵=CR⁶R⁷, wobei R⁵, R6 und R⁷ gleich oder verschieden sind und unabhängig voneinander Wasserstoffatome oder C₁-C₅ Alkylgruppen bedeuten, Hydroxygruppen, Hydroxygruppen, die eine C₁-C₁₀-Acylgruppe, eine C₃-C₁₀-Carbalkoxyalkylguppe, eine C₂-C₅-Cyanalkylgruppe, eine C₃-C₁₀ unsubstituierte oder substituierte Allylgruppe, eine C₃-C₁₀ unsubstituierte oder substituierte Propargylgruppe, eine C₂-C₅-Alkoxyalkylgruppe. eine teilweise oder vollständig durch Fluoratome substituierte C₁-C₅-Alkyloruppe tragen, der Cyan- oder Nitrogruppe, C₁-C₅-Alkoxygruppen, C₁-C₅-Alkylthiogruppen. mono- oder disubstituierten C₁-C₁₀ Aminogruppen oder teilweise oder vollständig fluorierten C₁-C₅ Alkylgruppen substituierten mono- oder bicyclischen carbocyclischen oder heterocyclischen aromatischen Ring,
- B: für eine Carbonyl- oder eine CH₂-Gruppe und
- Ar: für ein Ringsystem, ausgewählt aus der Gruppe der allgemeinen Teilformeln **2** - **11**, stehen, 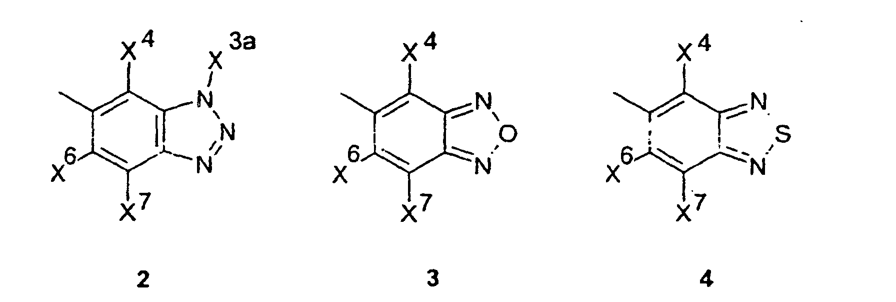 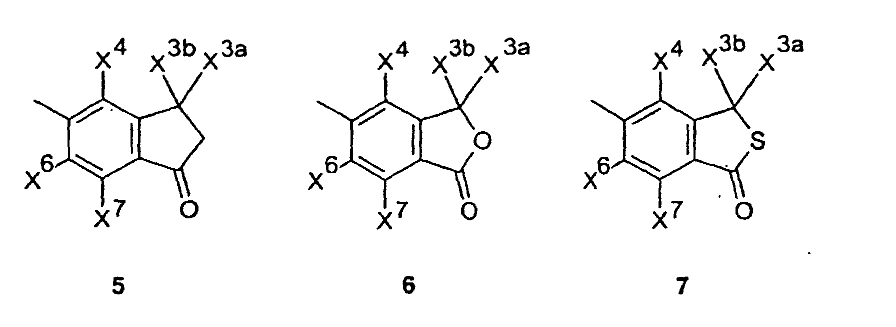 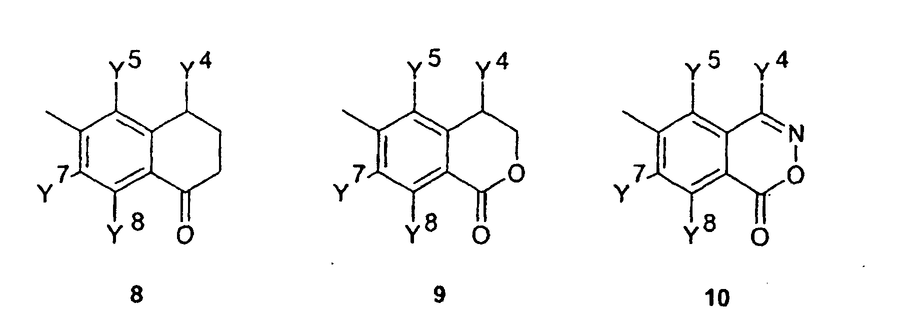 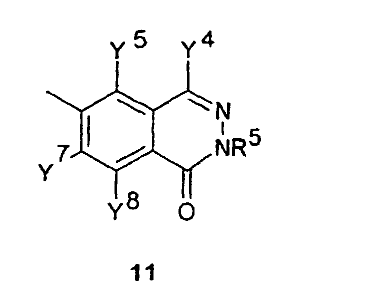 worin
die Reste X^{3a}, X⁴, X⁶, X⁷ (in der Teilformel **2**), X⁴, X⁶, X⁷ (in den Teilformeln **3** und **4**), X^{3a}, X^{3b}, X⁴, X⁶, X⁷ (in den Teilformeln **5**, **6** und **7**) oder Y⁴, Y⁵, Y⁷, Y⁸ (in den Teilformeln **8**, **9**, **10** und **11**) gleich oder verschieden und ausgewählt sind aus Wasserstoffatomen, C₁-C₅-Alkylgruppen, die zusätzlich noch eine gegebenenfalls mit einer C₁-C₅-Alkylgruppe veretherte oder mit einer C₁-C₅-Alkanoylgruppe veresterte Hydroxygruppe enthalten können, teilweise oder vollständig fluorierten C₁-C₅ Alkylgruppen, C₂-C₅-Alkenylgruppen - CR⁵=CR⁶R⁷, wobei R⁵, R⁶ und R⁷ die oben genannte Bedeutung haben, Alkinylgruppen - C≡CR⁵, wobei R⁵ die oben genannte Bedeutung hat,
die Reste X^{3a} und X^{3b} ferner gemeinsam mit dem C-Atom des benzokondensierten Ringsystems **5**, **6** oder **7** einen Ring mit insgesamt 3-7 Gliedern bilden können sowie darüber hinaus die Reste X⁴, X⁶, X⁷ (in den Teilformeln **2**, **3**, **4**, **5**, **6** und **7**) oder Y⁴, Y⁵, Y⁷, Y⁸ (in den Teilformeln **8**, **9**, **10** und **11**) ausgewählt sind aus Halogenatomen, Hydroxygruppen, C₁-C₅-Alkoxygruppen oder C₁-C₅-Alkanoyloxygruppen, sowie für den Fall,daß B für eine CH₂-Gruppe steht, die physiologisch verträglichen Salze der Verbindungen der allgemeinen Formel I mit Säuren.

Die erfindungsgemäßen Verbindungen unterscheiden sich von den bekannten nichtsteroidalen Verbindungen mit gestagener Wirksamkeit durch das Substitutionsmuster am in der allgemeinen Formel I rechts stehenden Arylrest. Bei den hier vorliegenden Verbindungen ist Ar ein benzokondensiertes, bicyclisches Ringsystem, während bei den aus der EP 0 253 500 B1 hervorgehenden, als nächstliegende Verbindungen anzusehenden Strukturen (Acylanilide) an dieser Stelle ein ein-, zwei- oder dreifach substituierter Phenylrest steht.

Die in EP0253503, EP0002892 und EP0040932 beschriebenen Acylanilide besitzen alle antiandrogene Wirksamkeit, die der EP0253503 vorzugsweise zusätzlich unter anderem gestagene Wirksamkeit. Ebenfalls Acylanilide sind in der EP0173516 beschrieben, die allerdings keine OH-Gruppe und keinen R3 vergleichbaren Substituenten aufweisen und über eine völlig andere Wirksamkeit (Leukotrienantagonisten) verfügen.

Die erfindungsgemäßen Verbindungen der allgemeinen Formel 1 können durch das Vorhandensein von Asymmetriezentren als unterschiedliche Stereoisomere vorliegen. Sowohl die Racemate wie auch die getrennt vorliegenden Stereoisimere gehören zum Gegenstand der vorliegenden Erfindung.

Die als Gruppen definierten Substituenten in den Verbindungen der allgemeinen Formel 1 können jeweils die nachfolgenden Bedeutungen haben.

Bei den C₁-C₅-Alkylgruppen kann es sich durchweg um eine Methyl-, Ethyl-, n-Propyl-, iso-Propyl-, n-, iso-, tert.-Butyl- oder um eine n-Pentyl-, 2,2-Dimethylpropyl- oder 3-Methylbutylgruppe handeln. Eine Methyl- oder Ethylgruppe ist bevorzugt.

Für ein Halogenatom kann ein Fluor-, Chlor-, Brom- oder Iodatom stehen. Bevorzugt ist hier Fluor, Chlor oder Brom.

Wenn R¹ und R² gemeinsam mit dem C-Atom der Kette einen 3-7 gliedrigen Ring bilden, so ist dies beispielsweise ein Cyclo-propyl-, -butyl-, -pentyl- oder -hexylring. Der Cyclopropylring ist bevorzugt.

Für eine teilweise oder vollständig fluorierte C₁-C₅-Alkylgruppe kommen die perfluorierten, oben stehenden Alkylgruppen, und von diesen vor allem die Trifluormethyl- oder Pentafluorethylgruppe sowie als teilweise fluorierte Alkylgruppen beispielsweise die 5,5.5,4,4-Pentafluorpentyl- oder 5,5,5,4,4,3,3-Heptafluorpentylgruppe in Betracht.

Als C₂-C₅-Alkenylgruppe kann beispielsweise eine Vinyl-, Allyl- oder 2,3-Dimethyl-2-propenylgruppe stehen; im Falle, daß der Aromat A mit einer Alkenylgruppe substituiert ist, steht hierfür vorzugsweise eine Vinylgruppe.

Für die C₂-C₅-Carbalkoxyalkylgruppe kann beispielsweise eine Carboxymethyl-, tert.-Butoxymethyl- oder Ethoxymethylgruppe stehen; die beiden zuerst genannten sind bevorzugt.

Als Vertreter für die C₂-C₅-Cyanalkylgruppe seien Cyanmethyl sowie 1- und 2-Cyanethyl genannt; Cyanmethyl ist bevorzugt.

Die C₃-C₁₀-Allylgruppe ist vorzugsweise eine unsubstituierte Allylgruppe; im Falle einer substituierten Allylgruppe seien beispielsweise 1-Methylallyl, 1,1-Dimethylallyl, 2-Methylallyl, 3-Methylallyl, 2,3-Dimethylallyl, 3,3-Dimethylallyl, Cinnamyl und 3-Cyciohexylallyl genannt.

Eine unsubstituierte Propargyl-, eine Methylpropargyl-, 3-Methylpropargyl-, 3-Phenylpropargyl- oder 3-Cyclohexylpropargylgruppe sind die exemplarischen Vertreter für eine C₃-C₁₀ Propargylgruppe; die unsubstituierte Propargylgruppe ist bevorzugt.

Für C₂-C₅-Alkoxyalkyl kann beispielsweise Methoxymethyl, Ethoxymethyl oder 2-Methoxyethyl stehen.

Vertreter für eine C₁-C₅-Alkoxygruppe sind ausgewählt aus Methoxy-, Ethoxy-, n-Propoxy-, iso-Propoxy-, n-, iso-, tert.-Butoxy- oder n-Pentoxy-, 2,2-Dimethylpropoxy- oder 3-Methylbutoxygruppen. Eine Methoxy- oder Ethoxygruppe ist bevorzugt.

C₁-C₅-Perfluoralkoxygruppen sind die entsprechenden perfluorierten Reste der vorstehenden C₁-C₅-Alkoxygruppen.

Der mono- oder bicyclische aromatische Ring A, der substituiert sein kann, ist ein carbocyclischer oder heterocyclischer Arylrest. Im ersten Fall handelt es sich beispielsweise um einen Phenyl- oder Naphthylrest, vorzugsweise um einen Phenylrest.
Als heterocyclischer Rest kann beispielsweise ein ein monocyclischer heterocyclischer Rest, beispielsweise der Thienyl-, Furyl-, Pyranyl-, Pyrrolyl-, Imidazolyl-, Pyrazolyl-, Pyridyl-, Pyrazinyl-, Pyrimidinyl-, Pyridazinyl-, Thiazolyl-, Oxazolyl-, Furazanyl-, Pyrrolinyl-, Imidazolinyl-, Pyrazolinyl-, Thiazolinyl-, Triazolyl-, Tetrazolylrest, und zwar alle möglichen Isomeren bezüglich der Positionen der Heteroatome. Der Thienylrest ist als Heteroarylrest A bevorzugt.

Als C₁-C₅-Alkylgruppe zur Veretherung von Hydroxygruppen kommen die genannten Alkylgruppen in Frage, in erster Linie eine Methyl- oder Ethylgruppe.
Als C₁-C₅-Alkanoylgruppe zur Veresterung von Hydroxygruppen kommt eine Formyl,- Acetyl-, Propionyl-, Butyryl-, iso-Butyryl-, Valeryl- oder iso-Valeryl- oder Pivaloylgruppe in Betracht, vorzugsweise eine Acetylgruppe.
Als C₁-C₁₀-Acylgruppe zur Veresterung von Hydroxygruppen seien beispielsweise die vorstehend genannten Alkanoylgruppen, vorzugsweise wiederum eine Acetylgruppe, oder eine Benzoyl-, Toluoyl-, Phenylacetyl-, Acryloyl-, Cinnamoyl- oder Cyclohexylcarbonylgruppe genannt.

Wenn X^{3a} und X^{3b} gemeinsam mit dem C-Atom des benzokondensierten Ringsystems einen 3-7 gliedrigen Ring bilder, so ist dies beispielsweise ein Cyclo-propyl-, -butyl-, -pentyl- oder - hexylring. Der Cyclopropylring ist bevorzugt.

Als C₁-C₅-Alkanoyloxygruppe für X⁴, X⁶, X⁷, Y⁴, Y⁵, Y⁷ oder Y⁸ kommt eine Formyloxy,-Acetoxy-, Propionyloxy-, Butyryloxy-, iso-Butyryloxy-, Valeryloxy- oder iso-Valeryloxygruppe in Betracht, vorzugsweise eine Acetoxygruppe.

Für C₁-C₅ Alkyl innnerhalb der C₁-C₅-Alkylthio-, C₁-C₅-Alkylsulfinyl- oder C₁-C₅-Alkylsulfonylgruppe können die oben erwähnten C₁-C₅ Alkylgruppen stehen.

Im Falle, daß die Verbindungen der allgemeinen Formel I (B = -CH₂-) als Salze vorliegen, kann dies beispielsweise in der Form des Hydrochlorids, Sulfats, Nitrats, Tartrats oder Benzoats sein.

Wenn die erfindungsgemäßen Verbindungen als racemische Gemische vorliegen, können sie nach dem Fachmann geläufigen Methoden der Racemattrennung in die reinen, optisch aktiven Formen aufgetrennt werden. Beispielsweise lassen sich die racemischen Gemische durch Chromatographie an einem selbst optisch aktiven Trägermaterial (CHIRALPAK AD®) in die reinen Isomere trennen. Es ist auch möglich, die freie Hydroxygruppe in einer racemischen Verbindung der allgemeinen Formel I mit einer optisch aktiven Säure zu verestern und die erhaltenen diastereoisomeren Ester durch fraktionierte Kristallisation oder chromatographisch zu trennen und die getrennten Ester jeweils zu den optisch reinen Isomeren zu verseifen. Als optisch aktive Säure kann beispielsweise Mandelsäure, Camphersulfonsäure oder Weinsäure verwendet werden.

Bevorzugt gemäß vorliegender Erfindung sind solche Verbindungen der allgemeinen Formel I, in denen:
- R¹ und R²: gleich oder verschieden sind und für ein Wasserstoffatom, eine Methyl- oder Ethylgruppe, ferner gemeinsam mit dem C-Atom der Kette für einen Cyclopropylring stehen, und/oder
- R³: für eine C₁-C₅ Perfluoralkylgruppe, und/oder
- A: für einen gegebenenfalls durch einen oder mehrere Reste, ausgewählt aus Fluoratornen, Chloratomen, Bromatomen, Methylgruppen, Ethylgruppen, Vinylguppen, Hydroxygruppen, Methoxygruppen, Ethoxygruppen substituierten Benzol-, Naphthalin- oder Thiophenring, und/oder entweder
- X^{3a}: für ein Wasserstoffatom oder eine C₁-C₅-Alkylgruppe, oder
- X^{3a} und X^{3b}: gleich oder verschieden sind und für ein Wasserstoffatom oder eine C₁-C₅-Alkylgruppe und/oder
- X⁴, X⁶ und X⁷: gleich oder verschieden sind und unabhängig voneinander für ein Wasserstoffatom oder ein Halogenatom, und/oder
- Y⁴: für eine C₁-C₅-Alkylgruppe oder eine C₁-C₅ Perfluoralkylgruppe, und/oder
- Y⁵, Y⁷ und Y⁸: gleich oder verschieden sind und unabhängig voneinander für ein Wasserstoffatom oder ein Halogenatom
stehen,
und die anderen Substituenten alle in der Formel 1 angegebenen Bedeutungen haben.

Desweiteren sind solche Verbindungen der allgemeinen Formel bevorzugt, in denen Ar für ein Ringsystem der Teilfonnel **6**, **7**, **10** oder **11** steht.

Die nachstehend genannten Verbindungen sind erfindungsgemäß insbesondere bevorzugt:
4-Brom-5-(2-hydroxy-4-methyl-4-phenyl-2-trifluormethyl-valeroylamino)-phthalid,
6-Brom-5-(2-hydroxy-4-methyl-4-phenyl-2-trifluormethyl-valeroylamino)-phthalid,
5-(2-Hydroxy-4-methyl-2-pentafluorethyl-4-phenyl-valeroylamino)-phthalid.
5-[2-Hydroxy-4-(3-methoxyphenyl)-4-methyl-2-trifluormethyl-valeroylamino]-phthalid.
5-[2-Hydroxy-4-(4-methoxyphenyl)-4-methyl-2-trifluormethyl-valeroylamino]-phthalid,
5-[2-Hydroxy-4-(2-hydroxyphenyl)-4-methyl-2-trifluormethyl-valeroylamino]-phthalid,
5-[4-(2-Fluorphenyl)-2-hydroxy-A-methyl-2-trifluormethyl-valeroylamino]-phthalid,
5-[4-(4-Fluorphenyl)-2-hydroxy-4-methyl-2-trifluormethyl-valeroylamino]-phthalid,
5-[4-(4-Chlorphenyl)-2-hydroxy-4-methyl-2-trifluormethyl-valeroylamino]-phthalid,
5-[4-(4-Bromphenyl)-2-hydroxy-4-methyl-2-trifluormethyl-valeroylamino]-phthalid,
5-[2-Hydroxy-4-methyl-4-(4-tolyl)-2-trifluormethyl-valeroylamino]-phthalid,
5-[2-Hydroxy-4-methyl-4-(3-tolyl)-2-trifluormethyl-valeroylamino]-phthalid,
5-[4-(4-Cyanphenyl)-2-hydroxy-4-methyl-2-trifluormethyl-valeroylamino]-phthalid,
5-[4-(3,4-Dimethylphenyl)-2-hydroxy-4-methyl-2-trifluormethyl-valeroylamino]-phthalid,
5-[4-(3,5-Dimethylphenyl)-2-hydroxy-4-methyl-2-trifluormethyl-valeroylamino]-phthalid,
5-[2-Hydroxy-4-(2-methoxy-5-methylyphenyl)-4-methyl-2-trifluormethyl-valeroylamino]-phthalid,
5-[4-(5-Chlor-2-hydroxyphenyl)-2-hydroxy-4-methyl-2-trifluormethyl-valeroylamino]-phthalid,
5-[4-(5-Fluor-2-hydroxyphenyl)-2-hydroxy-4-methyl-2-trifluormethyl-valeroylamino]-phthalid,
5-[2-Hydroxy-4-(2-Hydroxy-5-methylphenyl)-4-methyl-2-trifluormethyl-valeroylamino]-phthalid,
5-[4-(5-Fluor-2-methoxyphenyl)-2-hydroxy-4-methyl-2-trifluormethyl-valeroylamino]-phthalid,
5-[4-(2-Fluor-4-methoxyphenyl)-2-hydroxy-4-methyl-2-trifluormethyl-valeroylamino]-phthalid.
5-[4-(3-Fluor-4-methoxyphenyl)-2-hydroxy-4-methyl-2-trifluormethyl-valeroylamino]-phthalid.
5-[2-Hydroxy-4-phenyl-2-trifluormethyl-valeroylamino)-phthalid,
5-[2-Hydroxy-4-(2-methoxyphenyl)-4-methyl-2-trifluormethyl-valeroylamino]-phthalid,
5-[4-(5-Chlor-2-methoxyphenyl)-2-hydroxy-4-methyl-2-trifluormethyl-valeroylamino]-phthalid,
5-(2-Hydroxy-4-phenyl-2-trifluormethyl-pentylamino)-phthalid,
5-(2-Hydroxy-4-methyl-4-phenyl-2-trifluormethyl-pentylamino)-phthalid,
5-[4-(4-Fluorphenyl)-2-hydroxy-4-methyl-2-trifluormethyl-pentylamino]-phthalid,
5-[4-(5-Fluor-2-hydroxyphenyl)-2-hydroxy-4-methyl-2-trifluormethyl-pentylamino]-phthalid,
6-Acetyl-5-(2-hydroxy-4-methyl-4-phenyl-2-trifluormethyl-valeroylamino)-phthalid,
5-[4-(3-Fluor-4-hydroxyphenyl)-2-hydroxy-4-methyl-2-trifluormethyl-valeroylamino]-phthalid,
5-[4-(3-Fluorphenyl)-2-hydroxy-4-methyl-2-trifluormethyl-valeroylamino]-phthalid,
6-(3-Hydroxy-3-methyl-1-butinyl)-5-(2-hydroxy-4-methyl-4-phenyl-2-trifluormethylvaleroylamino)-phthalid,
6-(2-Hydroxy-4-methyl-4-phenyl-2-trifluormethyl-valeroylamino)-4-methyl-2,3-benzoxazin-1-on,
6-(2-Hydroxy-4-methyl-4-phenyl-2-trifluormethyl-valeroylamino)-4-trifluormethyl-2,3-benzoxazin-1-on,
4-Ethyl-6-(2-hydroxy-4-phenyl-2-trifluormethyl-pentylamino)-2,3-benzoxazin-1-on,
4-Ethyl-6-[2-hydroxy-4-(2-methoxyphenyl)-4-methyl-2-trifluormethyl-valeroylamino]-2,3-benzoxazin-1-on,
6-[2-Hydroxy-4-(2-methoxyphenyl)-4-methyl-2-trifluormethyl-valeroylamino]-4-methyl-2,3-benzoxazin-1-on,
4-Ethyl-6-[2-hydroxy-4-methyl-4-(4-methylphenyl)-2-trifluormethyl-valeroylamino]-2,3-benzoxazin-1-on.
6-[4-(4-Bromphenyl)-2-hydroxy-4-methyl-2-trifluormethyl-valeroylamino]-4-ethyl-2,3-benzoxazin-1-on,
4-Ethyl-6-[4-(5-fluor-2-methoxyphenyl)-2-hydroxy-4-methyl-2-trifluormethyl-valeroylamino]-2,3-benzoxazin-1-on,
6-[4-(5-Fluor-2-methoxyphenyl)-2-hydroxy-4-methyl-2-trifluotmethyl-valeroylamino]-4-methyl-2,3-benzoxazin-1-on,
1-(4-Nitro-3-trifluormethylanilino)-4-phenyl-2-trifluormethyl-2-pentanol,
5-[2-Hydroxy-4,4-dimethyl-2-trifluormethyl-5-hexenoylamino)-phthalid,
5-[2-Hydroxy-3-(1-phenyl-cyclopropyl)-2-trifluormethyl-propionylamino]-phthalid,
5-[2-Hydroxy-3-(1-phenyl-cyclobutyl)-2-trifluormethyl-propionylamino]-phthalid,
5-[2-Hydroxy-3-(1-phenyl-cyclohexyl)-2-trifluormethyl-propionyiamino]-phthalid,
6-(2-Hydroxy-2,4-dimethyl4-phenyl-valeroylamino)-4-methyl-2,3-benzoxazin-1-on,
5-[4-(3-Chlor-4-methoxyphenyl)-2-hydroxy-4-methyl-2-trifluormethyl-valeroylamino]-phthalid.

Desweiteren sind die aus den Tabellen 7 bis 15 hervorgehenden Verbindungen bevorzugt.

Alle genannten Verbindungen sind in Form der optischen Antipoden oder der getrennten Diastereomere besonders bevorzugt.

Im Gestagenrezeptor-Bindungstest auf gestagene Wirkung unter Verwendung von Cytosol aus Kaninchenuterushomogenat und von ³H-Progesteron als Bezugssubstanz zeigen die neuen Verbindungen eine starke bis sehr starke Affinität zum Gestagenrezeptor (siehe Tab. 1).

Zusätzlich zu ihrer gestagenen Wirksamkeit, die je nach betrachteter Verbindung der allgemeinen Formel I unterschiedlich stark ausgeprägt ist, zeichnen sich die neuen Verbindungen auch durch eine mehr oder minder stark ausgeprägte Affinität zum Androgenrezeptor aus. Der Androgenrezeptor-Bindungstest auf androgene Wirkung wurde unter Verwendung von Cytosol aus Rattenprostatahomogenat und von ³H-Methyltrienolon als Bezugssubstanz durchgeführt.

Die neuen Verbindungen stellen sich somit gegenüber den gestagenen Verbindungen aus der EP 0 253 500 B1 als Verbindungen mit einem ganz neuartigen Mischprofil, welches aus gestagener und androgener Wirkung zusammengesetzt ist, dar.

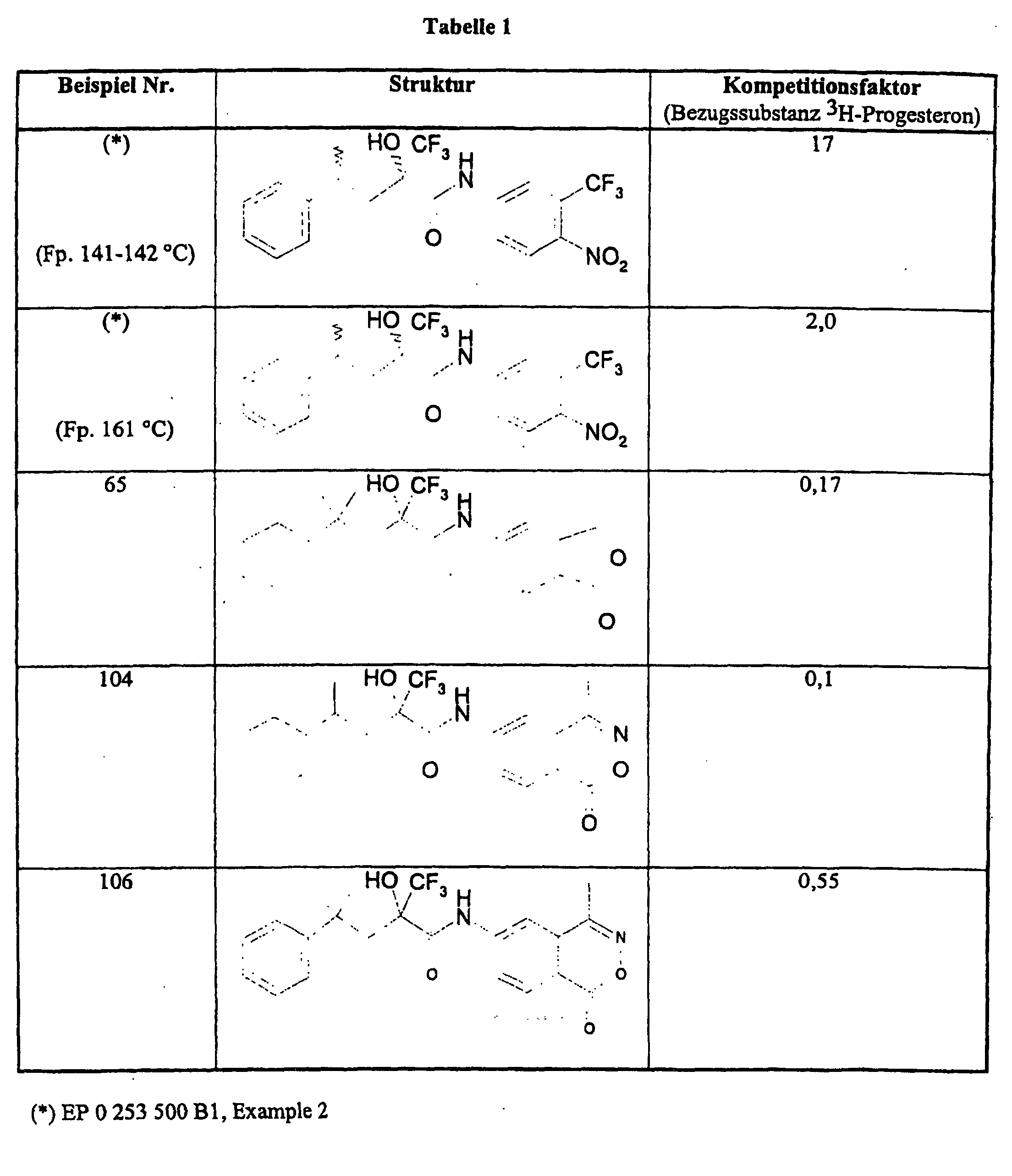

1) Verbindungen mit stärkerer gestagener und weniger ausgeprägter androgener Wirkung (KF_{Prog} <1 und KF_{Andro} >5);
2) Verbindungen mit stärkerer androgener und weniger ausgeprägter gestagener Wirkung (KF_{Andro} <5 und KF_{Prog} >1);
3) Verbindungen mit ausgeprägter gestagener und ausgeprägter androgener Wirkung (KF_{Prog} <1 und KF_{Andro} <5).

Je nach ihrer Klassifizierung nach 1), 2) oder 3) können die neuen Verbindungen erfindungsgemäß für unterschiedliche medizinische oder pharmazeutische Zwecke verwendet werden.

Bei den unter 1) klassifizierten Verbindungen mit stärkerer gestagener und weniger ausgeprägter androgener Wirkung handelt es sich um sehr wirksame Gestagene, die wie die bereits zahlreich bekannten gestagenen Verbindungen zur Erhaltung von Schwangerschaften bei parenteraler wie auch bei oraler Applikation geeignet sind.
In Kombination mit einem Estrogen sind Kombinationspräparate erhältlich, die für die Kontrazeption und und für die Behandlung klimakterischer Beschwerden eingesetzt werden können.

Aufgrund ihrer hohen gestagenen Wirksamkeit können die neuen, unter 1) klassifizierten Verbindungen der allgemeinen Formel I beispielsweise allein oder in Kombination mit Estrogenen in Präparaten zur Kontrazeption verwendet werden. Aber auch alle anderen heutzutage für Gestagene bekannten Verwendungsmöglichkeiten stehen diesen neuen Verbindungen offen (siehe z.B. "Kontrazeption mit Hormonen", Hans-Dieter Taubert und Herbert Kuhl, Georg Thieme Verlag Stuttgart - New York, 1995).

Geeignete Dosierungen können routinemäßig bestimmt werden, z. B. durch Bestimmung der Bioequivalenz, beispielsweise. im Schwangerschaftserhaltungstest, gegenüber einem bekannten Gestagen für eine bestimmte Verwendung, beispielsweise eine Menge, die bioequivalent zu 30 bis 150 µg Levonorgestrel für die Kontrazeption ist.

Die Dosierung der erfindungsgemäßen Verbindungen unter 1) in Kontrazeptionspräparaten soll vorzugsweise 0,01 bis 2 mg pro Tag betragen.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene kommen alle natürlichen und synthetischen, als estrogen wirksame bekannten Verbindungen in Frage.

Als natürliche Estrogene sind dies insbesondere Estradiol sowie auch dessen länger wirkende Ester wie das Valerat etc. oder Estriol.

Vorzugsweise sind jedoch synthetische Estrogene wie Ethinylestradiol, 14α,17α-Ethano-1,3,5(10)-estratrien-3,17β-diol (WO 88/01275), 14α,17α-Ethano-1,3,5(10)-estratrien-3,16α,17β-triol (WO 91/08219) oder die 15,15-Dialkyl-Derivate des Estradiols, und hiervon insbesondere das 15,15-Dimethylestradiol (WO 95/04070), zu nennen. Als synthetisches Estrogen ist Ethinylestradiol bevorzugt.
Auch die kürzlich bekannt gewordenen Estratrien-3-amidosulfonate (WO 96/05216 und WO 96/05217), abgeleitet vom Estradiol oder Ethinylestradiol, die sich durch geringe hapatische Estrogenität auszeichnen, sind als Estrogene zur gemeinsamen Verwendung mit den unter 1) klassifizierten Verbindungen der allgemeinen Formel I geeignet.
Schließlich seien noch die 14α,15α-Methylensteroide aus der Estranreihe, insbesondere das 14α,15α-Methylen-17α-estradiol sowie die entsprechenden 3-Amidosulfonat-Derivate erwähnt.

Das Estrogen wird in einer Menge verabfolgt, die der von 0,01 bis 0,05 mg Ethinylestradiol entspricht.

Die neuen. unter 1) klassifizierten Verbindungen der allgemeinen Formel I können auch in Präparaten zur Behandlung gynäkologischer Störungen und zur Substitutionstherapie eingesetzt werden. Wegen ihres günstigen Wirkungsprofils sind diese erfindungsgemäßen Verbindungen besonders gut geeignet zur Behandlung prämenstrueller Beschwerden, wie Kopfschmerzen, depressive Verstimmung, Wasserretention und Mastodynie. Die Tagesdosis bei der Behandlung prämenstrueller Beschwerden liegt bei etwa 1 bis 20 mg.

Analog wie bereits für andere Gestagene bekannt, können die neuen Verbindungen auch zur Behandlung der Endometriose dienen.

Schließlich können diese neuen Verbindungen auch als gestagene Komponente in den neuerdings bekannt gewordenen Zusammensetzungen für die weibliche hertilitätskontrolle, die sich durch die zusätzliche Verwendung eines kompetitiven Progesteronantagonisten auszeichnen, zum Einsatz kommen (H.B. Croxatto und A.M. Salvatierra in Female Contraception and Male Fertility Regulation, ed. by Runnebaum, Rabe & Kiesel - Vol. 2, Advances in Gynecological and Obstetric Research Series, Parthenon Publishing Group - 1991, Seite 245; WO 93/17686, WO 93/21927, US-Pat. 5,521,166).
Die Dosierung liegt im bereits angegebenen Bereich, die Formulierung kann wie bei konventionellen OC-Präparaten erfolgen. Die Applikation des zusätzlichen, kompetitiven Progesteronantagonisten kann dabei auch sequentiell vorgenommen werden.

Diejenigen Verbindungen der allgemeinen Formel, die wie obenstehend unter 2) oder 3) einzuordnen sind, d. h. also Verbindungen, die in jedem Fall über eine starke androgene Wirkung verfügen (androgene Gestagene), können zur Herstellung pharmazeutischer Präparate für die männliche Fertilitätskontrolle verwendet werden.

Gegenwärtig wird in mehreren WHO-Studien die kontrazeptive Wirksamkeit einer Kombination aus einem oral applizierten Gestagen (Depot-Medroxyprogesteronacetat, Levonorgestrel-Ester, Cyproteronacetat) mit einem parenteral verabreichten Androgen (Testosteronönanthat) an Männern geprüft.
Im Gegensatz hierzu ist mit den vorliegenden Verbindungen die Fertilitätskontrolle beim Mann in einer, und zwar oral oder transdermal zu verabreichenden, Dosierungsform möglich.

Außerdem können die erfindungsgemäßen Verbindungen mit androgener Wirkung beim älteren Mann zur männlichen HRT (Hormone Replacement Therapy) verwendet werden.

Diejenigen Verbindungen der allgemeinen Formel I, die sich eher unter 2) klassifizieren lassen, d.h. Verbindungen mit überwiegend androgener und schwächerer gestagener Wirkung, können für die männliche Hormontherapie verwendet werden. Mit ihnen lassen sich Präparate zur Behandlung eines Hypergonadismus oder zur Behandlung der männlichen Infertilität und von Potenzstörungen herstellen.

Für die männliche Fertilitätskontrolle und zur Behandlung der vorstehend genannten androgenen Krankheitsbilder werden die erfindungsgemäßen Verbindungen in Dosierungen eingesetzt, die wirkequivalent zu den in den WHO-Studien verwendeten Testosteronönanthatmengen bzw. zur Dosierung bereits in der Androgentherapie eingesetzter Verbindungen sind.

Wirkequivalente Mengen sind solche Mengen, die im Test auf androgene Wirkung an der Samenblase und/oder Prostata der Ratte (Hershberger-Test) vergleichbare Wirkung erzielen.
Für die HRT beim Mann wird bisher eine Substitutionsdosis von ungefähr 10 mg/Tag Testosteronönanthat verwendet.

Für die von der WHO durchgeführten Fertilitätskontrollstudien beim Mann werden verschiedene Testosteronester (Önanthat, Bucyclat, Undecanoat) im Bereich von ungefähr 10 - 30 mg/Tag eingesetzt.

Es ist an dieser Stelle darauf hinzuweisen, daß die Übergänge zwischen 1), 2) und 3) was die erfindungsgemäße Zuordnung der unterschiedlichen Indikationen zu diesen unterschiedlichen Mischprofilen 1), 2) und 3) anbelangt, fließend sind. Diejenigen Verbindungen, die aufgrund ihres KF_{Prog} und/oder Kf_{Andro} eher am Rande der angegebenen KF-Bereiche liegen, können ohne weiteres auch für die dem benachbarten Mischprofil zugeordneten Indikationen zum Einsatz kommen.

Die Verbindungen der allgemeinen Formel 1 zeigen teilweise auch Wirkungen am Glucocorticoid- und/oder Mineralocorticoidrezeptor.

Die Formulierung der pharmazeutischen Präparate auf Basis der neuen Verbindungen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.

Für die bevorzugte orale Applikation kommen insbesondere Tabletten, Dragees, Kapseln, Pillen, Suspensionen oder Lösungen in Frage.

Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Die Verbindungen der allgemeinen Formel I können auch kontinuierlich durch ein intrauterines Freisetzungssystem.(IntraUterineSystem = IUS; z.B. MIRENA® ) appliziert werden; die Freisetzungsrate der aktiven Verbindung(en) wird dabei so gewählt, daß die täglich freigesetzte Dosis innerhalb der bereits angegebenen Dosierungsbereiche liegt.
Es ist auch möglich, die erfindungsgemäßen Substanzen in ein transdermales System einzuarbeiten und sie damit transdermal zu applizieren.

Die erfindungsgemäßen Verbindungen der allgemeinen Formel I lassen sich wie nachstehend beschrieben herstellen:

### Herstellungsverfahren

1. Eine Carbonylverbindung der allgemeinen Formel II 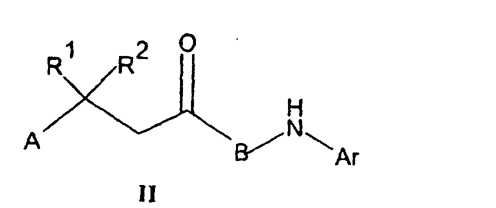 worin A, B, Ar, R¹ und R² die in Formel I angegebene Bedeutung haben, wird mit einer Verbindung der allgemeinen Formel R³-SiMe₃, worin R³ die in der allgemeinen Formel I angegebene Bedeutung hat, in Gegenwart eines Katalysators oder mit einer Alkylmetallverbindung, beispielsweise einem Grignard-Reagenz oder einem Lithiumalkyl, zu einer Verbindung der Formel I umgesetzt. Als Katalysator kommen Fluorid-Salze oder basische Verbindungen wie Alkalicarbonate infrage (J. Amer Chem. Soc.**111**, 393 (1989)).
2. Eine Verbindung der allgemeinen Formel III 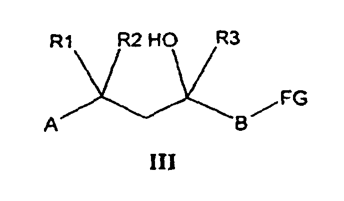 worin A,B, R¹,R² und R³ die in Formel I angegebene Bedeutung haben und FG eine Fluchtgruppe bedeutet, wird mit einer Verbindung Ar-NH-R¹¹ , wobei R¹¹ ein Wasserstoffatom oder eine C₁-C₅ Acylgruppe bedeutet und Ar die in der allgemeinen Formel I angegebene Bedeutung hat, umgesetzt, wobei gegebenenfalls anschließend der Rest R¹¹ abgespalten wird, um zu einer Verbindung der Formel I zu gelangen. Die Verbindung der allgemeinen Formel III kann dabei gegebenenfalls nur als Zwischenprodukt gebildet werden, z.B. kann es sich um ein intermediär aus einer entsprechenden Carbonsäure gebildetes Säurechlorid handeln. Als Fluchtgruppen seien beispielsweise ein Chlor- oder Bromatom oder der Tosylatrest genannt.
3. Eine Verbindung der allgemeinen Formel IV 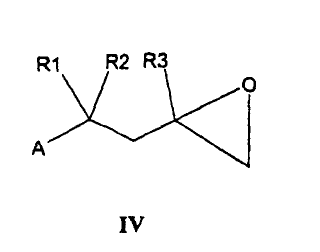 worin A, R¹,R² und R³ die in Formel I angegebene Bedeutung haben, wird mit einer Verbindung der Formel Ar-NH-R¹¹, wobei R¹¹ und Ar die oben angegebenen Bedeutungen haben, umgesetzt, wobei gegebenenfalls anschließend der Rest R¹¹ abgespalten wird, um zu einer Verbindung der Formel I mit B in der Bedeutung einer CH₂-Gruppe zu gelangen.
4. Eine Verbindung der Formel I, die im Rest A oder im Rest Ar die Gruppierung Aryl-X enthält, wobei "Aryl" einen isocyclischen oder heterocyclischen Aromaten entsprechend den für Formel I gegebenen Definitionen und X ein Brom- oder Jodatom oder die Gruppe -O-SO₂R¹², worin R¹² eine C₁-C₅-Perfluoralkylgruppe bedeutet, darstellt, wird nach an sich bekannten Verfahren mit einer Verbindung der Formel R¹³-Y, wobei R¹³ einen gegebenenfalls substituierten Aryl-, Ethenyl- oder Ethinylrest und Y ein Wasserstoffatom (J. Org. Chem. **43**, 2947 (1978)), die Gruppe B (O-R¹⁴)₂ (J. Org. Chem. **58**, 2201 (1993)) oder Sn(R¹⁵)₃ (J. Org. Chem. **52**. 422 (1987)) mit R¹⁴ und R¹⁵ in der Bedeutung eines Phenylrestes oder von C₁-C₅-Alkyl und für R¹⁴ auch Wasserstoff. Mg- Halogen oder ein Alkalimetallatom darstellt, unter Metallkatalyse zur Verbindung Aryl-R¹³ umgesetzt.
5. In einer Verbindung der Formel I, die in A oder Ar einen Alkoxy- oder Acyloxysubstituenten enthält, wird die OH-Gruppe freigesetzt und gegebenenfalls in einer weiteren Reaktion verethert oder verestert oder, nach Umwandlung in einen 1-Phenyl-5-tetrazolylether, durch Hydrierung völlig eliminiert (J. Amer Chem. Soc.**88**, 4271 (1966)).

Von den vorstehenden Verfahrensvarianten sind 1. und 2. für die Herstellung aller Verbindungen, die unter die allgemeine Formel I fallen, geeignet.

Mit der 3. Variante sind Verbindungen der allgemeinen Formel I herstellbar, worin B für eine - CH₂-Gruppe steht.

Mittels der 4. und 5. Verfahrenvariante lassen sich Funktionalisierungen an bereits vorliegenden Verbindungen der allgemeinen Formel I vornehmen.

Gewünschtenfalls lassen sich Verbindungen, die nach einem der vorstehenden Verfahren hergestellt wurden und in denen A ein gegebenenfalls substituierter aromatischer Ring ist, nach bekannten Verfahren an diesem aromatischen Rest selektiv substituieren, Beispiele für dieses Verfahren sind die katalytische Hydrierung von Mehrfachbindungen, die Nitrierung und die Halogenierung.

Die in den Beispielen verwendeten Ausgangsmaterialien werden folgendermaßen hergestellt:

### Herstellung der Ausgangsmaterialien

### 4-Methyl-4-phenyl-2-oxovaleriansäure

Eine aus 26.4 g Magnesium und 162 ml 2-Methyl-2-phenyl-1-chlorpropan in 150 ml Diethylether hergestellte Grignard-Lösung wurde bei -30°C zu 600 ml Oxalsäurediethylester getropft. Nach 2 Stunden bei Raumtemperatur wurde auf Ammoniumchloridlösung gegeben, mit Diethylether extrahiert, getrocknet (Na₂SO₄) und fraktioniert destilliert; man erhält 84 g des Ethylesters (Kp. 115-120°C / 0,03 hPa), der in 11 Methanol gelöst, mit 500 ml 1m Natriumhydroxid versetzt und 1,5 Stunden bei Raumtemperatur gerührt wird. Nach Abdampfen des Methanols im Vakuum wird der Rückstand zwischen Wasser und Diethylether verteilt, die wässrige Phase wird mit Salzsäure angesäuert und mit Diethylether extrahiert. Nach Eindampfen erhält man 57 g 4-Methyl-4-phenyl-2-oxovaleriansäure als dickes Öl.

### 4,4-Dimethyl-2-oxo-5-hexensäure

Aus 50 g 3,3-Dimethyl-4-pentensäuremethylester werden durch Verseifung mit 10%iger Kalilauge 36 g 3,3-Dimethyl-4-pentensäure als Öl erhalten. Durch Rühren mit Thionylchlorid (20 Stunden, Raumtemperatur) wird das Säurechlorid erhalten, Kp. 59°C / 30 hPa. 16 g davon werden mit 15 g Trimethylsilylcyanid und 0,16 g Zinkjodid 4 Tage gerührt. Nach Destillation erhält man 13 g 4,4-Dimethyl-2-oxo-5-hexensäurenitril, Kp. 75-85°C / 30 hPa. 2 g davon werden mit 0,6 ml Methanol in 13 ml Hexan unter Eiskühlung mit Salzsäuregas gesättigt und nach 2 Stunden mit Wasser versetzt. Aus der Hexanphase erhält man nach Trocknen (Na₂SO₄) und Eindampfen 0,558 g 4,4-Dimethyl-2-oxo-5-hexensäuremethylester, Kp. 48°C / 0,003 hPa. 0,535 g davon werden mit 1,3 ml 3 N Natronlauge verseift, wobei man 0,32 g 4,4-Dimethyl-2-oxo-5-hexensäure als gelbliche Flüssigkeit erhält.

### 3-(1-Phenyl-cyclobutyl)-2-oxo-propionsäure

10 g 1-Phenyl-cyclobutancarbonitril, gelöst in 70 ml Toluol, werden mit 56 ml Diisobutylaluminiumhydrid in Toluol (1,2 molar) bei -72 bis -69°C versetzt. Nach 4 Stunden bei - 75°C werden 30 ml Ethylacetat zugetropft. Nach Erwärmen auf Raumtemperatur wird weiteres Ethylacetat und Wasser zugesetzt. Man filtriert über Kieselgur, trennt die organische Phase ab, trocknet (Na₂SO₄) und dampft ein. Nach Chromatographie an Kieselgel (Hexan mit 0-10% Ethylacetat) erhält man 7,6 g 1-Phenyl-cyclobutancarbaldehyd. 3 g davon werden in 10 ml Tetrahydrofuran gelöst und bei 0°C zu einer Lösung getropft, bei der zuvor 5 g Triethyl-2-ethoxyphosphonoacetat in 70 ml Tetrahydrofuran bei 0°C mit 10,3 ml einer 2 molaren Lösung von Lithiumdiisopropylamid in Tetrahydrofuran / Heptan / Ethylbenzol versetzt wurden. Nach 20 Stunden bei Raumtemperatur wird Wasser zugegeben, mit Ethylacetat extrahiert, getrocknet (Na₂SO₄) und eingedampft. 2 g dieses Rohproduktes werden mit 28 ml 1 N Natronlauge verseift. Man erhält 1,32 g der Säure, die mit 25 ml 1 molarer Schwefelsäure unter starkem Rühren 20 Stunden auf 90°C erwärmt wird. Nach Extraktion mit Ether, Trocknen (Na₂SO₄) und Eindampfen erhält man 0, 89 g 3-(1-Phenyl-cyclobutyl)-2-oxo-propionsäure als gelbliches Öl.

### 3-[1-(2-Methoxyphenyl)-cyclopropyl-2-oxo-propionsäure

Entsprechend J. Org. Chem. **40** (1975) 3497 wurden 16,7 g 2-Methoxyphenylacetonitril, 158 ml Lithiumtriisopropylamid (2 molare Lösung) und 46,7 ml 1,2-Dichloroethan in 96 ml Tetrahydrofuran und 58,6 ml Hexamethylphosphorsäuretriamid miteinander umgesetzt. Man erhält 5,6 g 1-(2 Methoxyphenyl)-cyclopmpyl-carbonitril, Kp. 104-115°C / 0.1 mbar, die weiter wie für 3-(1-Phenyl-cyclobutyl)-2-oxo-propionsäure beschrieben, umgesetzt wurden. Man erhält so 3-[1-(2-Methoxyphenyl)-cyclopropyl]-2-oxo-propionsäure als Öl.

Analog dem für 3-(1-Phenyl-cyclobutyl)-2-oxo-propionsäure und für 3-[1-(2-Methoxyphenyl)-cyclopropyl]-2-oxo-propionsäure beschriebenen Verfahren wurden die in Tabelle 2 beschriebenen Säuren erhalten.

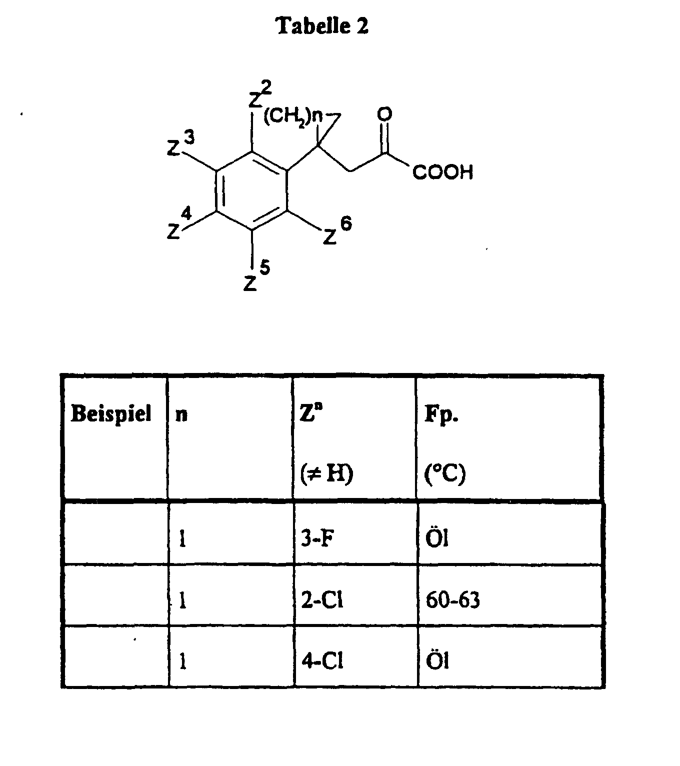

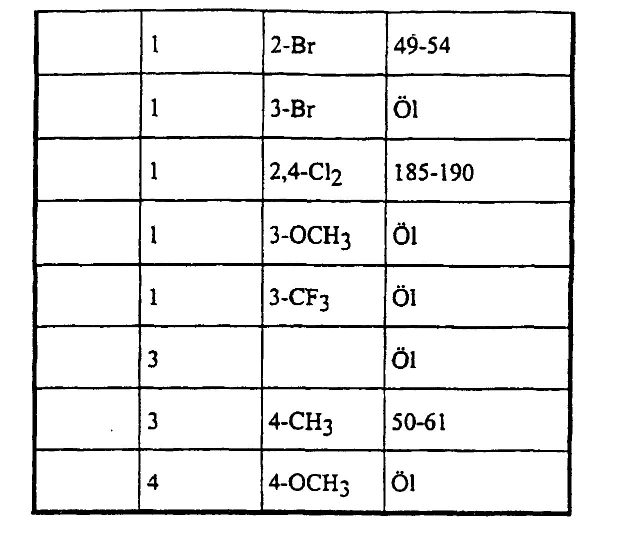

### 3-(1-Phenyl-cyclopropyl)-2-oxo-propionsäure

wird analog dem für 3-(1-Phenyl-cyclobutyl)-2-oxo-propionsäure beschriebenen Verfahren erhalten.

### 3-(1-Phenyl-cyclohexyl)-2-oxo-propionsäure.

wird analog dem für 3-(1-Phenyl-cyclobutyl)-2-oxo-propionsäure beschriebenen Verfahren erhalten.

### 4-(3-Methoxyphenyl)-4-methyl-2-oxo-valeriansäure

4,2 ml einer 0.6 m Lösung von 3-Methoxyphenylmagnesiumbromid in Tetrahydrofuran werden bei -70°C mit 257 mg Kupferbromid-Dimethylsulfid-Komplex versetzt und dann bei -40°C 20 Minuten gerührt. Man kühlt erneut auf-70°C, gibt 0,33 ml 1,3-Dimethyl-tetrahydro-2-1Hpyrimidinon und ein Gemisch von 400 mg 4-Methyl-2-oxo-3-pentensäuremethylester (Liebigs Annalen **1974**, 477) und 0,71 ml Trimethylchlorsilan in 3,5 ml Tetrahydrofuran langsam zu. Man rührt eine Stunde bei -70°C und erwärmt dann auf Raumtemperatur. Dann gibt man 2 N Salzsäure und Ethylacetat zu, trennt die Ethylacetatphase ab, dampft sie ein und löst den Rückstand in 5 ml Dichlormethan. Nach Zusatz von 200 mg Tetrabutylammoniunfluorid wird eine Stunde bei Raumtemperatur belassen, dann mit Wasser gewaschen und die Dichlormethan-Phase getrocknet (Na₂SO₄) und eingedampft. Man erhält nach Chromatographie an Kieselgel mit Hexan / Ethylacetat (97:3) 63 mg 4-(3-Methoxyphenyl)-4-methyl-2-oxo-valeriansäuremethylester, der mit 1 ml Kaliumhydroxid in Methanol( 10%). versetzt wird. Nach 45 Minuten wird eingedampft, der Rückstand in Wasser gelöst und mit Diethylether extrahiert. Die wässrige Phase wird dann mit 6 N Salzsäure angesäuert und mit Diethylether extrahiert. Die Diethyletherphase wird getrocknet (Na₂SO₄) und eingedampft. Man erhält 50 mg 4-(3-Methoxyphenyl)-4-methyl-2-oxo-valeriansäure.

### 2-Hydroxy-4-methyl-4-phenyl-2-trifluormethyl-valeriansäure

Aus 1,5 g Magnesium und 10 g 2-Methyl-2-phenylpropylchlorid in 100 ml Diethylether wird das Grignard-Reagenz hergestellt, das nach Reaktion mit 10 g Trifluorbrenztraubensäureethylester 9.5 g 2-Hydroxy-4-methyl-4-phenyl-2-trifluormethyl-valeriansäureethylester, Kp. 90°C / 0,045 hPa, gibt.
7.5 g des Ethylesters werden mit 100 ml Kaliumhydroxid in Methanol (10%) 18 Stunden im Rückfluß gekocht. Nach Einengen im Vakuum wird der Rückstand in Wasser gelöst und mit Diethylether extrahiert. Die wässrige Phase wird mit 2 N Salzsäure angesäuert und mit Diethylether extrahiert. Nach Eindampfen des Lösemittels erhält man 3,2 g 2-Hydroxy-4-methyl-4-phenyl-2-trifluormethyl-valeriansäure als farblose Kristalle, Fp. 124-126°C.

### 4-(5-Fluor-2-methoxyphenyl)-2-hydroxy-4-methyl-2-trifluormethyl-valeriansäure

1,3 g wasserfreies Zinkchlorid und 13,2 g gekömtes Mangan werden in 100 ml Tetrahydrofuran zum Sieden erhitzt und mit 0,2 ml Methallylbromid 30 Minuten gekocht. Dann wird die Lösung von 25 g Methallylbromid und 17 g Trifluorbrenztraubensäure-ethylester in 80 ml Tetrahydrofuran im Laufe von 2 Stunden bei Siedehitze zugetropft und eine weitere Stunde gekocht. Dann gibt man unter Eiskühlung gesättigte Ammoniumchloridlösung und 300 ml Ethylacetat zu, rührt 30 Minuten bei 0°C und wäscht die abgetrennte Ethylacetat-Phase mit gesättigter Ammoniumchloridlösung und dreimal mit Wasser. Das Lösemittel wird getrocknet (Na₂SO₄) und eingedampft und der Rückstand im Vakuum destilliert. Man erhält 17,6 g 2-Hydroxy-4-methylen-2-trifluomnethyl-valeriansäureethylester, Kp. 48°C / 1 hPa.

Zu 5 ml 4-Fluoranisol und 0,9 g 2-Hydroxy-4-methylen-2-trifluormethyl-valeriansäureethylester werden 0,8 g wasserfreies Aluminiumchlorid gegeben. Nach 40 Stunden Rühren bei Raumtemperatur wird auf eiskalte 2 N Salzsäure gegeben und mit Ethylacetat extrahiert. Die Ethylacetat-Phase wird mit 1 N Salzsäure und Wasser gewaschen, getrocknet (Na₂SO₄) und eingedampft. Nach Chromatographie an Kieselgel mit Hexan / Ethylacetat (1:1) erhält man 1 g 4-(5-Fluor-2-methoxyphenyl)-2-hydroxy-4-methyl-2-trifluormethyl-valeriansäureethylester, Fp. 38-39°C.

1,9 g 4-(5-Fluor-2-methoxyphenyl)-2-hydroxy-4-methyl-2-trifluormethyl-valeriansäureethylester werden mit 40 ml Kaliumhydroxid im Methanol (10%) 2 Stunden im Rückfluß gekocht. Nach Eindampfen des Lösemittels im Vakuum wird Wasser zugegeben, mit Hexan extrahiert und die abgetrennte Wasserphase mit 6 N Salzsäure angesäuert. Nach Extraktion mit Ethylacetat wird die Ethylacetat-Phase mit Wasser gewaschen, getrocknet (Na₂SO₄) und eingedampft. Der Rückstand wird aus Hexan kristallisiert. Man erhält 1,55 g 4-(5-Fluor-2-methoxyphenyl)-2-hydroxy-4-methyl-2-trifluonnethyl-valeriansäure, Fp. 102-104 °C.

### 2-Hydroxy-4-methyl-4-(2-thienyl)-2-trifluormethyl-valeriansäure und 2-Hydroxy-4-methyl-4-(3-thienyl)-2-trifluormethyl-valeriansäure

Analog wurde das Gemisch von 2-Hydroxy-4-methyl-4-(2-thienyl)-2-trifluormethylvaleriansäure und 2-Hydroxy-4-methyl-4-(3-thienyl)-2-trifluormethyl-valeriansäure (9:1) hergestellt, Fp. 150-151°C.

Die Säuren der Tabelle 3 wurden analog hergestellt.

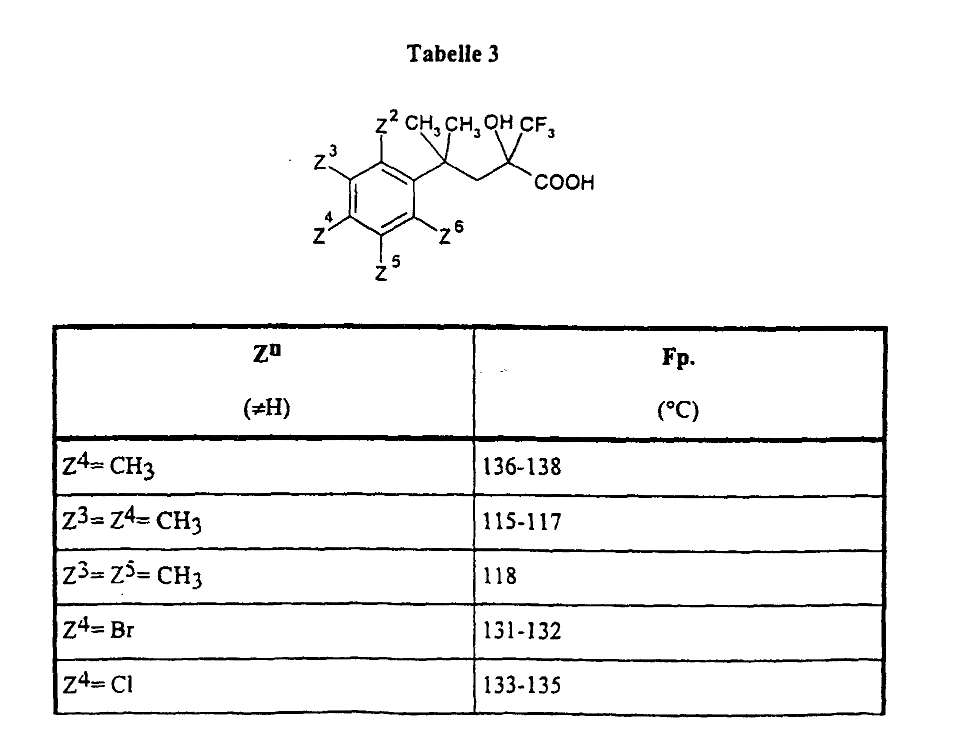

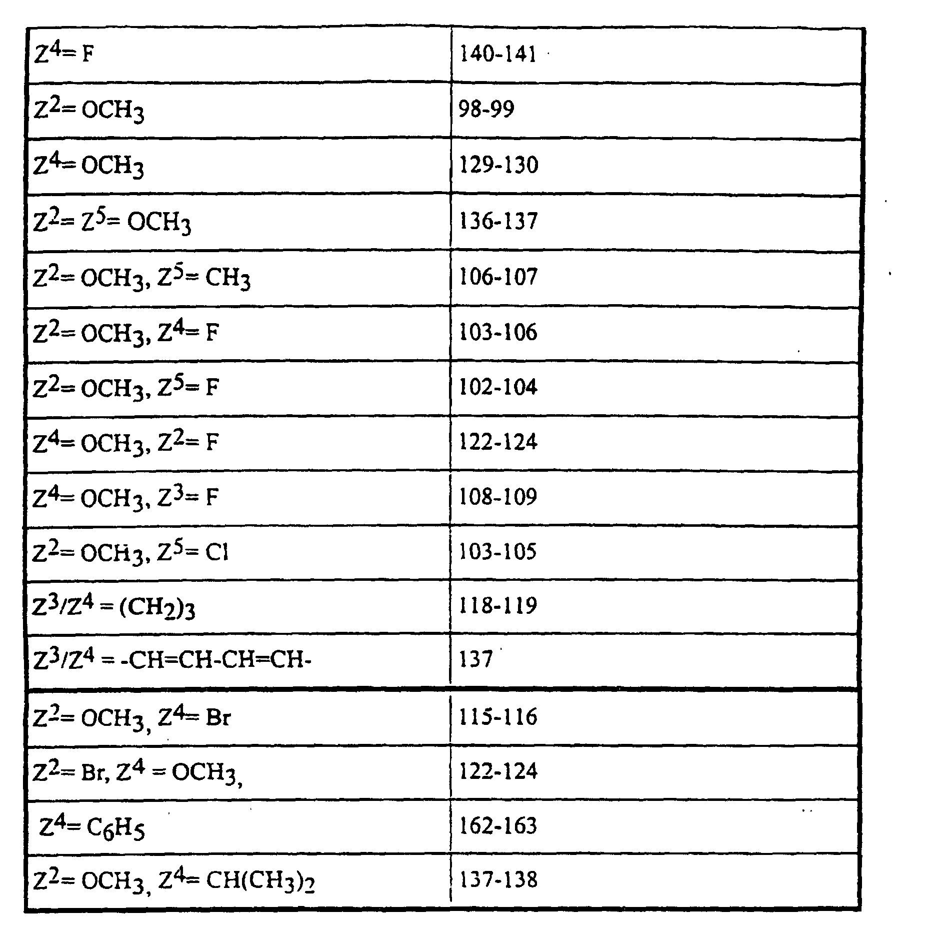

Durch Umwandlung nach Standardverfahren werden weitere Säuren aus vorstehenden Säuren oder ihren Vorstufen erhalten:

### 2-Hydroxy-4-methyl-2-trifluormethyl-4-(4-vinylphenyl)-valeriansäure

Durch Erhitzen von 4-(4-Bromphenyl)-2-hydroxy-4-methyl-2-trifluormethylvaleriansäureethylester, Tributylvinylzinn, Tri-o-tolylphosphin und Bis-tri-o-tolylphosphinpalladium-II-chlorid in Dimethylformamid auf 120°C erhält man 2-Hydroxy-4-methyl-2-trifluormethyl-4-(4-vinylphenyl)-valeriansäureethylester, der durch alkalische Verseifung die Titelverbindung,Fp. 73-74°C, gibt.

### 4-(4-Acetylphenyl)-2-hydroxy-4-methyl-2-trifluormethyl-valeriansäure

Analog zur vorstehenden Verbindung aus 4-(4-Bromphenyl)-2-hydroxy-4-methyl-2-trifluormethyl-valeriansäureethylester, Tributyl-1-ethoxyvinylzinn, Tri-o-tolylphosphin und Bistri-o-tolylphosphin-palladium-II-chlorid in Dimethylformamid auf 120°C und anschließende saure Hydrolyse des Enolethers und alkalische Verseifung, Fp.158-162°C.

### 4-(4-Acetyl-3-methoxyphenyl)-2-hydroxy-4-methyl-Z-trifluormethyl-valeriansäure

Analog zur vorstehenden Verbindung aus 4-(4-Brom-3-methoxyphenyl)-2-hydroxy-4-methyl-2-trifluormethyl-valeriansäureethylester, Tributyl-1-ethoxyvinylzinn, Tri-o-tolylphosphin und Bistri-o-tolylphosphin-palladium-II-chlorid in Dimethylformamid auf 120°C, Öl.

### 4-(4-Cyanphenyl)-2-hydroxy-4-methyl-2-trifluormethyl-valeriansäure

Aus 4-(4-Bromphenyl)-2-hydroxy-4-methyl-2-trifluorrnethyl-valeriansäureethylester, Zinkcyanid und Tetrakis-triphenylphosphin-palladium in Dimethylformamid bei 140°C. Nach Verseifung erhält man die Titelsäure als Schaum.

### 4-(4-Carbamoylphenyl)-2-hydroxy-4-methyl-2-trifluormethyl-valeriansäure

erhält man durch Behandlung des Ethylesters der vorstehenden Säure mit Wasserstoffperoxid und Verseifung, Fp. 244-245°C.

### 4-(4-Cyan-2-methoxyphenyl)-2-hydroxy-4-methyl-2-trifluormethyl-valeriansäure

Aus 4-(4-Brom-2-methoxyphenyl)-2-hydroxy-4-methyl-2-trifluormethyl-valeriansäureethylester, Zinkcyanid und Tetrakis-triphenylphosphin-palladium in Dimethylformamid bei 140°C. Nach Verseifung erhält man die Titelsäure als amorphes Pulver.

### 4-(3-Brom-4-methoxyphenyl)-2-hydroxy-4-methyl-2-trifluormethyl-valeriansäure

Aus 2-Hydroxy-4-(4-methoxyphenyl)-4-methyl-2-trifluormethyl-valenansäureethylester durch Bromierung mit N-Bromsuccinimid in Dimethylformamid bei 0°C und anschließende Verseifung. Fp. 94-96°C.

### 2-Hydroxy-4-methyl-4-(3-nitro-4-methoxyphenyl)-2-trifluormethyl-valeriansäure

Diese Verbindung wird durch Umsetzung von 2,5 g 2-Hydroxy-4-(4-methoxyphenyl)-4-methyl-2-trifluormethyl-valeriansäureethylester mit 4 ml 100 prozentiger Salpetersäure in 12 ml Trifluoressigsäure über eine Stunde bei 0°C erhalten, Fp. 79-80°C.

### 4-(4-Jod-2-methoxyphenyl)-4-methyl-2-oxo-valeriansäure

Zu 24,2 mmol Methylmagnesiumbromid in 23 ml Diethylether werden 3,2 g 4-Jod-2-methoxybenzoesäure-methylester in 10 ml Diethylether gegeben.Nach 20 Stunden wird Ammoniumchloridlösung zugegeben, die Etherphase abgetrennt, getrocknet und eingedampft. 2,4 g des Rückstands werden in 10 ml Dichlormethan gelöst, mit 714 mg 2-Trimethylsilyloxyacrylsäure-ethylester versetzt, auf -70°C gekühlt und mit 0,27 ml Zinn(IV)chlorid versetzt. Nach 15 Minuten wird die Lösung auf Kaliumcarbonatlösung gegeben. Nach Extraktion mit Diethylether wird die organische Phase mit Wasser gewaschen, getrocknet und eingedampft. 500 mg des so erhaltenen 4-(4-Jod-2-methoxyphenyl)-4-methyl-2-oxo-valeriansäureethylesters werden mit 8,6 ml 1 M Natriumhydroxid in Ethanol / Wasser (2 : 1, v/v) 3 Stunden bei Raumtemperatur gerührt. Nach Zugabe von Wasser wird mit Diethylether extrahiert, die W asserphase mit 1 m Salzsäure angesäuert und mit Diethylether extrahiert. Nach Trocknen und Eindampfen erhält man 410 mg 4-(4-Jod-2-methoxyphenyl)-4-methyl-2-oxo-valeriansäure als gelbliches Öl.

### 4-(3-Chlorphenyl)-4-methyl-2-oxo-valeriansäure

wird analog dem vorstehenden Ausführungsbeispielals amorphes Pulver erhalten.

### 4-(3-Bromphenyl)-4-methyl-2-oxo-valeriansäure

wird analog dem vorstehenden Ausführungsbeispielals amorphes Pulver erhalten.

### 4-(2-Jodphenyl)-4-methyl-2-oxo-valeriansäure

wird analog dem vorstehenden Ausführungsbeispiel als amorphes Pulver erhalten.

### 4-(3-Jodphenyl)-4-methyl-2-oxo-valeriansäure

wird analog dem vorstehenden Ausführungsbeispielals amorphes Pulver erhalten.

### 4-(4-Jodphenyl)-4-methyl-2-oxo-valeriansäure

wird analog dem vorstehenden Ausführungsbeispiel-als Öl erhalten.

### 4-(5-Fluor-2-methoxyphenyl)-4-methyl-2-oxo-valeriansäure

wird analog dem vorstehenden Ausführungsbeispiel erhalten, Fp. 58-60°C.

### 4-(4-Brom-2-methoxyphenyl)-2-oxo-valeriansäure

wird analog dem vorstehenden Ausführungsbeispiel als Öl erhalten.

### 3-(1-Phenylcyclopentyl)-brenztraubensäure

wird analog dem vorstehenden Ausführungsbeispiel aus 1-Phenylcyclopentanol mit 2-Trimethylsilyloxy-acrylsäure-ethylester und Zinn(IV)chlorid als Öl erhalten.

### 4-Toluolsulfonsäure-(2-hydroxy-4-phenyl-2-trifluormethyl-pentyl)ester

Aus 2,6 g Magnesiumspänen und 15 ml 2-Phenyl-1-chlorpropan in Diethylether wird eine Grignardlösung bereitet, zu der bei -30°C innerhalb von 30 Minuten 15 ml Oxalsäurediethylester gegeben werden. Es wird eine Stunde bei -20°C und 2 Stunden bei 0°C gerührt und dann mit gesättigter Ammoniumchloridlösung versetzt. Die Diethylether-Phase wird abgetrennt, getrocknet (Na₂SO₄) und eingedampft und im Vakuum destilliert. Man erhält 17,7 g 2-Oxo-4-phenylvaleriansäureethylester, Kp. 98-100°C / 0,03 hPa.

4,4 g 2-Oxo-4-phenylvaleriansäureethylester werden in 40 ml Tetrahydrofuran gelöst und bei -78°C mit 3,6 ml Trifluormethyl-trimethylsilan und 2 ml 1 M Tetrabutylammoniunfluorid in Tetrahydrofuran versetzt. Nach 24 Stunden bei -78°C werden weitere 20 ml 1 M Tetrabutylammoniumfluorid in Tetrahydrofuran zugegeben. Man rührt 1,5 Stunden bei 0°C. setzt Ethylacetat und gesättigte Kochsalzlösung zu, trennt die organische Phase ab und wäscht sie mit ges. Kochsalzlösung und Wasser. Dann wird getrocknet (Na₂SO₄) und eingedampft und am Kugelrohr destilliert. Man erhält 4,4 g 2-Hydroxy-4-phenyl-2-trifluormethylvaleriansäureethylester, Kp. 95-100°C / 0,04 hPa.

4,35 g 2-Hydroxy-4-phenyl-2-trifluormethyl-valeriansäureethylester werden in 100 ml Diethylether gelöst und bei 0°C mit 1,3 g Lithiumaluminiumhydrid eine Stunde bei 0°C und 16 Stunden bei Raumtemperatur gerührt. Man setzt unter Kühlung wenig Wasser zu und rührt eine Stunde. Die Diethyletherphase wird abgetrennt, getrocknet (Na₂SO₄) und eingedampft und am Kugelrohr destilliert. Man erhält 4,1 g 4-Phenyl-2-trifluormethyl-1,2-pentandiol, Kp. 120 °C /0,04 hPa.

4,25 g 4-Phenyl-2-trifluormethyl-1,2-pentandiol in 30 ml Pyridin werden bei 0°C mit 3,8 g 4-Toluolsulfonsäurechlorid versetzt. Nach 16 Stunden bei 0°C wird im Vakuum eingeengt, mit Ethylacetat versetzt, mit Wasser gewaschen, getrocknet (Na₂SO₄) und eingedampit.Durch Kristallisation aus Ethylacetat/Hexan erhält man 4,9 g 4-Toluolsulfonsäure-(2-hydroxy-4-phenyl-2-trifluormethyl-pentyl)ester, Fp. 95-96°C.

Analog wird 4-Toluolsulfonsäure-(2-hydroxy-4-methyl-4-phenyl-2-trifluormethyl-pentyl)ester erhalten, Fp. 78°C.

Analog wurde 4-Toluolsulfonsäure-[4-(4-fluorphenyl)-2-hydroxy-4-methyl-2-trifluormethylpentyl]ester, Fp. 80-81°C, und 4-Toluolsulfonsäure-[2-hydroxy-4-(2-methoxy-5-fluorphenyl)-4-methyl-2-trifluormethyl-pentyl]ester hergestellt, Fp. 93-95°C.

### 2-(2-Phenylpropyl)-2-trifluormethyl-oxiran

400 mg 4-Toluolsulfonsäure-(2-hydroxy-4-phenyl-2-trifluotmethyl-pentyl)ester in 5 ml Dimethylformamid werden bei 0°C mit 35 mg Natriumhydrid (80% in Mineralöl) versetzt. Nach einer Stunde bei 0°C wird mit Wasser verdünnt und mit Dichlormethan extrahiert. Die Dichlormethan-Phase wird mit Wasser gewaschen, getrocknet (Na₂SO₄) und eingedampft. Der Rückstand wird destilliert. Man erhält 200 mg 2-(2-Phenylpropyl)-2-trifluormethy-oxiran, Kp. 110°C / 1 hPa.

### 4-Brom-5-aminophthalid

23 g 3-Brom-4-nitro-1,2-xylol werden in 200 ml Pyridin und 600 ml Wasser suspendiert und bei 60°C portionsweise mit 260 g Kaliumpermanganat versetzt, wobei die Temperatur auf 90°C steigt. Man erwärmt noch 2 Stunden auf 95°C , filtriert, säuert das Filtrat mit Salzsäure an und extrahiert mit Diethylether. Nach Eindampfen des Lösemittels erhält man 27 g 3-Brom-4-nitrophthalsäure.

12 g der Säure werden 15 Minuten auf 220°C erhitzt und danach am Kugelrohr destilliert. Bei 0,03 hPa destillieren 10 g 3-Brom-4-nitrophthalsäureanhydrid über.

Das Anhydrid wird in 120 ml Dimethylformamid gelöst und bei 0°C mit 78,8 ml einer 0,5 M Lösung von Natriumborhydrid in Dimethylformamid langsam versetzt. Nach drei Stunden bei 0°C wird vorsichtig 2 n Salzsäure zugesetzt und mit Ethylacetat extrahiert. Nach Waschen mit Kaliumbicarbonatlösung, Trocknen (Na₂SO₄) und Eindampfen der Ethylacetatphase erhält man 6,6 g 4-Brom-5-nitrophthalid.

6,6 g 4-Brom-5-nitrophthalid werden in 45 ml Ethanol gelöst und zu einem auf 60°C erwärmten und gut gerührten Gemisch von 65 g Eisen-II-sulfat, 220 ml Wasser und 65 ml Ammoniak (33%) getropft. Nach 2 Stunden bei 60°C wird das Gemisch fünfmal mit 200 ml Diethylether ausgerührt. Die Diethyletherphasen werden eingedampft. Als Rückstand werden 4,1 g 4-Brom-5-aminophthalid erhalten, Fp.176-180°C.

### 6-Brom-5-aminophthalid

4-Brom-5-nitrophthalsäureanhydrid wird analog dem vorstehend beschriebenen Verfahren aus 4-Brom-5-nitro-1,2-xylol hergestellt.

Durch Kochen mit Ethanol wird daraus ein Gemisch von 2-Brom-6-ethoxycarbonyl-3-nitrobenzoesäure und 3-Brom-2-ethoxycarbonyl-4-nitro-benzoesäure gewonnen.

Zu 7,2 ml einer 0,66 m Lösung von Dimethylformamid in Dichlormethan werden bei 0°C vorsichtig 1,2 ml Oxalylchlorid getropft. Die Lösung wird 1 Stunde bei 0°C und 5 Minuten bei Raumtemperatur gerührt. Nach Eindampfen im Vakuum wird der Rüchstand in 7 ml Acetonitril suspendiert. auf-35°C gekühlt und tropfenweise mit 1,5 g des Estergemisches versetzt. Nach einer Stunde bei derselben Temperatur wird auf -70°C abgekühlt und 2,4 ml einer 2 m Lösung von Natriumborhydrid in Dimethylformamid zugetropft. Man rührt 20 Stunden bei Raumtemperatur, setzt Wasser zu, alkalisiert mit Kaliumcarbonat und extrahiert mit Diethylether. Die Diethylether-Phase wird getrocknet (Na₂SO₄) und eingedampft. Man erhält ein Gemisch von 5-Brom-6-nitrophthalid und 6-Brom-5-nitrophthalid, das an Kieselgel mit Hexan Ethylacetat ( 95:5) getrennt wird.

Die Reduktion zum Aminophthalid erfolgt wie oben beschrieben. Man erhält 6-Brom-5-aminophthalid, Fp. 235-241°C.

### 5-Amino-3-(1-propenyl)-phthalid

5 g 2-Brom-4-nitrobenzoesäure werden durch 2-stündiges Kochen mit 30 ml Thionylchlorid und Abdestillieren überschüssigenThionylchlorids ins Säurechlorid überführt, das in 50 ml Tetrahydrofuran gelöst und zu 3 ml Allylamin in 20 ml Tetrahydrofuran getropft wird. Nach 20 Stunden bei Raumtemperatur wird zwischen 1 N Salzsäure und Ethylacetat verteilt, die Ethylacetatphase mit Wasser gewaschen,getrocknet (Na₂SO₄) und eingedampft. Der Rückstand wird mit Hexan kristallisiert. Man erhält 5,6 g 2-Brom-4-nitrobenzoesäure-allylamid, Fp. 98-100°C.

Dieses Material wird in 35 ml Ethanol gelöst und zu einem auf 60°C erwärmten und gut gerührten Gemisch von 50 g Eisen-II-sulfat, 170 ml Wasser und 50 ml Ammoniak (33%) getropft. Nach 2 Stunden bei 60°C wird das Gemisch 5 mal mit 200 ml Diethylether ausgerührt. die Diethyletherphasen werden eingedampft und der Rückstand wird mit Hexan kristallisiert. Man erhält 3,1 g 4-Amino-2-brom-benzoesäure-allylamid, Fp. 115-117°C.

11 g 4-Amino-2-brom-benzoesäure-allylamid, 5,2 ml Acetonylaceton und 200 mg 4-Toluolsulfonsäure werden 1,5 Stunden mit Wasserabscheider im Rückfluß gekocht. Dann wird die Lösung mit Ethylacetat verdünnt, mit 1 N Salzsäure und danach mit Kaliumcarbonatlösung gewaschen, getrocknet (Na₂SO₄) und eingedampft. Der Rückstand wird mit Hexan kristallisiert. Man erhält 13,4 g N-allyl-2-brom-4-(2,5-dimethylpyrrol-1-yl)-benzamid, Fp. 136-138°C.

3 g N-allyl-2-brom-4-(2,5-dimethylpyrrol-1-yl)-benzamid In 100 ml Dimethoxyethan werden bei -70°C mit 14,2 ml 1,4 M Butyllithium in Hexan versetzt. Nach 30 Minuten bei -70°C werden 1,63 ml Crotonaldehyd zugegeben. Man läßt die Lösung auf Raumtemperatur erwärmen, rührt 20 Stunden weiter, setzt 50 ml 50-prozentige Essigsäure zu und erwärmt 6 Stunden auf 60°C. Dann verdünnt man mit Wasser, extrahiert mit Ethylacetat, wäscht die Ethylacetatphase mit Kaliumcarbonatlösung. Die Ethylacetatphase wird getrocknet (Na₂SO₄) und eingedampft. Der Rückstand ergibt nach Chromatographie an Kieselgel mit Hexan Ethylacetat (98:2) 1,1 g kristallines 5-(2,5-Dimethylpyrrol-1-yl)-3-(1-propenyl)-phthalid, Fp. 91-95°C

1,1 g 5-(2,5-Dimethylpyrrol-1-yl)-3-(1-propenyl)-phthalid, 8,56 g Hydroxylamin-hydrochlorid und 4,58 g Kaliumhydroxid in 75 ml Ethanol/Wasser (16:6,8, vv) werden 24 Stunden bei 120 °C erhitzt. Das Lösemittel wird abdestilliert, der Rückstand mit Waser versetzt und mit Ethylacetat extrahiert. Die Ethylacetatphase wird getrocknet (Na₂SO₄) und eingedampft und an Kieselgel chromatographiert. Mit Dichlormethan / Methanol (99:1) erhält man 640 mg 5-Amino-3-(1-propenyl)-phthalid, Fp. 125-130°C.

Analog erhält man die Phthalide der Tabelle 4.

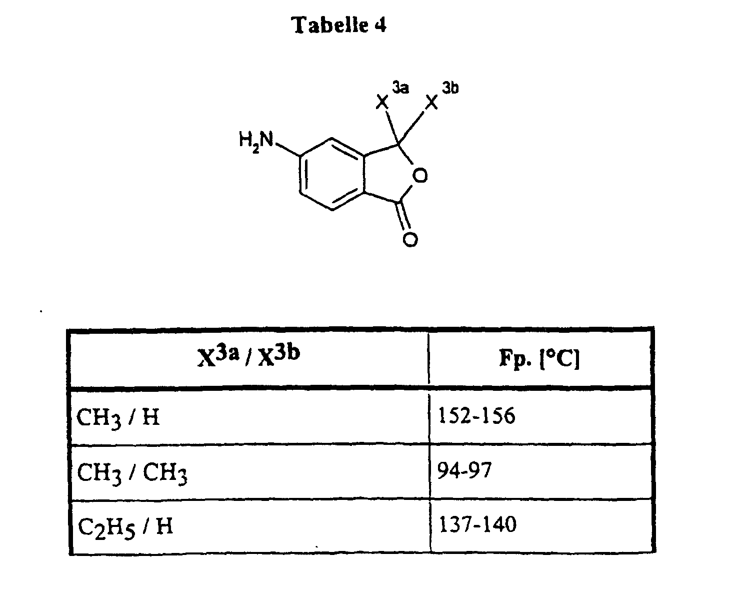

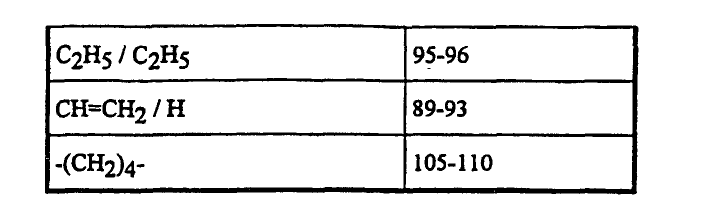

### 4-Brom-5-(4-methyl-2-oxo-4-phenyl-valeroylamino)-phthalid

412 mg 4-Methyl-4-phenyl-2-oxovaleriansäure werden in 10 ml Dimethylacetamid gelöst und unter Argon bei -8°C mit 261 mg Thionylchlorid versetzt. Nach 20 Minuten Rühren bei -3 bis +3°C werden 228 mg 4-Brom-5-aminophthalid zugegeben. Man rührt 1,5 Stunden bei Raumtemperatur, versetzt dann mit Wasser, extrahiert mit Ethylacetat, wäscht die organische Phase mit Wasser, trocknet (Na₂SO₄) und erhält nach Eindampfen des Lösemittels und Behandeln mit Diethylether 360 mg 4-Brom-5-(4-methyl-2-oxo-4-phenyl-valeroylamino)-phthalid, Fp. 150-152°C.

Analog zur Darstellung von 4-Brom-5-(4-methyl-2-oxo-4-phenyl-valeroylamino)-phthalid werden die Verbindungen der Tabellen 5 und 6 erhalten.

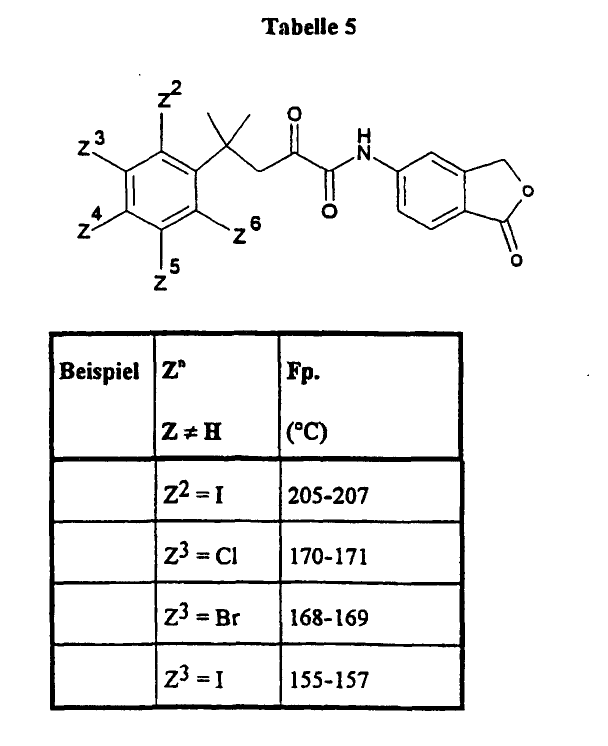

### 5-[3-(1-Phenyl-cyclopropyl)-2-oxo-propionylamino]-phthalid

wurde analog dem für 4-Brom-5-(4-methyl-2-oxo-4-phenyl-valeroylamino)-phthalid beschriebenen Verfahren aus 5-Aminophthalid und 3-(1-Phenyl-cyclopropyl)-2-oxopropionsäure erhalten, Fp.132-138°C.

### 5-[3-(1-Phenyl-cyclobutyl)-2-oxo-propionylamino]-phthalid

wurde analog dem für 4-Brom-5-(4-methyl-2-oxo-4-phenyl-valeroylamino)-phthalid beschriebenen Verfahren aus 5-Aminophthalid und 3-(1-Phenyl-cyclobutyl)-2-oxo-propionsäure erhalten, Fp.142-146°C.

### 5-[3-(1-Phenyl-cyclohexyl)-2-oxo-propionylamino]-phthalid

wurde analog dem für 4-Brom-5-(4-methyl-2-oxo-4-phenyl-valeroylamino)-phthalid beschriebenen Verfahren aus 5-Aminophthalid und 3-(1-Phenyl-cyclohexyl)-2-oxo-propionsäure erhalten, Fp.120-123°C.

### Ebenso wurden die Verbindungen der Tabelle 6 hergestellt:

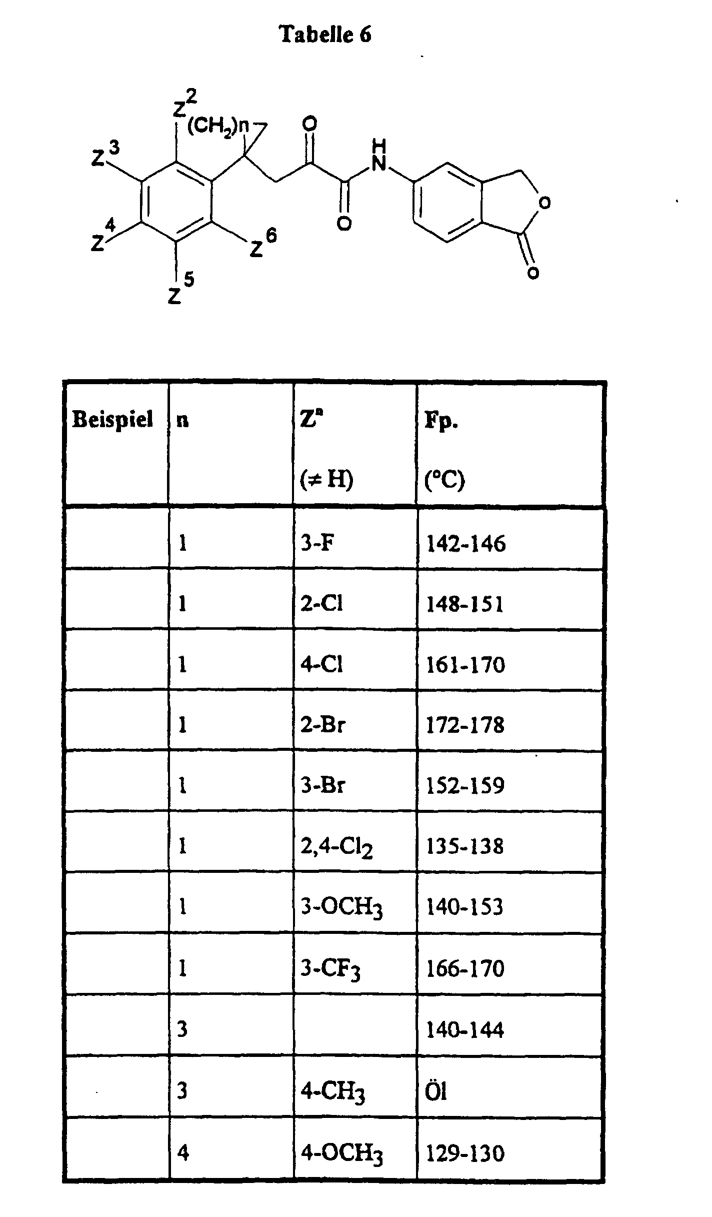

### 6-[3-(1-Phenyl-cyclopropyl)-2-oxo-propionylamino]-4-methyl-2,3-benzoxazin-1-on

wurde analog dem für 4-Brom-5-(4-methyl-2-oxo-4-phenyl-valeroylamino)-phthalid beschriebenen Verfahren aus 6-Amino-4-methyl-2,3-benzoxazin-1-on und 3-(1-Phenylcyclopropyl)-2-oxo-propionsäure erhalten, Fp.197-200°C.

### 6-[3-(1-Phenyl-cyclobutyl)-2-oxo-propionylamino]-4-methyl-2,3-benzoxazin-1-on

wurde analog 6-[3-(1-Phenyl-cyclopropyl)-2-oxo-propionylamino]-4-methyl-2,3-benzoxazin-1-on unter Verwendung von 3-(1-Phenyl-cyclobutyl)-2-oxo-propionsäure erhalten, Fp.155-156°C.

### 6-[3-(1-Phenyl-cyclohexyl)2-oxo-propionylamino]-4-methyl-2,3-benzoxazin-1-on

wurde analog 6-[3-(1-Phenyl-cyclopropyl)-2-oxo-propionylamino]-4-methyl-2,3-benzoxazin-1-on unter Verwendung von 3-(1-Phenyl-cyclohexyl)-2-oxo-propionsäure erhalten, Fp.132-134°C

### 5-(4,4- Dimethyl-2-oxo-5-hexenoylamino)-phthalid

wurde analog dem für 4-Brom-5-(4-methyl-2-oxo-4-phenyl-valeroylamino)-phthalid beschriebenen Verfahren aus 5-Aminophthalid und 4,4-Dimethyl-2-oxo-5-hexensäure erhalten, Fp.103-104°C.

### 5-Brom-5-(4-methyl-2-oxo-4-phenyl-valeroylamino)-phthalid

Analog dem vorhergehenden Beispiel wird aus 2,0 g 4-Methyl-4-phenyl-2-oxovaleriansäure und 1,11 g 6-Brom-5-aminophthalid mit 1,27 g Thionylchlorid in 60 ml Dimethylacetamid 1,7 g 6-Brom-5-(4-methyl-2-oxo-4-phenyl-valeroylamino)-phthalid erhalten, Fp. 148-150°C.

### 5-[4-(4-Jod-2-methoxyhenyl)-4-methyl-2-oxo-valeroylamino)-phthalid

wurde analog dem für 4-Brom-5-(4-methyl-2-oxo-4-phenyl-valeroylamino)-phthalid beschriebenen Verfahren aus 5-Aminophthalid und 4-(4-Jod-2-methoxyphenyl)-4-methyl-2-oxovaleriansäure als Schaum erhalten.

### 5-[4-(4-Jodpbenyl)-4-methyl-2-oxo-valeroylamino)-phthalid

wurde analog dem für 4-Brom-5-(4-methyl-2-oxo-4-phenyl-valeroylamino)-phthalid beschriebenen Verfahren aus 5-Aminophthalid und 4-(4-Jodphenyl)-4-methyl-2-oxovaleriansäure als Öl erhalten.

### 5-[4-(3-Jodphenyl)-4-methyl-2-oxo-valeroylamino)-phthalid

wurde analog dem für 4-Brom-5-(4-methyl-2-oxo-4-phenyl-valeroylamino)-phthalid beschriebenen Verfahren aus 5-Aminophthalid und 4-(3-Jodphenyl)-4-methyl-2-oxovaleriansäure erhalten, Fp. 160-161 °C.

### 5-[4-(4-Brom-2-methoxyhenyl)-2-oxo-valeroylamino)-phthalid

wurde analog dem für 4-Brom-5-(4-methyl-2-oxo-4-phenyl-valeroylamino)-phthalid beschriebenen Verfahren aus 5-Aminophthalid und 4-(4-Brom-2-methoxyphenyl)-2-oxovaleriansäure erhalten, Fp. 136-140°C.

### 5-[3-(1-Phenyl-cyclopentyl)-2-oxo-propionylamino]-phthalid

wurde analog dem für 4-Brom-5-(4-methyl-2-oxo-4-phenyl-valeroylamino)-phthalid beschriebenen Verfahren aus 5-Aminophthalid und 3-(1-Phenyl-cyclopentyl)-2-oxopropionsäure erhalten, Fp.140-144°C.

### 6-[4-(5-Fluor-2-methoxyhenyl)-4-methyl-2-oxo-valeroylamino)-4-methyl-2,3-benzoxazin-1-on

wurde analog dem für 4-Brom-5-(4-methyl-2-oxo-4-phenyl-valeroylamino)-phthalid beschriebenen Verfahren aus 4-Methyl-2,3-benzoxazin-1-on und 4-(5-Fluor-2-methoxyphenyl)-4-methyl-2-oxo-valeriansäure erhalten, Fp. 171-173°C.

### 6-[4-(5-Fluor-2-methoxyhenyl)-4-methyl-2-oxo-valeroylamino)-4-ethyl-2,3-benzoxazin-1-on

wurde analog dem für 4-Brom-5-(4-methyl-2-oxo-4-phenyl-valeroylamino)-phthalid beschriebenen Verfahren aus 4-Ethyl-2,3-benzoxazin-1-on und 4-(5-Fluor-2-methoxyphenyl)-4-methyl-2-oxo-valeriansäure erhalten, Fp. 157-158°C.

### 6-Amino-4-methyl-2,3-benzoxazin-1-on

60 g 2-Methyl-5-nitroacetophenon, 38,5 2,2-Dimethyl-1,3-propandiol und 6 g p-Toluolsulfonsäure werden in 11 Toluol mit einem Wasserabscheider bis zum Ende der Wasserentwicklung gekocht. Die Lösung wird mit Kalumbicarbonat gewaschen,getrocknet (Na₂SO₄) und eingedampft. Aus Pentan erhält man 71,7 g des kristallinen Ketals.

Dieses wird in 1,5 l Pyridin und 4,5 l Wasser mit 350 g Kaliumpermanganat, wie oben bei der Herstellung von 4-Brom-5-aminophthalid beschrieben, oxidiert. Man erhält 56,4 g 4-Nitro-2-(2,5,5-trimethyl-1,3-dioxan-2-yl)-benzoesäure.

52 g der Säure werden in 500 ml Methanol und 500 ml Ethylacetat mit 10 g Palladium/Kohle (10%) hydriert. Man erhält aus Pentan 45,5 g der kristallinen Aminoverbindung.

10 g des Amins werden mit 100 ml konzentrierter Salzsäure 2 Stunden am Rückfluß gekocht. Das Lösemittel wird im Vakuum eingedampft und der Rückstand mit 15,7 g Hydroxylamin Hydrochlorid, 8,4 g Kaliumhydroxid, 120 ml Ethanol und 50 ml Wasser 12 Stunden am Rückfluß gekocht. Man verdünnt mit Wasser und saugt die Kristalle ab. Nach Trocknen erhält man 3,5 g 6-Amino-4-methyl-2,3-benzoxazin-1-on, Fp. 291-296°C.

### 6-Amino-4-ethyl-2,3-benzoxazin-1-on

wird analog aus 2-Methyl-5-nitropropiophenon erhalten, Fp 89-93°C.

### 6-Amino-1-methyl-1H-benzotriazol

ist in Heterocycles **36**, 259 (1993) beschrieben.

### 5-Amino-benz[1.2.5]oxadiazol

ist in Boll. Sci. Fac. Chim. Ind. Bologna, **22**, 33,36.37 (1964) beschrieben.

### 5-Amino-benz[1.2.5]thiadiazol

ist in J. Heterocycl. Chem **11**, 777 (1974) beschrieben.

### 5-Amino-1-indanon

ist in J. Org. Chem **27**, 70 (1962) beschrieben.

### 6-Amino-1,2,3,4-tetrahydro-1-naphthalinon

ist in J. Org. Chem **27**, 70 (1962) beschrieben.

### 6-Amino-3,4-dihydro-1H-2-benzopyran-1-on

wird durch katalytische Hydrierung (Palladium/Kohle) in Ethanol aus der entsprechenden Nitroverbindung (Canad. J. Chem. **61**, 2643 (1983) hergestellt.

Die nachfolgenden Beispiele dienen der näheren Erläuterung der Erfindung. Weitere Verbindungen lassen sich durch Verwendung homologer/analoger Reagenzien herstellen. Die erforderlichen Ausgangsverbindungen sind vorstehend unter den "Ausgangsverbindungen" beschrieben.

### Beispiel 1 (Verfahren 1)

### 4-Brom-5-(2-hydroxy-4-methyl-4-phenyl-2-trifluormethyl-valeroylamino)-phthalid

350 mg 4-Brom-5-(4-methyl-2-oxo-4-phenyl-valeroylamino)-phthalid werden unter Argon in 15 ml Dimethylformamid gelöst und unter Eiskühlung mit 0,77 ml Trifluormethyltrimethylsilan und 350 mg Caesiumcarbonat versetzt. Nach 3 Stunden Rühren bei Raumtemperatur werden 5 ml einer 1 M Lösung von Tetrabutylammoniumfluorid in Tetrahydrofuran und einige Tropfen Wasser zugesetzt und eine Stunde bei Raumtemperatur gerührt. Nach Zusatz von 100 ml Wasser wird mit Ethylacetat extrahiert, die organische Phase getrocknet (Na₂SO₄) und eingedampft. Man erhält 250 mg 4-Brom-5-(2-hydroxy-4-methyl-4-phenyl-2-trifluonnethyl-valeroylamino)-phthalid, Fp. 187-194°C.

### Beispiel 2

### 6-Brom-5-(2-hydroxy-4-methyl-4-phenyl-2-trifluormethyl-valeroylamino)-phthalid

wird analog Beispiel 1 aus 1,6 g 6-Brom-5-(4-methyl-2-oxo-4-phenyl-valeroylamino)-phthalid, 3,5 ml Trifluormethyltrimethylsilan und 1,6 g Caesiumcarbonat erhalten, Fp.205-210°C.

### Beispiel 3

### 5-[2-Hydroxy-4-methyl-2-pentafluorethyl-4-phenyl-valeroylamino)-phthalid

wird analog Beispiel 1 aus 20 mg 5-(4-Methyl-2-oxo-4-phenyl-valeroylamino)-phthalid, 0,1 ml Trimethyl-pentafluorethylsilan und 20 mg Caesiumcarbonat erhalten, Fp. 187-189°C.

### Beispiel 4

### 5-[2-Hydroxy-4-(3-methoxyphenyl)-4-methyl-2-trifluormethyl-valeroylamino]-phthalid

wird analog Beispiel 1 aus 30 mg 5-[4-(3-Methoxyphenyl)-4-methyl-2-oxo-valeroylamino]-phthalid. 0.13 ml Trifluormethyltrimethylsilan und 30 mg Caesiumcarbonat erhalten, Fp. 173-178°C.

### Beispiel 5

### 5-(2-Hydroxy-4,4-dimethyl-2-trifluormethyl-5-hexenoylamino)-phthalid

wird analog Beispiel 1 aus 200 mg 5-(4,4- Dimethyl-2-oxo-5-hexenoylamino)-phthalid, 0,22 ml Trifluormethyl-trimethylsilan und 258 mg Caesiumcarbonat erhalten, Fp. 153-157°C.

### Analog Beispiel 1 werden die Verbindungen der Tabelle 7 erhalten

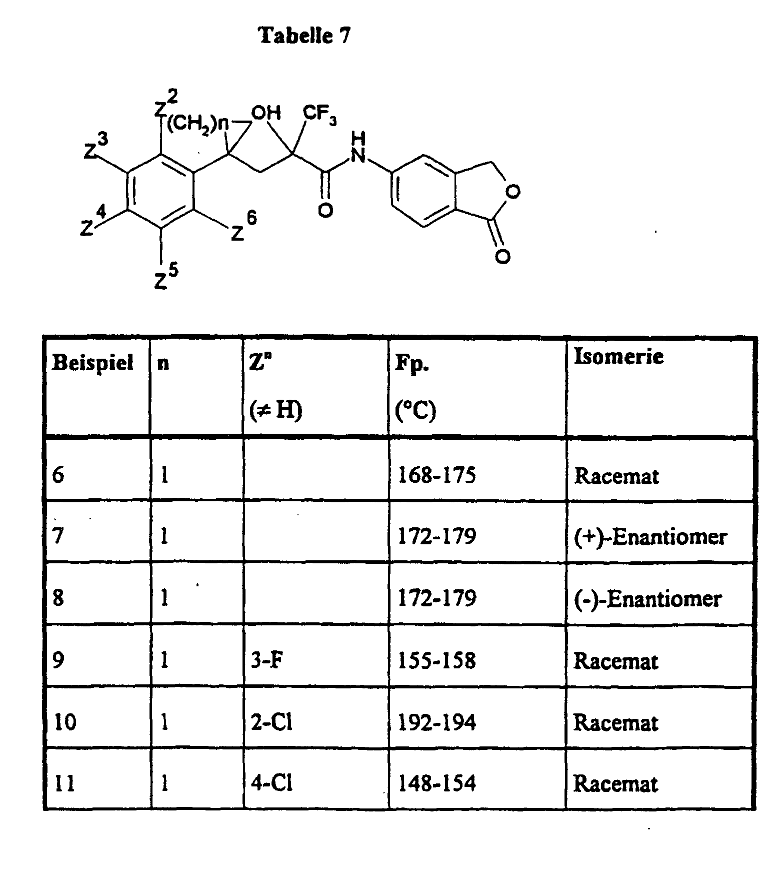

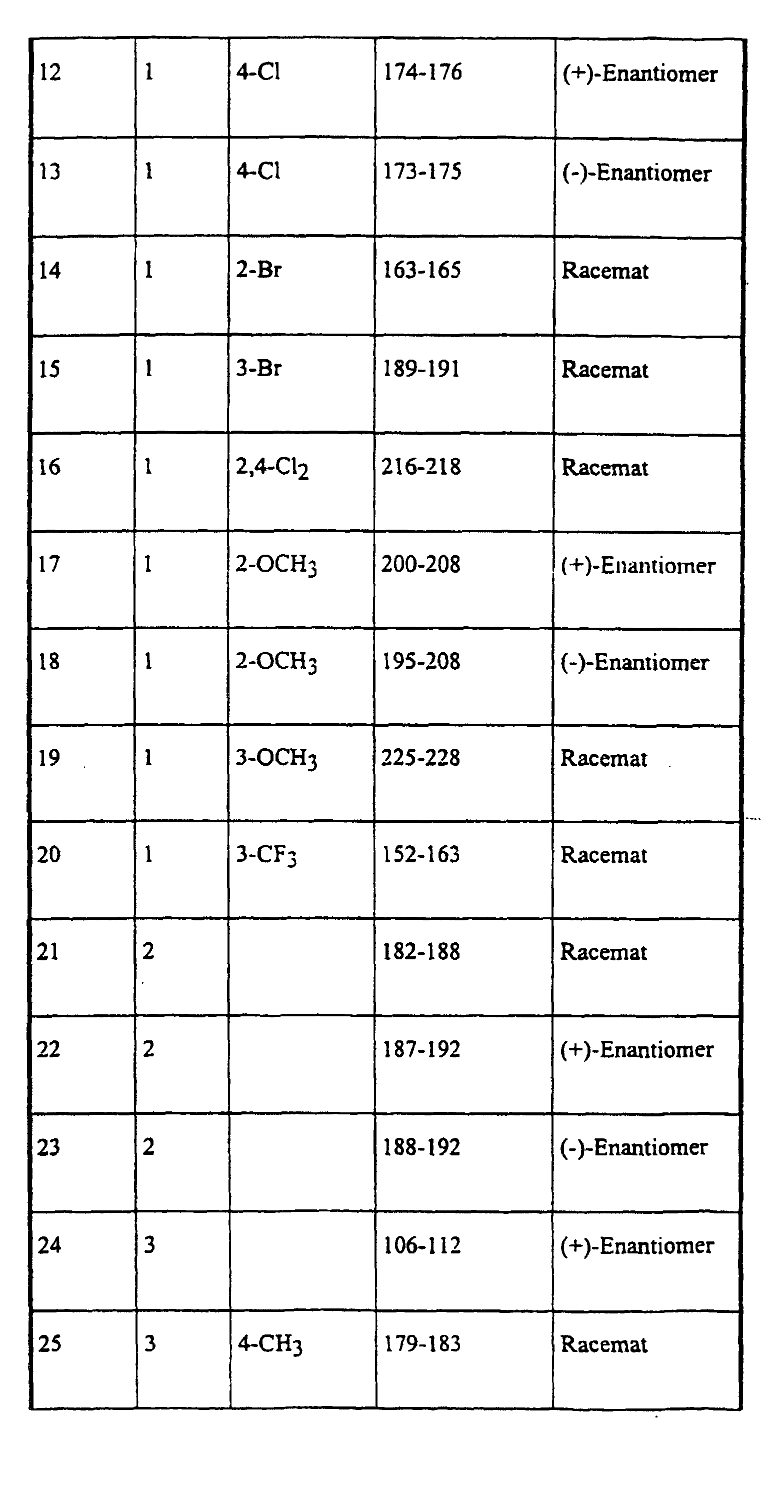

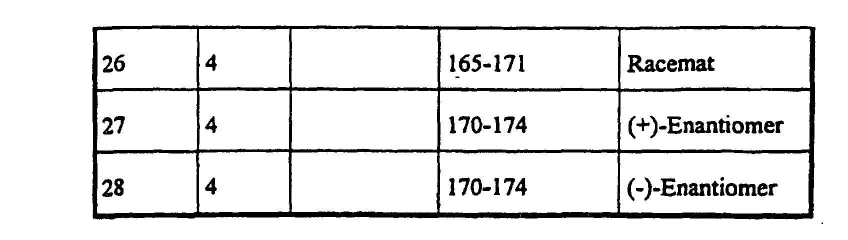

Verwendet man in Beispiel 1 anstelle des Aminophthalids 6-Amino-4-methylbenzoxazinon, so erhält man die in Tabelle 8 zusammengestellten Verbindungen.

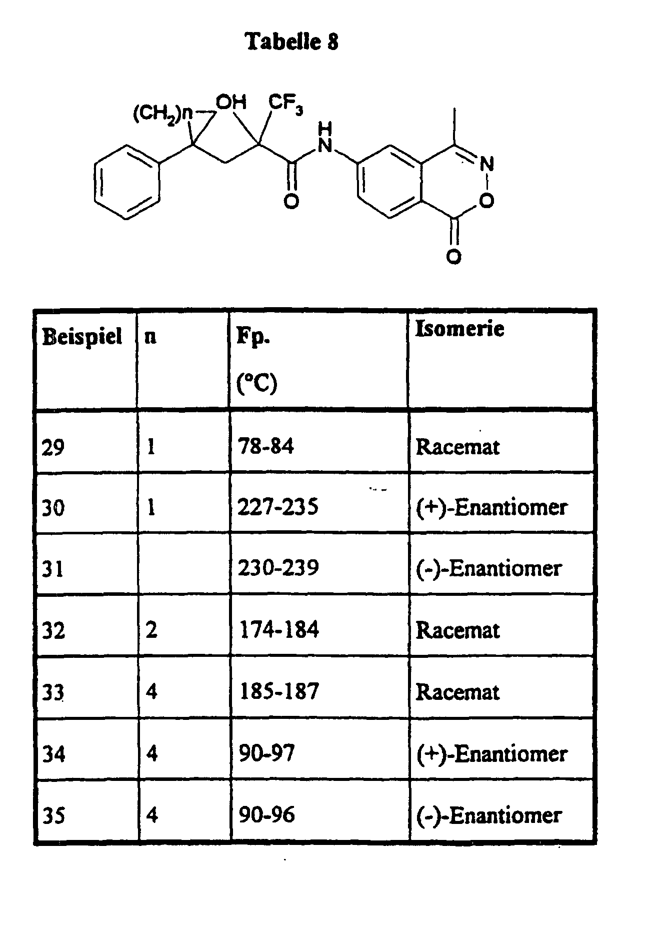

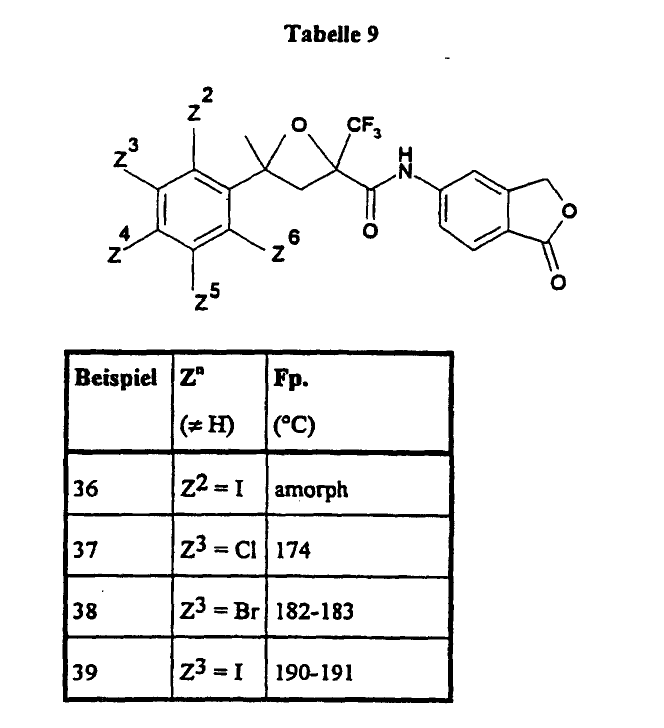

### Beispiel 40

### 6-(2-Hydroxy-2,4-dimethyl-4-phenyl-valeroylamino)-4-methyl-2,3-benzoxazin-1-on

72 mg 6-(4-methyl4-phenyl-2-oxo-valeroylamino)-4-methyl-2,3-benzoxazin-1-on in 4 ml Tetrahydrofuran werdenmit 3 ml Methylmagnesiumbromid (3 molar) bei 0°C versetzt. Nach 30 Minuten wird Ammoniumchloridlösung zugesetzt, die organische Phase abgetrennt, getrocknet und eingedampft.Nach Chromatographie an Kieselgel (Hexan / Ethylacetat 1:1) erhält man 39 mg 6-(2-Hydroxy-2,4-dimethyl4-phenyl-valeroylamino)-4-methyl-2,3-benzoxazin-1-on, Fp. 173-175°C.

### Beispiel 41 (Verfahren 2)

### 5-(2-Hydroxy-4-phenyl-2-trifluormethyl-valeroylamino)-phthalid

500 mg 2-Hydroxy-4-phenyl-2-trifluormethyl-valeriansäure ( Eur. Anm. 0 253 500 (Imperial Chemical Industries)) in 10 ml Dimethylacetamid werden bei -15°C mit 0,14 ml Thionylchlorid versetzt. Nach 3 Stunden Rühren bei -15°C werden 600 mg 5-Aminophthalid (fest) zugegeben.

Die Lösung wird 2 Stunden bei -15°C gerührt und dann 18 Stunden bei Raumtemperatur belassen, danach mit Wasser versetzt und mit Ethylacetat extrahiert. Die Ethylacetat-Phase wird getrocknet (Na₂SO₄) und eingedampft. Der Rückstand wird mit Diethylether verrührt und abgesaugt. Man erhält 290 mg 5-(2-Hydroxy-4-phenyl-2-trifluormethyl-valeroylamino)-phthalid, Fp. 166-168°C.

### Beispiel 42

### 6-(2-Hydroxy-4-phenyl-2-trifluormethyl-valeroylamino)-4-methyl-2,3-benzoxazin-1-on

wird analog Beispiel 41 aus 784 mg 2-Hydroxy-4-phenyl-2-trifluormethyl-valeriansäure in 17 ml Dimethylacetamid und 500 mg 6-Amino-4-methyl-2,3-benzoxazin-1-on erhalten. Fp. 172-173°C.

Analog Beispiel 41 werden die in Tabelle 10 aufgeführten Verbindungen hergestellt:

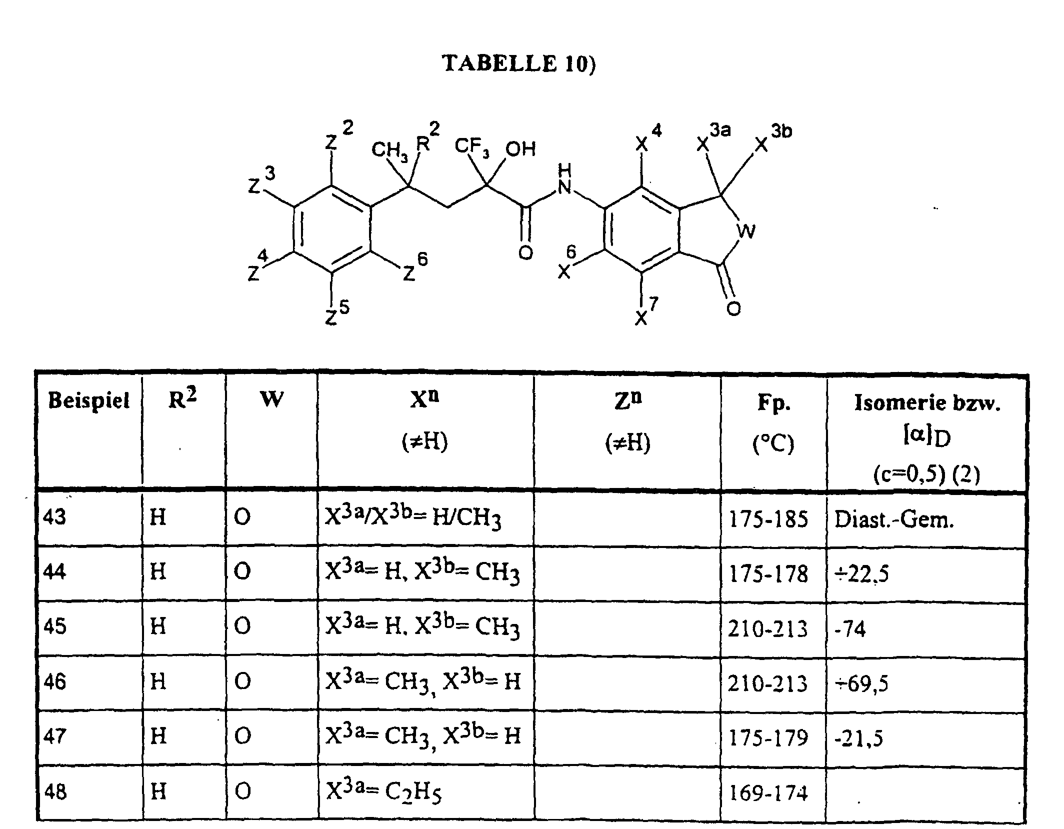

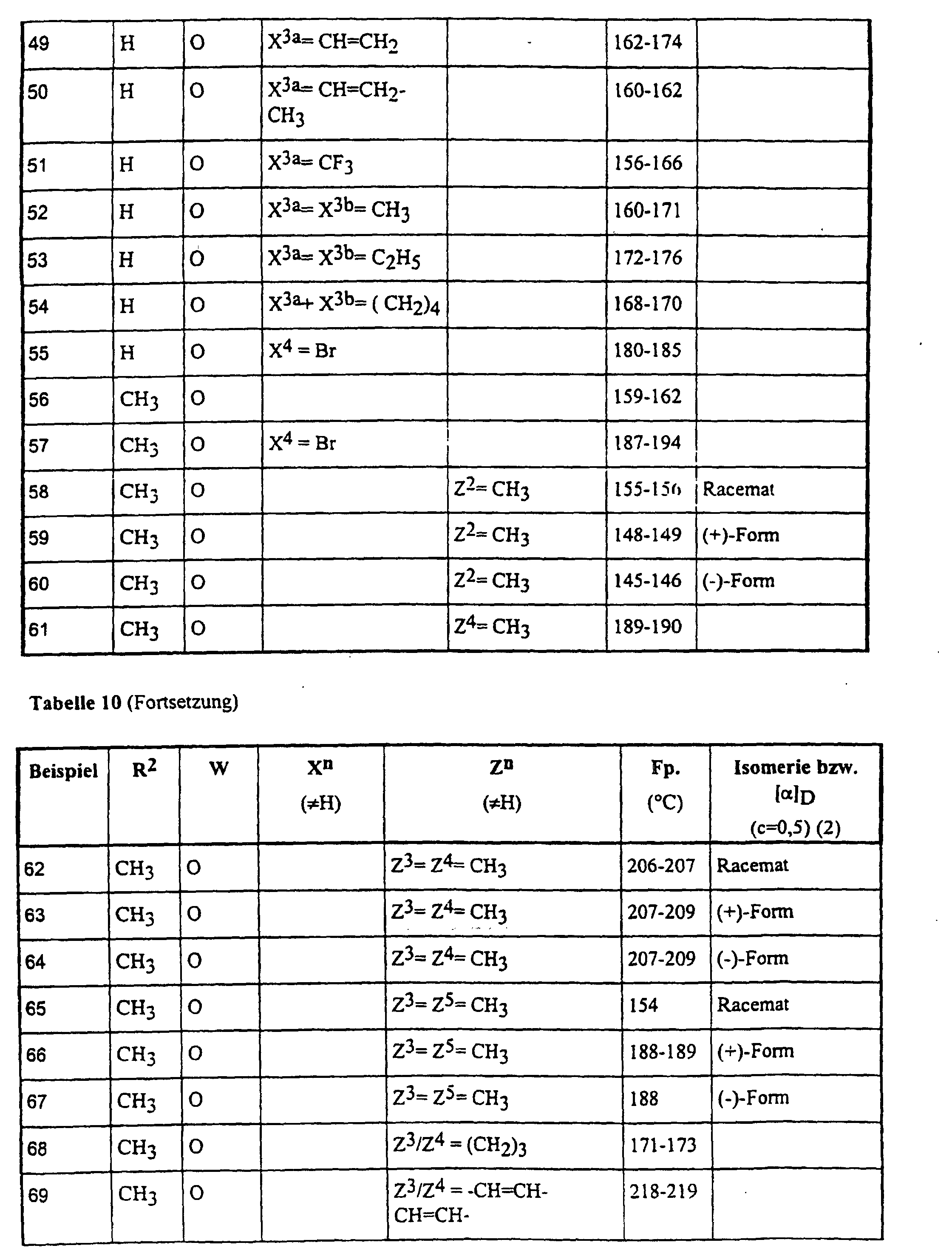

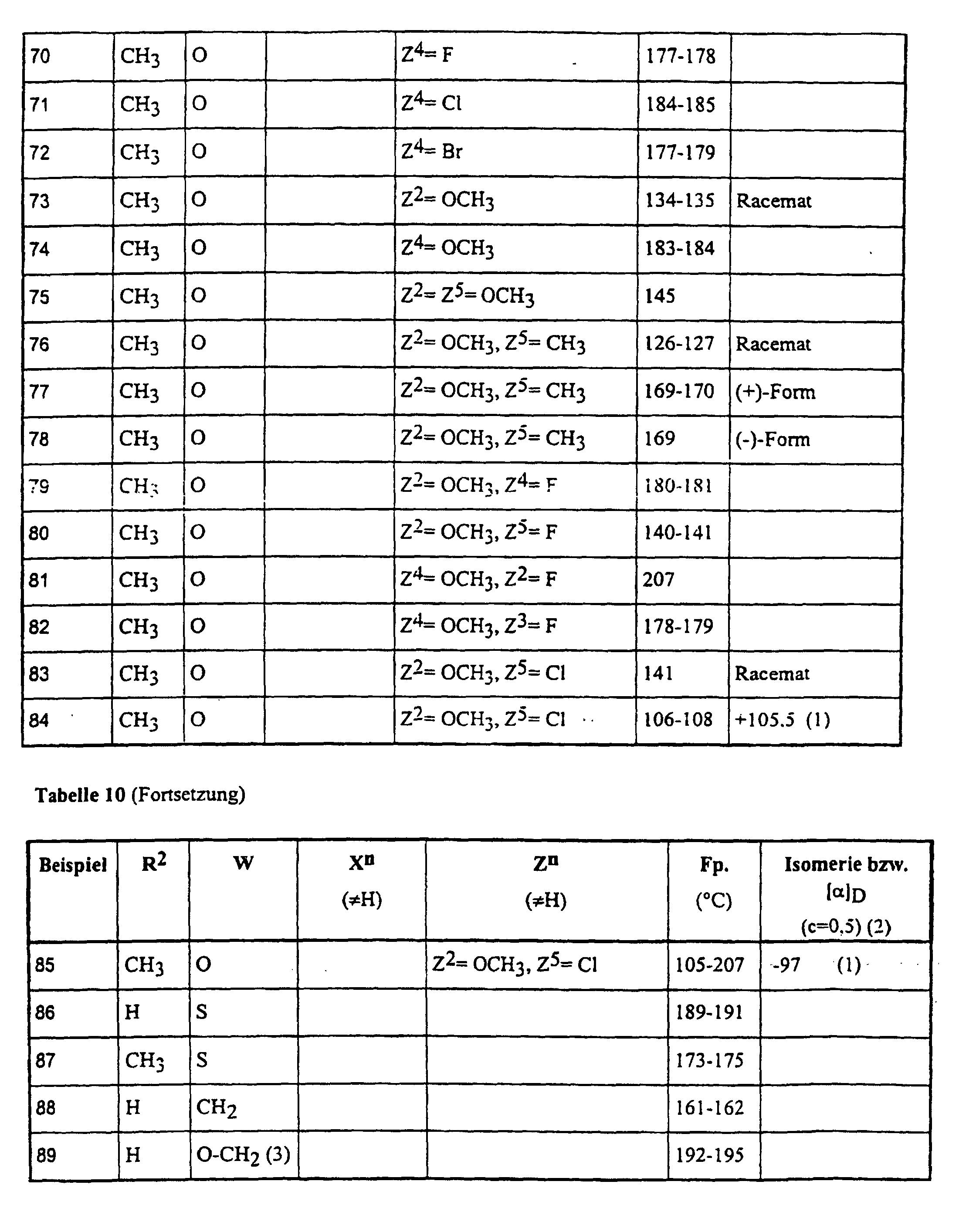

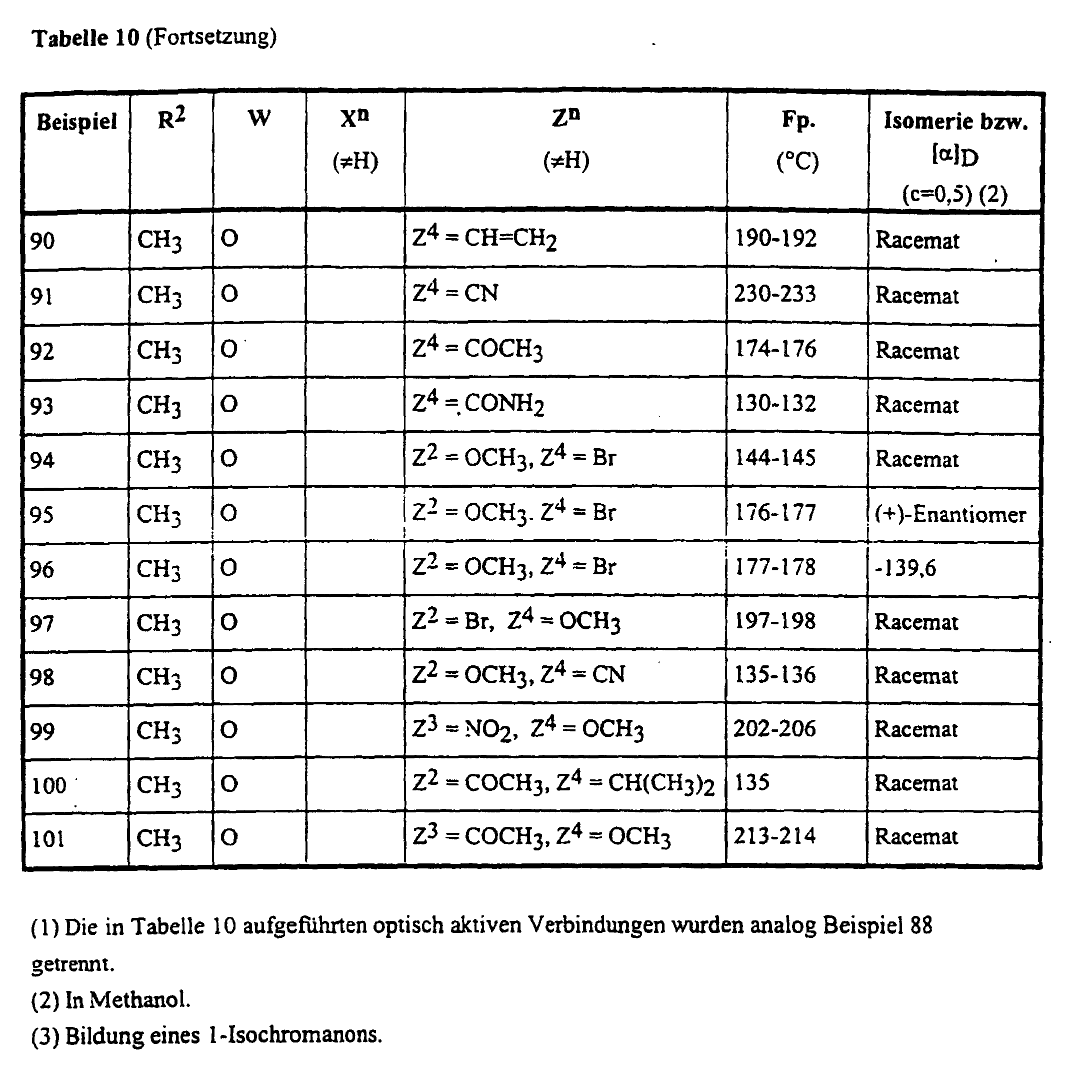

### Beispiel 102

### (+) und (-) 5-[2-Hydroxy-4-methyl-4-(2-methoxyphenyl)-2-trifluormethyl-valeroylamino]-phthalid

Das Enantiomerengemisch aus Beispiel 73 wird durch Chromatographie an chiralem Trägermaterial (CHIRALPAK AD®, Fa DAICEL) mit Hexan/2-Propanol/Ethanol (900:25:25, vvv) getrennt. Man erhält so aus 200 mg Racemat
73 mg (-)-Form, Fp. 136-137°C, α_{D}= -194,8° (c= 0,5 in Chloroform),
59 mg (+)-Form, Fp. 135-136°C, α_{D}= +192,2° (c= 0,5 in Chloroform).

### Beispiel 103

### 5-[2-Hydroxy-4-methyl-4-(2-thienyl)-2-trifluormethyl-valeroylamino]-phthalid und 5-[2-Hydroxy-4-methyl-4-(3-thienyl)-2-trifluormethyl-valeroylamino]-phthalid

Das Gemisch von 2-Hydroxy-4-methyl-4-(2-thienyl)-2-trifluormethyl-valeriansäure und 2-Hydroxy-4-methyl-4-(3-thienyl)-2-trifluormethyl-valeriansäure (9:1) wurde analog Beispiel 41 mit 5-Aminophthalid umgesetzt. Nach chromatographischer Trennung der Positionsisomeren und Racemattrennung analog Beispiel 102 erhält man
(+)-5-[2-Hydroxy-4-methyl-4-(2-thienyl)-2-trifluormethyl-valeroylamino]-phthalid, Fp. 166°C, α_{D}= +163,6° (c= 0,5 in Chloroform), (-)-5-[2-Hydroxy-4-methyl-4-(2-thienyl)-2-trifluormethyl-valeroylamino]-phthalid, Fp. 166°C, α_{D}= -160,8° (c= 0,5 in Chloroform), (+)-5-[2-Hydroxy-4-methyl-4-(3-thienyl)-2-trifluormethyl-valeroylamino]-phthalid, Fp. 135°C und (-)-5-[2-Hydroxy-4-methyl-4-(3-thienyl)-2-trifluormethyl-valeroylamino]-phthalid, Fp. 135°C.

### Beispiel 104 (Verfahren 3)

### 5-(2-Hydroxy-4-phenyl-2-trifluormethyl-pentylamino)-phthalid

760 mg 5-Acetamido-phthalid in 20 ml Dimethylformamid werden bei 0°C mit 144 mg Natriumhydrid (80% in Mineralöl) und nach 20 Minuten mit 800 mg 4-Toluolsulfonsäure-(2-hydroxy-4-phenyl-2-trifluormethyl-pentyl)ester versetzt.Nach 16 Stunden bei 60°C wird das Lösemittel im Vakuum eingedampft, der Rückstand in Ethylacetat gelöst, mit Wasser gewaschen, getrocknet (Na₂SO₄) und eingedampft. Nach Chromatographie an Kieselgel mit Cyclohexan/Ethylacetat (2:1) werden 266 mg 5-(2-Hydroxy-4-phenyl-2-trifluormethylpentylamino)-phthalid erhalten, Fp. 110°C.

Analog Beispiel 104 werden die Verbindungen der Tabelle 11 erhalten.

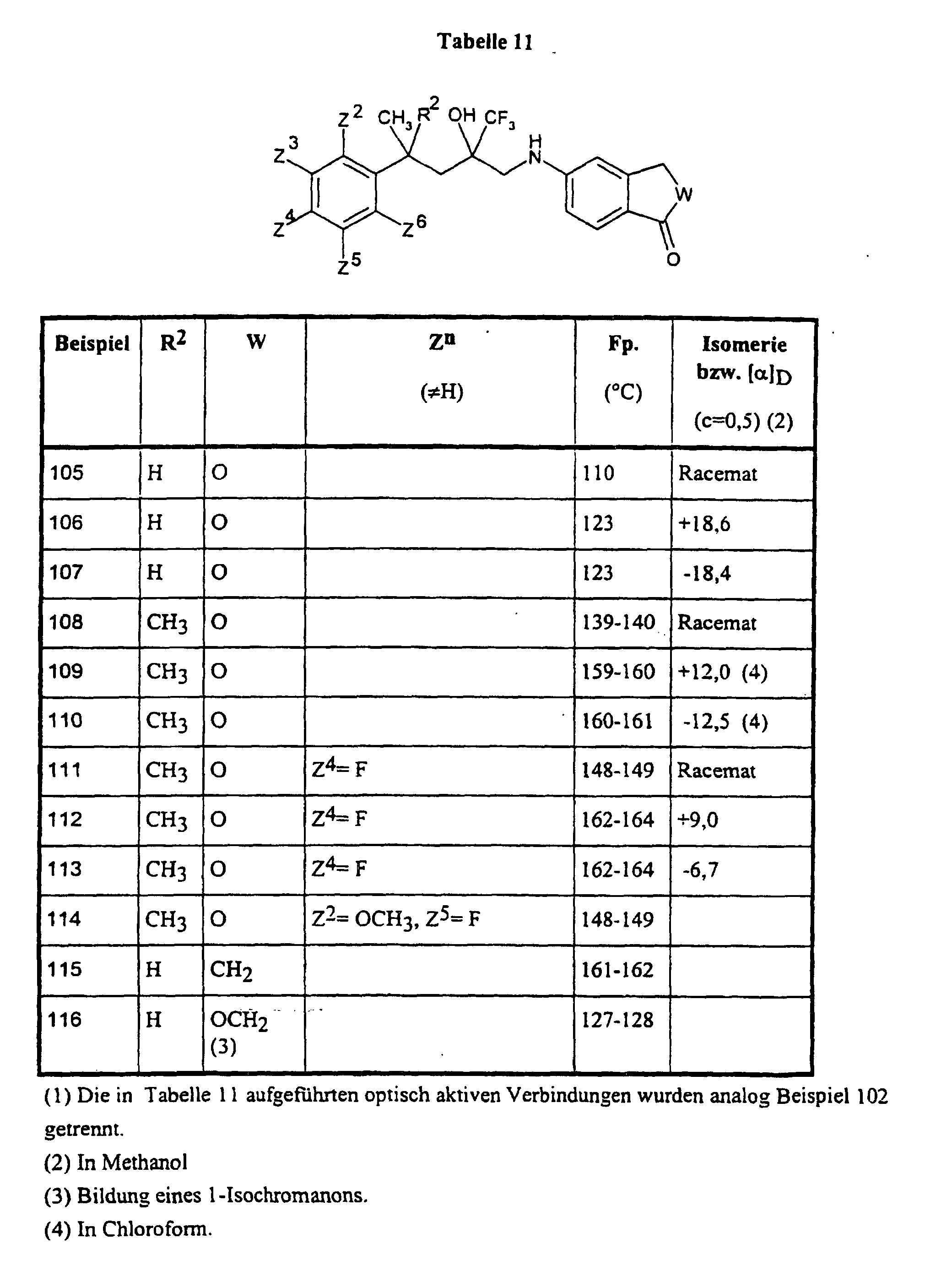

### Beispiel 117

### 4-Ethyl-6-(2-hydroxy-4-phenyl-2-trifluormethyl-pentylamino)-2,3-benzoxazin-1-on

Die Verbindung wird analog Beispiel 104 aus 151 mg 6-Acetamido-4-ethyl-2,3-benzoxazin-2-on, 208 mg 4-Toluolsulfonsäure-(2-hydroxy-4-phenyl-2-trifluormethyl-pentyl)ester und 36 mg Natriumhydrid erhalten, Fp. 161-163°C.

### Beispiel 118

### 1-(4-Nitro-3-trifluormethylanilino)-4-phenyl-2-trifluormethyl-2-pentanol

100 mg 4-Nitro-3-trifluormtethylacetanilid in 2 ml Dimethylformamid werden bei 0°C mit 12 mg Natriumhydrid (80% in Mineralöl) und nach 20 Minuten mit 150 mg 2-(2-Phenylpropyl)-2-trifluormethyl-oxiran versetzt. Man rührt 16 Stunden bei 60°C, verdünnt mit Wasser und extrahiert mit Ethylacetat. Nach Waschen mit Wasser wird die Ethylacetat-Phase getrocknet (Na₂SO₄) und eingedampft. Man erhält 80 mg N-(2-Hydroxy-4-methyl-4-phenyl-2-trifluormethyl-valeroyl)-4-nitro-3-trifluormethylanilin, Fp.119-120°C. Nach Racemattrennung analog Beispiel 102 erhält man
die (-)-Form, [α]_{D}= -49,6° (c= 0,5 in Chloroform),
die (+)-Form, [α]_{D}= +48,8° (c= 0,5 in Chloroform).

### Beispiel 119 (Verfahren 4)

### 6-(3-Hydroxy-3-methyl-1-butinyl)-5-(2-hydroxy-4-methyl-4-phenyl-2-trifluormethylvaleroylamino)-phthalid

150 mg der Bromverbindung aus Beispiel 2 werden zusammen mit 30 mg 2-Methyl-3-butin-2-ol, 0.24 mg Kupfer-I-jodid und 0,9 mg Triphenylphosphin in 1,5 ml Pyridin gelöst und unter Argon mit 0,25 mg Bis-triphenylphosphin-palladium-II-chlorid versetzt. Nach 5 Stunden Kochen am Rückfluß werden weitere 30 mg 2-Methyl-3-butin-2-ol zugegeben und man kocht 20 Stunden im Rückfluß. Man verdünnt mit Wasser und extrahiert mit Ethylacetat. Die Ethylacetat-Phase wird getrocknet (Na₂SO₄) und eingedampft. Das Rohprodukt wird an Kieselgel chromatographiert. Mit Cyclohexan/Ethylacetat (1:1) erhält man 60 mg kristallines 6-(3-Hydroxy-3-methyl-1-butinyl)-5-(2-hydroxy-4-methyl-4-phenyl-2-trifluormethyl-valeroylamino)-phthalid, Fp. 162-168 °C.

### Beispiel 120

### 6-Acetyl-5-(2-hydroxy-4-methyl-4-phenyl-2-trifluormethyl-valeroylamino)-phthalid

100 mg der Bromverbindung aus Beispiel 2 werden zusammen mit 95 mg Tributyl-(1-ethoxyvinyl)-zinn und 8 mg Bis-triphenylphosphin-palladium-II-chlorid in 4 ml Toluol unter Argon gelöst. Nach 5 Stunden Kochen am Rückfluß werden weitere 45 mg Bis-triphenylphosphin-palladium-II-chlorid zugegeben und man kocht 20 Stunden am Rückfluß. Man gibt 1 N Salzsäure zu und extrahiert mit Ethylacetat. Die Ethylacetat-Phase wird getrocknet (Na₂SO₄) und eingedampft. Das Rohprodukt wird in 3 ml Tetrahydrofuran und 3 ml 2 N Salzsäure 2 Tage bei Raumtemperatur gerührt. Man versetzt mit Wasser und extrahiert mit Ethylacetat. Nach Waschen mit Wasser wird getrocknet (Na₂SO₄) und eingedampft. Der Rückstand wird mit Diethylether / Pentan verrieben und man erhält so 21 mg kristallines 6-Acetyl-5-(2-hydroxy-4-methyl-4-phenyl-2-trifluormethyl-valeroylamino)-phthalid, Fp. 195-199°C.

Analog Beispiel 119 und 120 werden die Verbindungen der Tabelle 12 erhalten.

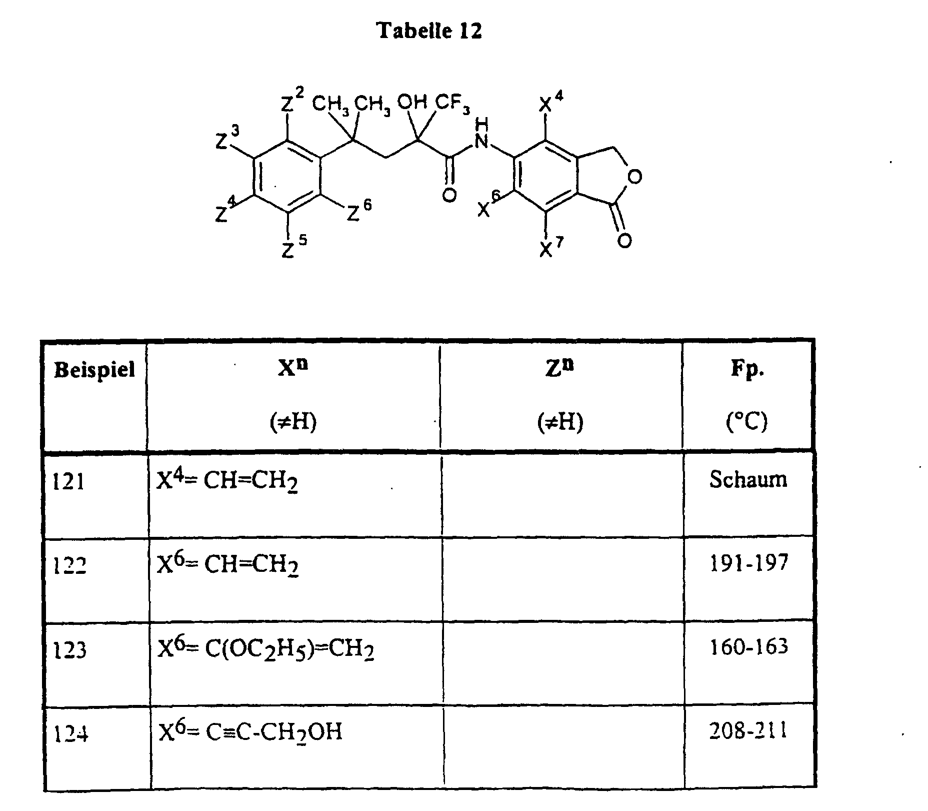

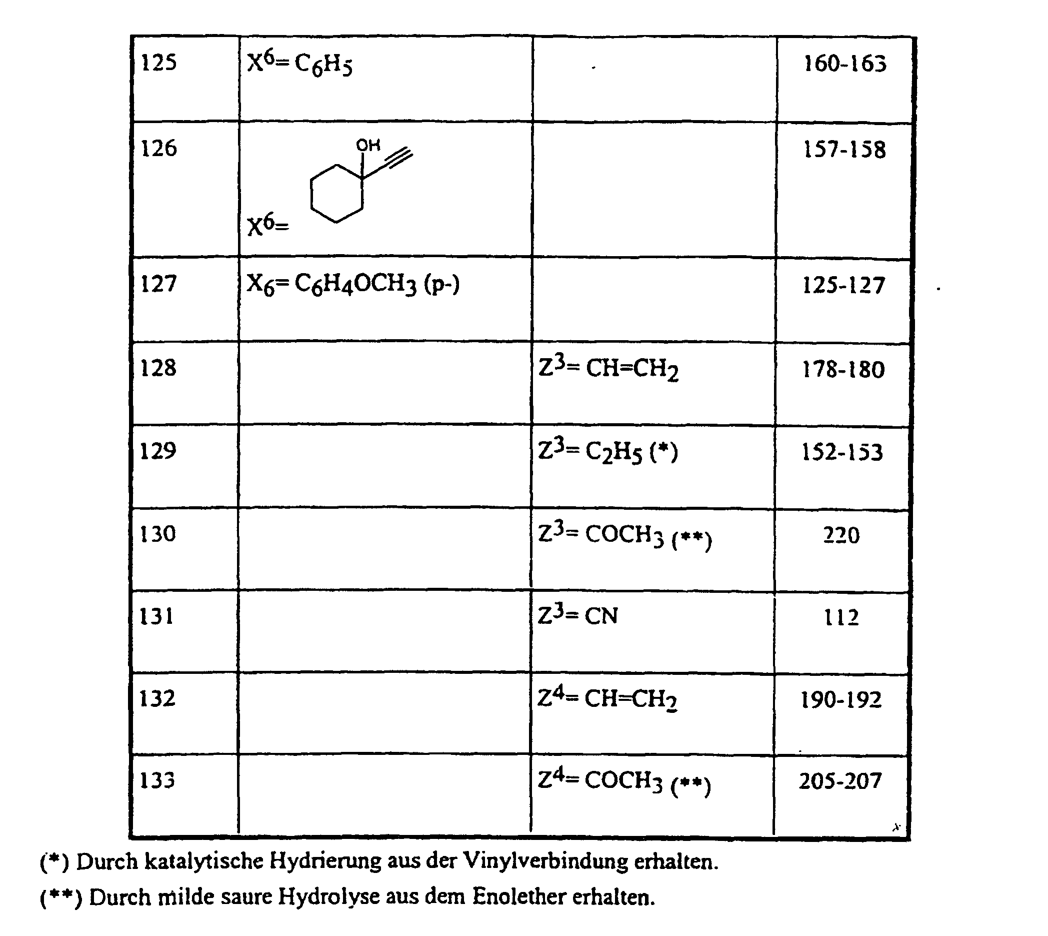

### Beispiel 134 (Verfahren 5)

### 5-[4-(3-Fluor-4-hydroxyphenyl)-2-hydroxy-4-methyl-2-trifluormethyl-valeroylamino]-phthalid

132 mg 5-[4-(3-Fluor-4-methoxyphenyl)-2-hydroxy-4-methyl-4-2-trifluormethylvaleroylamino]-phthalid (Beispiel 82) werden in 15 ml Dichlormethan gelöst und bei 0°C mit 1,2 ml einer 1 M Lösung von Bortribromid in Dichlormethan versetzt. Nach 16 Stunden bei 0°C wird dem Gemisch Eis, Ethylacetat und Kalumbicarbonat zugesetzt und die Ethylacetat-Phase abgetrennt, getrocknet (Na₂SO₄) und eingedampft. Aus Ethylacetat/ Diisopropylether / Hexan erhält man 120 mg 5-[4-(3-Fluor-4-hydroxyphenyl)-2-hydroxy-4-methyl-4-2-trifluormethylvaleroylamino]-phthalid, Fp. 139-140°C.

Analog Beispiel 86 werden die in Tabelle 13 aufgeführten Verbindungen erhalten.

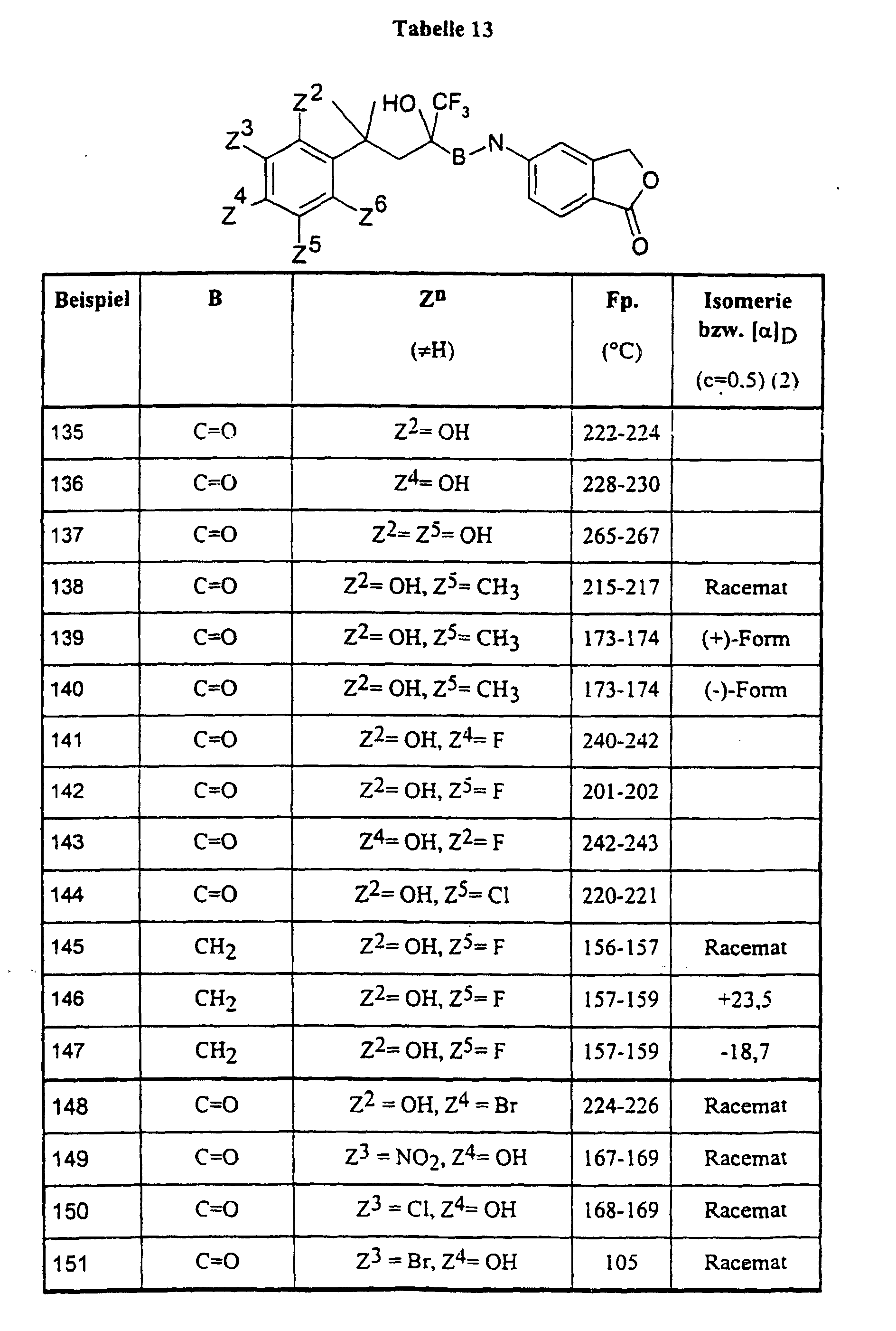

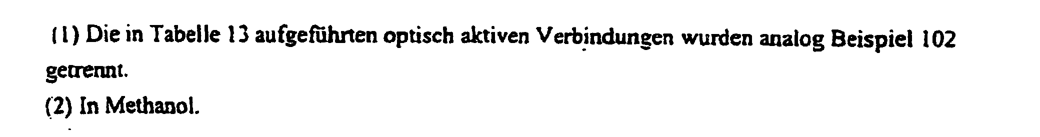

### Beispiel 152

### 5-[4-(3-Fluorphenyl)-2-hydroxy-4-methyl-2-trifluormethyl-valeroylamino]-phthalid

66 mg (3-Fluor-4-hydroxyphenyl)-2-hydroxy-4-methyl-4-2-trifluormethyl-valeroylamino]-phthalid werden mit 126 mg Kaliumcarbonat und 108 mg 5-Chlor-1phenyl-1H-tetratazol in 3 ml Dimethylformamid 16 Stunden gerührt. Dann wird das Dimethylformamid im Vakuum abdestilliert und der Rückstand zwischen 1N Salzsäure und Ethylacetat verteilt. Nach Waschen mit Wasser wird die Ethylacetat-Phase getrocknet (Na₂SO₄) und eingedampft und der Rückstand an Kieselgel mit Hexan /Ethylacetat (1:1) Chromatographiert. Das Produkt wird in 10 ml Methanol mit 30 mg Palladium/Kohle (10%) hydriert. Nach Entfernen des Katalysators und Eindampfen des Lösemittels wird das Produkt an Kieselgel mit Hexan **/** Ethylacetat (1:1) chromatographiert. Man erhält 49 mg 5-[4-(3-Fluorphenyl)-2-hydroxy-4-methyl-4-2-trifluormethyl-valeroylamino]-phthalid, Fp. 157°C.
Durch Racematspaltung analog Beispiel 102 erhält man die (+)-Form mit Fp. 140-141 °C und die (-)-Form mit Fp. 141 °C.

### Beispiel 153

### 5-[2-Hydroxy-4-methyl-4-(3-tolyl)-2-trifluormethyl-valeroylamino]-phthalid

Die Verbindung wird analog dem vorhergehenden Beispiel aus 57 mg 5-[2-Hydroxy-4-(2-hydroxy-5-methylphenyl)-4-methyl-2-trifluormethyl-valeroylamino]-phthalid hergestellt, Fp. 152-153°C.
Durch Racematspattung analog Beispiel 102 erhält man die (+)-Form mit Fp. 148-149°C und die (-)-Form mit Fp. 145-146°C .

### Beispiel 154

### 5-[4-(5-Fluor-2-ethoxyphenyl)-2-hydroxy-4-methyl-4-2-trifluormethyl-valeroylamino]-phthalid

44 mg 5-[4-(5-Fluor-2-hydroxyphenyl)-2-hydroxy-4-methyl-4-2-trifluormethyl-valeroylamino]-phthalid werden in 1 ml Dimethylformamid mit 28 mg Kaliumcarbonat und 50 mg Ethyljodid 24 stunden bei Raumtemperatur gerührt. Man versetzt dann mit Wasser, extrahiert mit Ethylacetat, wäscht die organische Phase mit Wasser, trocknet (Na₂SO₄) und erhält nach Eindampfen des Lösemittels 35 mg 5-[4-(5-Fluor-2-ethoxyphenyl)-2-hydroxy-4-methyl-4-2-trifluormethylvaleroylamino]-phthalid, Fp. 108°C.

### Analog Beispiel 154 wurden die Verbindungen der Tabelle 14 hergestellt

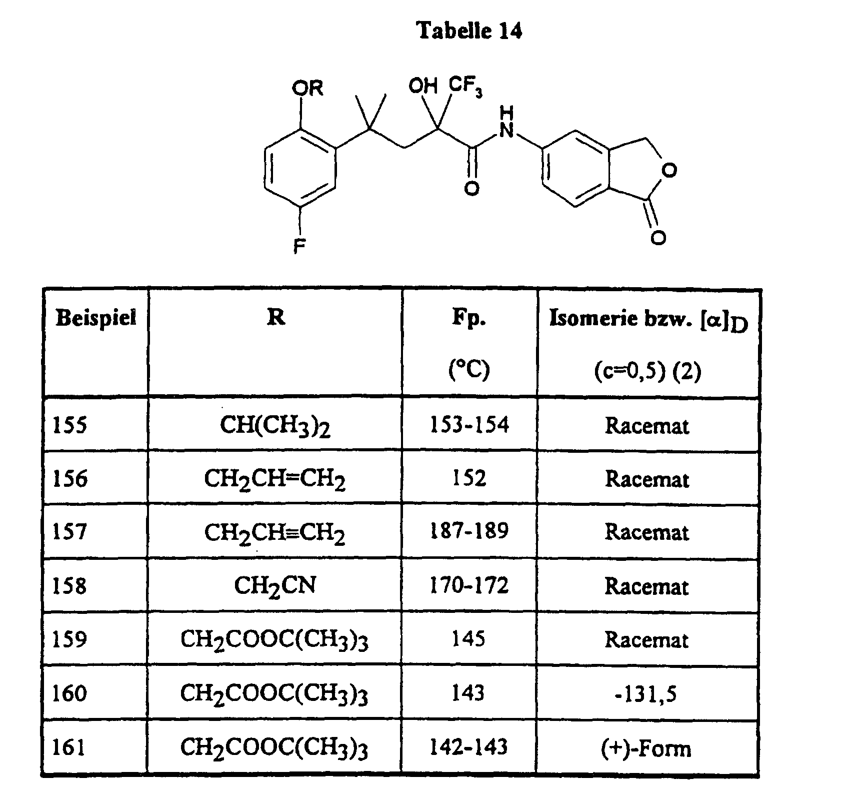

### Beispiel 162

### 5-[4-(3-Chlor-4-methoxyphenyl)-2-hydroxy-4-methyl-2-trifluormethyl-valeroylamino]-phthalid

22 mg 5-[2-Hydroxy-4-(4-methoxyphenyl)-4-methyl-2-trifluormethyl-valeroylamino]-phthalid werden in 1,5 ml Methanol mit 20 mg N-Chlorsuccinimid 5 Stunden gerührt. Man verteilt das Gemisch dann in Eiswasser, Natriumhydrogencarbonatlösung und Ethylacetat, trocknet die Ethylacetatphase und dampt sie ein. So erhält man 20 mg 5-[4-(3-Brom-4-methoxyphenyl)-2-hydroxy-4-methyl-2-trifluormethyl-valeroylamino]-phthalid, das nach Umkristallisation aus Isopropylether bei 189-191°C schmilzt.

### 5-[4-(3-Chlor-4-hydroxyphenyl)-2-hydroxy-4-methyl-2-trifluormethyl-valeroylomino]-phthalid

wird aus 5-[4-(3-Chlor-4-methoxyphenyl)-2-hydroxy-4-methyl-2-trifluormethyl-valeroylamino]-phthalid durch Etherspaltung analog Beispiel 134 erhalten, Fp. 105°C.

Nach den obengenannten Verfahren wurden die 2,3-Benzoxazinon- und Phthalazinonderivate der Tabelle 15 hergestellt.

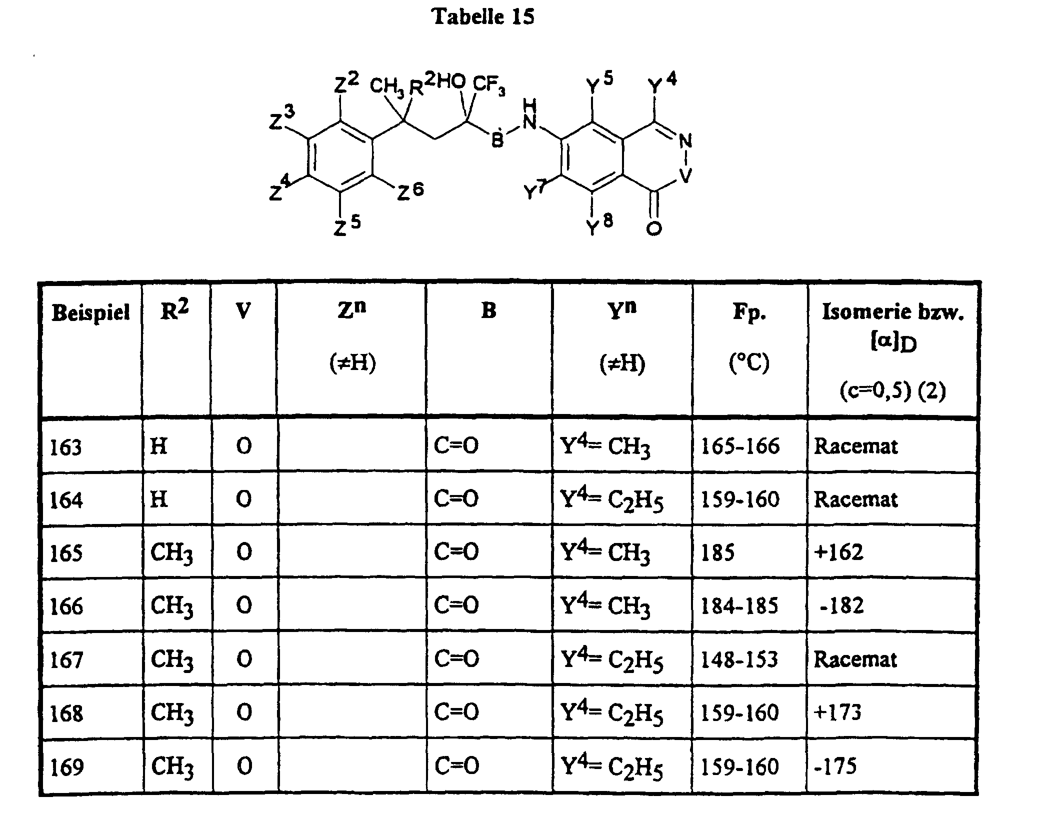

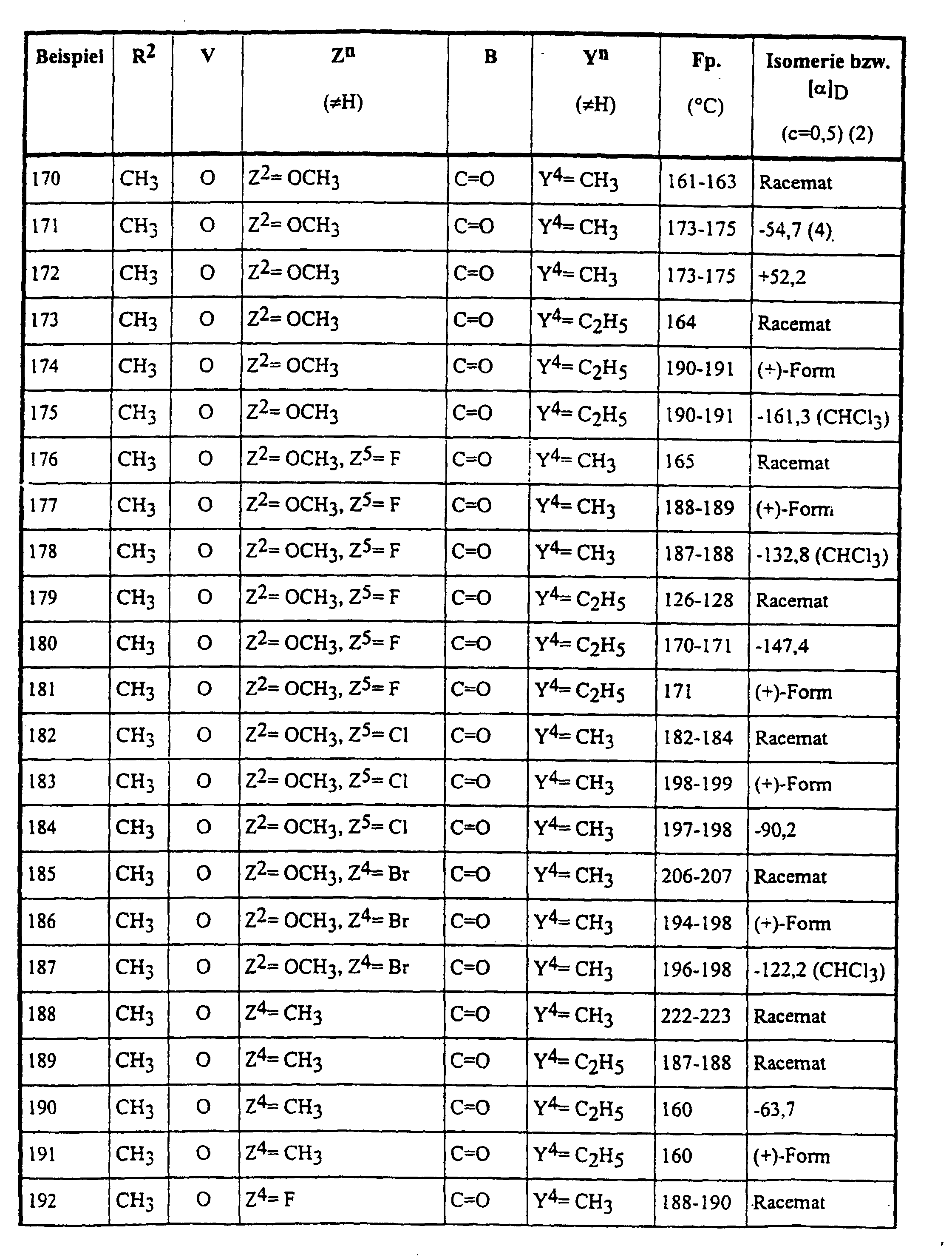

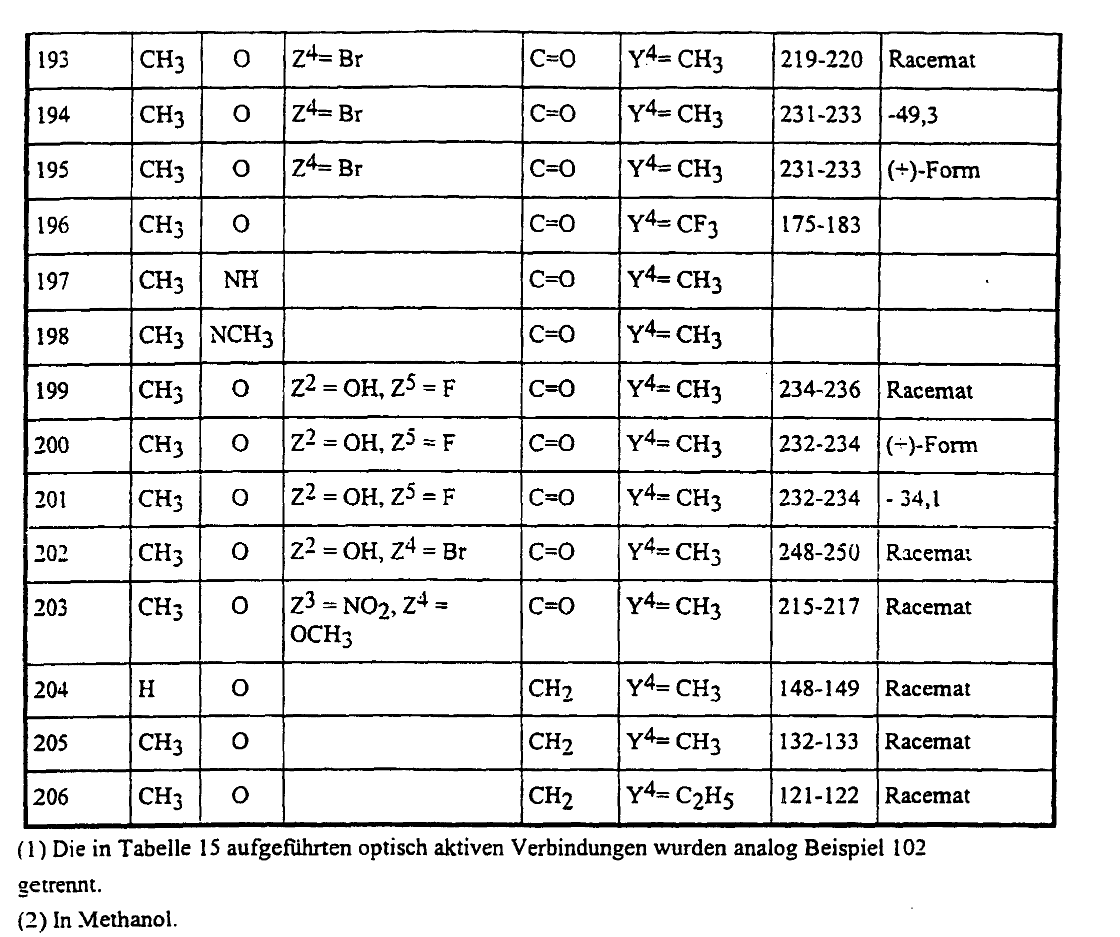

### Beispiel 207

### 5-(2-Hydroxy-4-phenyl-2-trifluormethyl-valeroylamino)-benz[1.2.5)oxadiazol

Die Verbindung wurde analog Beispiel 41 aus 2-Hydroxy-4-phenyl-2-trifluormethylvaleriansäure und 5-Amino-benz[1.2.5]oxadiazol erhalten. Fp. 192°C.

### Beispiel 208

### 5-(2-Hydroxy-4-phenyl-2-trifluormethyl-valeroylamino)-benzo[1.2.5]thiadiazol

Die Verbindung wurde analog Beispiel 41 aus 2-Hydroxy-4-phenyl-2-trifluormethylvaleriansäure und 5-Amino-benzo[1.2.5]thiadiazol erhalten. Fp. 166-167°C.

### Beispiel 209

### 6-(2-Hydroxy-4-phenyl-2-trifluormethyl-valeroylamino)-1-methyl-benzotriazol

Die Verbindung wurde analog Beispiel 41 aus 2-Hydroxy-4-phenyl-2-trifluormethylvaleriansäure und 6-Amino-1-methyl-benzotriazol erhalten. Fp. 194-196°C.

## Patentansprüche

1. Verbindungen der allgemeinen Formel I 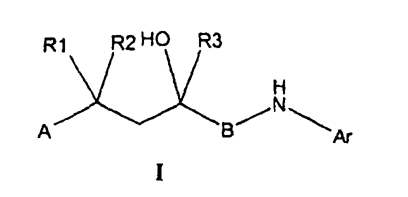 worin
R¹ und R² gleich oder verschieden sind und für ein Wasserstoffatom, eine C₁ -C₅-Alkylgruppe oder ein Halogenatom, ferner gemeinsam mit dem C-Atom der Kette für einen Ring mit insgesamt 3-7 Gliedern,
R³ für eine C₁-C₅ Alkylgruppe oder eine teilweise oder vollständig fluorierte C₁-C₅ Alkylgruppe,
A für einen gegebenenfalls durch einen oder mehrere Reste, ausgewählt aus Halogenatomen, C₁-C₅-Alkylgruppen, C₂-C₅-Alkenylgruppen -CR⁵=CR⁶R⁷, wobei R⁵, R⁶ und R⁷ gleich oder verschieden sind und unabhängig voneinander Wasserstoffatome oder C₁-C₅ Alkylgruppen bedeuten, Hydroxygruppen, Hydroxygruppen, die eine C₁-C₁₀-Acylgruppe, eine C₃-C₁₀-Carbalkoxyalkylgruppe, eine C₂-C₅-Cyanalkylgruppe, eine C₃-C₁₀ unsubstituierte oder substituierte Allylgruppe, eine C₃-C₁₀ unsubstituierte oder substituierte Propargylgruppe, eine C₂-C₅-Alkoxyalkylgruppe, eine teilweise oder vollständig durch Fluoratome substituierte C₁-C₅-Alkylgruppe tragen, der Cyan- oder Nitrogruppe, C₁-C₅-Alkoxygruppen, C₁-C₅-Alkylthiogruppen, mono- oder disubstituierten C₁-C₁₀ Aminogruppen oder teilweise oder vollständig fluorierten C₁-C₅ Alkylgruppen substituierten mono- oder bicyclischen carbocyclischen oder heterocyclischen aromatischen Ring,
B für eine Carbonyl- oder eine CH₂-Gruppe und
Ar für ein Ringsystem, ausgewählt aus der Gruppe der allgemeinen Teilformeln **2-11**, stehen. 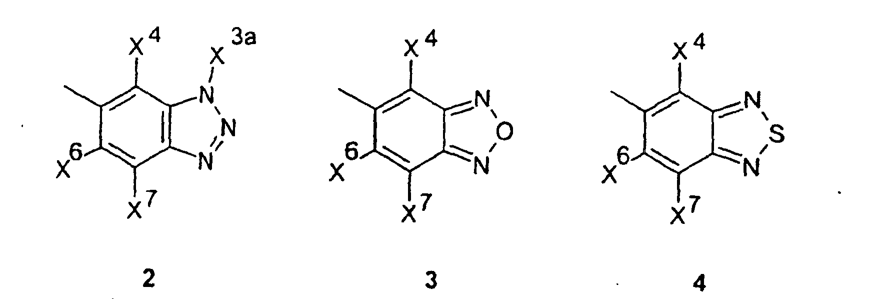 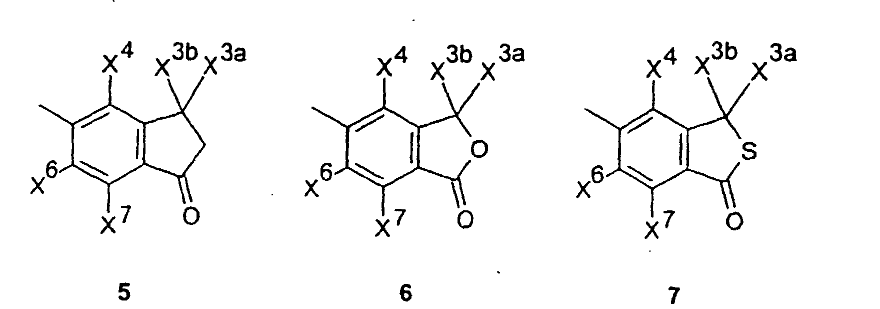 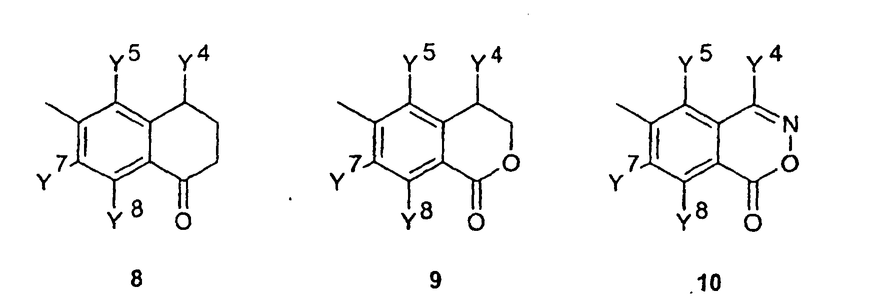 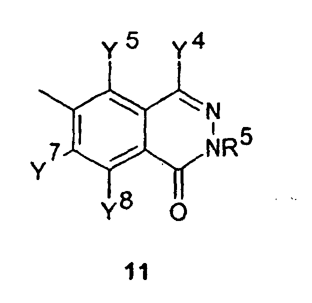 worin
die Reste X^{3a}, X⁴, X⁶, X⁷ (in der Teilformel **2**), X⁴, X⁶, X⁷ (in den Teilformeln **3** und **4**), X^{3a}, X^{3b}, X⁴, X⁶, X⁷ (in den Teilformeln **5**, **6** und **7**) oder Y⁴, Y⁵, Y⁷, Y⁸ (in den Teilformeln **8**, **9**, **10** und **11**) gleich oder verschieden und ausgewählt sind aus Wasserstoffatomen, C₁-C₅-Alkylgruppen, die zusätzlich noch eine gegebenenfalls mit einer C₁-C₅-Alkylgruppe veretherte oder mit einer C₁-C₅-Alkanoylgruppe veresterte Hydroxygruppe enthalten können, teilweise oder vollständig fluorierten C₁-C₅ Alkylgruppen, C₂-C₅-Alkenylgruppen -CR⁵=CR⁶R⁷, wobei R⁵, R⁶ und R⁷ die oben genannte Bedeutung haben. Alkinylgruppen -C≡CR⁵, wobei R⁵ die oben genannte Bedeutung hat,
die Reste X^{3a} und X^{3b} ferner gemeinsam mit dem C-Atom des benzokondensierten Ringsystems 5, 6 oder 7 einen Ring mit insgesamt 3-7 Gliedern bilden können sowie darüber hinaus die Reste X⁴, X⁶, X⁷ (in den Teilformeln **2**, **3**, **4**, **5**, **6** und **7**) oder Y⁴, Y⁵, Y⁷, Y⁸ (in den Teilfonneln **8**, **9**, **10** und **11**) ausgewählt sind aus Halogenatomen, Hydroxygruppen, C₁-C₅-Alkoxygruppen oder C₁-C₅-Alkanoylgruppen, sowie für den Fall,daß B für eine CH₂-Gruppe steht, die physiologisch verträglichen Salze der Verbindungen der allgemeinen Formel I mit Säuren.

2. Verbindungen der allgemeinen Formel I nach Anspruch 1 in Form des Racemats oder Diastereomerengemisches.

3. Verbindungen der allgemeinen Formel I nach Anspruch 1 in Form der getrennten optischen Isomeren.

4. Verbindungen der allgemeinen Formel I nach Anspruch 1, **dadurch gekennzeichnet, daß** R¹ und R² gleich oder verschieden sind und für ein Wasserstoffatom, eine Methyl- oder Ethylgruppe, ferner gemeinsam mit dem C-Atom der Kette für einen Cyclopropylring stehen.

5. Verbindungen der allgemeinen Formel I nach Anspruch 1, **dadurch gekennzeichnet, daß** R³ für eine C₁-C₅-Perfluoralkylgruppe steht.

6. Verbindungen der allgemeinen Formel I nach Anspruch 1, **dadurch gekennzeichnet, daß** A für einen gegebenenfalls durch einen oder mehrere Reste, ausgewählt aus Fluoratomen, Chloratomen, Bromatomen, Methylgruppen, Ethylegruppen, Vinylgruppen, Hydroxygruppen, Methoxygruppen, Ethoxygruppen substituierten Benzol-, Naphthalin- oder Thiophenring steht.

7. Verbindungen der allgemeinen Formel I nach Anspruch 1, **dadurch gekennzeichnet, daß** X^{3a} für ein Wasserstoffatom oder eine C₁-C₅-Alkylgruppe steht.

8. Verbindungen der allgemeinen Formel I nach Anspruch 1, **dadurch gekennzeichnet, daß** X^{3a} und X^{3b} gleich oder verschieden sind und für ein Wasserstoffatom oder eine C₁-C₅-Alkylgruppe stehen.

9. Verbindungen der allgemeinen Formel I nach Anspruch 1, **dadurch gekennzeichnet, daß** X⁴, X⁶ und X⁷ gleich oder verschieden sind und unabhängig voneinander für ein Wasserstoffatom oder ein Halogenatom stehen.

10. Verbindungen der allgemeinen Formel I nach Anspruch 1, **dadurch gekennzeichnet, daß** Y⁴ für eine C₁-C₅-Alkylgruppe oder eine C₁-C₅ Perfluoralkylgruppe steht.

11. Verbindungen der allgemeinen Formel I nach Anspruch 1, **dadurch gekennzeichnet, daß** Y⁵, Y⁷ und Y⁸ gleich oder verschieden sind und unabhängig voneinander für ein Wasserstoffatom oder ein Halogenatom stehen.

12. Verbindungen der allgemeinen Formel I nach Anspruch 1, **dadurch gekennzeichnet, daß** R¹ und R² gleich oder verschieden sind und für ein Wasserstoffatom, eine Methyl- oder Ethylgruppe, ferner gemeinsam mit dem C-Atom der Kette für einen Cyclopropylring, und R³ für eine C₁-C₅ Perfluoralkylgruppe, A für einen gegebenenfalls durch einen oder mehrere Reste, ausgewählt aus Fluoratomen, Chloratomen, Bromatomen, Methylgruppen, Ethylgruppen, Vinylgruppen, Hydroxygruppen, Methoxygruppen, Ethoxygruppen substituierten Benzol-, Naphthalin- oder Thiophenring und entweder X^{3a} für ein Wasserstoffatom oder eine C₁-C₅-Alkylgruppe oder X^{3a} und X^{3b} gleich oder verschieden sind und für ein Wasserstoffatom oder eine C₁-C₅-Alkylgruppe, und X⁴, X⁶ und X⁷ gleich oder verschieden sind und unabhängig voneinander für ein Wasserstoffatom oder ein Halogenatom oder Y⁴ für eine C₁-C₅-Alkylgruppe oder eine C₁-C₅ Perfluoralkylgruppe und Y⁵, Y⁷ und Y⁸ gleich oder verschieden sind und unabhängig voneinander für ein Wasserstoffatom oder ein Halogenatom stehen, und die anderen Substituenten alle in der Formel 1 angegebenen Bedeutungen haben.

13. Verbindungen der allgemeinen Formel I nach Anspruch 1, worin Ar für ein Ringsystem der Teilformel **6** steht.

14. Verbindungen der allgemeinen Formel I nach Anspruch 1, worin Ar für ein Ringsystem der Teilformel **7** steht.

15. Verbindungen der allgemeinen Formel I nach Anspruch 1, worin Ar für ein Ringsystem der Teilformel **10** steht.

16. Verbindungen der allgemeinen Formel I nach Anspruch 1, worin Ar für ein Ringsystem der Teilformel **11** steht.

17. Verbindungen der allgemeinen Formel I nach Anspruch 1, nämlich
4-Brom-5-(2-hydroxy-4-methyl-4-phenyl-2-trifluormethyl-valeroylamino)-phthalid,
6-Brom-5-(2-hydroxy-4-methyl-4-phenyl-2-trifluormethyl-valeroylamino)-phthalid,
5-(2-Hydroxy-4-methyl-2-pentafluorethyl-4-phenyl-valeroylamino)-phthalid,
3-[2-Hydroxy-4-(3-methoxyphenyl)-4-methyl-2-trifluormethyl-valeroylamino]-phthalid,
5-[2-Hydroxy-4-(4-methoxyphenyl)-4-methyl-2-trifluormethyl-valeroylamino]-phthalid,
5-[2-Hydroxy-4-(2-hydroxyphenyl)-4-methyl-2-trifluormethyl-valeroylamino]-phthalid,
S-[4-(2-Fluorphenyl)-2-hydroxy-4-methyl-2-trifluormethyl-valeroylamino]-phthalid,
5-[4-(4-Fluorphenyl)-2-hydroxy-4-methyl-2-trifluormethyl-valeroylamino]-phthalid,
5-[4-(4-Chlorphenyl)-2-hydroxy-4-methyl-2-trifluormethyl-valeroylamino]-phthalid,
5-[4-(4-Bromphenyl)-2-hydroxy-4-methyl-2-trifluormethyl-valeroylamino]-phthalid,
5-[2-Hydroxy-4-methyl-4-(4-tolyl)-2-trifluormethyl-valeroylamino]-phthalid,
5-[2-Hydroxy-4-methyl-4-(3-tolyl)-2-trifluormethyl-valeroylamino]-phthalid,
5-[4-(4-Cyanphenyl)-2-hydroxy-4-methyl-2-trifluormethyl-valeroylamino]-phthalid,
5-[4-(3,4-Dimethylphenyl)-2-hydroxy-4-methyl-2-trifluormethyl-valeroylamino]-phthalid,
5-[4-(3,5-Dimethylphenyl)-2-hydroxy-4-methyl-2-trifluormethyl-valeroylamino]-phthalid,
5-[2-Hydroxy-4-(2-methoxy-5-methylyphenyl)-4-methyl-2-trifluormethyl-valeroylamino]-phthalid,
5-[4-(5-Chlor-2-hydroxyphenyl)-2-hydroxy-4-methyl-2-trifluormethyl-valeroylamino]-phthalid,
5-[4-(5-Fluor-2-hydroxyphenyl)-2-hydroxy-4-methyl-2-trifluormethyl-valeroylamino]-phthalid,
5-(2-Hydroxy-4-(2-Hydroxy-5-methylphenyl)-4-methyl-2-trifluormethyl-valeroylamino]-phthalid,
5-[4-(5-Fluor-2-methoxyphenyl)-2-hydroxy-4-methyl-3-trifluormethyl-valeroylamino]-phthalid,
5-[4-(2-Fluor-4-methoxyphenyl)-2-hydroxy-4-methyl-2-trifluormethyl-valeroylamino]-phthalid,
5-[4-(3-Fluor-4-methoxyphenyl)-2-hydroxy-4-methyl-2-trifluormethyl-valeroylamino]-phthalid,
3-(2-Hydroxy-4-phenyl-2-trifluormethyl-valeroylamino)-phthalid,
5-[2-Hydroxy-4-(2-methoxyphenyl)-4-methyl-2-trifluormethyl-valeroylamino]-phthalid,
5-[4-(5-Chlor-2-Methoxyphenyl)-2-hydroxy-4-methyl-2-trifluormethyl-valeroylamino]-phthalid,
5-(2-Hydroxy-4-phenyl-2-trifluormethyl-pentylamino)-phthalid,
5-(2-Hydroxy-4-methyl-4-phenyl-2-trifluormethyl-pentylamino)-phthalid,
5-[4-(4-Fluorphenyl)-2-hydroxy-4-methyl-2-trifluormethyl-pentylamino]-phthalid,
5-[4-(5-Fluor-2-hydroxyphenyl)-2-hydroxy-4-methyl-2-trifluormethyl-pentylamino]-phthalid,
6-Acetyl-5-(2-hydroxy-4-methyl-4-phenyl-2-trifluormethyl-valeroylamino)-phthalid,
5-[4-(3-Fluor-4-hydroxyphenyl)-2-hydroxy-4-methyl-2-trifluormethyl-valeroylamino]-phthalid,
5-[4-(3-Fluorphenyl)-2-hydroxy-4-methyl-2-trifluormethyl-valeroylamino]-phthalid,
6-(3-Hydroxy-3-methyl-1-butinyl)-5-(2-hydroxy-4-methyl-4-phenyl-2-trifluormethyl-valeroylamino)-phthalid,
6-(2-Hydroxy-4-methyl-4-phenyl-2-trifluormethyl-valeroylamino)-4-methyl-2,3-benzoxazin-1-on,
6-(2-Hydroxy-4-methyl-4-phenyl-3-trifluormethyl-valeroylamino)-4-trifluormethyl-2,3-benzoxazin-1-on,
4-Ethyl-6-(2-hydroxy-4-phenyl-2-trifluormethyl-pentylamino)-2,3-benzoxazin-1-on,
4-Ethyl-6-[2-hydroxy-4-(2-methoxyphenyl)-4-methyl-2-trifluormethyl-valeroylamino]-2,3-benzoxazin-1-on,
6-[2-Hydroxy-4-(2-methoxyphenyl)-4-methyl-2-trifluormethyl-valeroylamino]-4-methyl-2,3-benzoxazin-1-on,
4-Ethyl-6-[2-hydroxy-4-methyl-4-(4-methylphenyl)-2-trifluormethyl-valeroylamino]-2,3-benzoxazin-1-on,
6-[4-(4-Bromphenyl)-2-hydroxy-4-methyl-2-trifluormethyl-valeroylamino]-4-ethyl-2,3-benzoxazin-1-on,
4-Ethyl-6-[4-(5-fluor-2-methoxyphenyl)-2-hydroxy-4-methyl-2-trifluormethyl-valeroylamino)-2,3-benzoxazin-1-on,
6-[4-(5-Fluor-2-methoxyphenyl)-2-hydroxy-4-methyl-2-trifluormethyl-valeroylamino)-4-methyl-2,3-benzoxazin-1-on,
1-(4-Nitro-3-trifluormethylanilino)-4-phenyl-2-trifluormethyl-2-pentanol,
5-(2-Hydroxy-4,4-dimethyl-2-trifluormethyl-5-hexenoylamino)-phthalid,
5-[2-Hydroxy-3-(1-phenyl-cyclopropyl)-2-trifluormethyl-propionylamino]-phthalid,
5-[2-Hydroxy-3-(1-phenyl-cyclobutyl)-2-trifluormethyl-propionylamino]-phthalid,
5-[2-Hydroxy-3-(1-phenyl-cyclohexyl)-2-trifluormethyl-propionylamino]-phthalid,
6-(2-Hydroxy-2,4-dimethyl4-phenyl-valeroylamino)-4-methyl-2,3-benzoxazin-1-on,
5-[4-(3-Chlor-4-methoxyphenyl)-2-hydroxy-4-methyl-2-trifluormethyl-valeroylamino]-phthalid.

18. Verbindungen der allgemeinen Formel I nach Anspruch 1, nämlich die Verbindungen 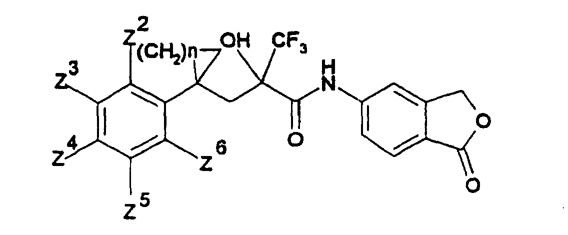
| **n** | **Z**^{**n**} (≠ H) | **Isomerie** |
|---|---|---|
| 1 | | Racemat |
| 1 | | (+)-Enantiomer |
| 1 | | (-)-Enantiomer |
| 1 | 3-F | Racemat |
| 1 | 2-Cl | Racemat |
| 1 | 4-Cl | Racemat |
| 1 | 4-Cl | (+)-Enantiomer |
| 1 | 4-Cl | (-)-Enantiomer |
| 1 | 2-Br | Racemat |
| 1 | 3-Br | Racemat |
| 1 | 2,4-Cl₂ | Racemat |
| 1 | 2-OCH₃ | (+)-Enantiomer |
| 1 | 2-OCH₃ | (-)-Enantiomer |
| 1 | 3-OCH₃ | Racemat |
| 1 | 3-CF₃ | Racemat |
| 2 | | Racemat |
| 2 | | (+)-Enantiomer |
| 2 | | (-)-Enantiomer |
| 3 | | (+)-Enantiomer |
| 3 | 4-CH₃ | Racemat |
| 4 | | Racemat |
| 4 | | (+)-Enantiomer |
| 4 | | (-)-Enantiomer |

19. Verbindungen der allgemeinen Formel nach Anspruch 1, nämlich die Verbindungen 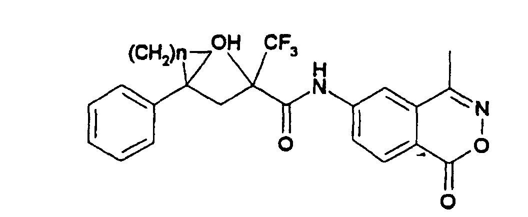
| **n** | **Isomerie** |
|---|---|
| 1 | Racemat |
| 1 | (+)-Enantiomer |
| | (-)-Enantiomer |
| 2 | Racemat |
| 4 | Racemat |
| 4 | (+)-Enantiomer |
| 4 | (-)-Enantiomer |

20. Verbindungen der allgemeinen Formel nach Anspruch 1, nämlich die Verbindungen 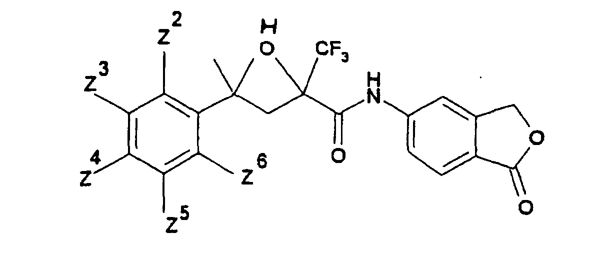
| _{**Z**}**n** (≠ **H)** |
|---|
| Z²=I |
| Z³ = Cl |
| Z³ = Br |
| Z³=I |

21. Verbindungen der allgemeinen Formel nach Anspruch 1, nämlich die Verbindungen 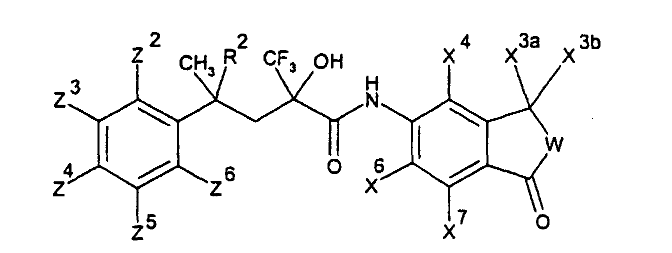
| **R**^{**2**} | **W** | **X**^{**n**} (≠H) | **Z**^{**n**} (≠H) | **Isomerie** |
|---|---|---|---|---|
| H | O | X^{3a}/X^{3b}=H/CH₃ | | Diast.-Gem. |
| H | O | X^{3a}= H, X^{3b}= CH₃ | | (+)-Form |
| H | O | X^{3a}= H, X^{3b}= CH₃ | | (-)-Form |
| H | O | X^{3a}= CH₃, X^{3b}= H | | (+)-Form |
| H | O | X^{3a}= CH₃, X^{3b}= H | | (-)-Form |
| H | O | X^{3a}= C₂H₅ | | |
| H | O | X^{3a}= CH=CH₂) | | |
| H | O | X^{3a}= CH=CH₂-CH₃ | | |
| H | O | X^{3a}= CF₃ | | |
| H | O | X^{3a}= X^{3b}= CH₃ | | |
| H | O | X^{3a}= X^{3b}= C₂H₅ | | |
| H | O | X^{3a}+ X^{3b}= (CH₂)₄ | | |
| H | O | X⁴=Br | | |
| CH₃ | O | | | |
| CH₃ | O | X⁴ = Br | | |
| CH₃ | O | | Z²=CH₃ | Racemat |
| CH₃ | O | | Z²=CH₃ | (+)-Form |
| CH₃ | O | | Z²= CH₃ | (-)-Form |
| CH₃ | O | | Z⁴= CH₃ | |
| **R**^{**2**} | **W** | **X**^{**n**} (≠H) | **Z**^{**n**} (≠H) | **Isomerie** |
|---|---|---|---|---|
| CH₃ | O | | Z³= Z⁴= CH₃ | Racemat |
| CH₃ | O | | Z³= Z⁴= CH₃ | (+)-Form |
| CH₃ | O | | Z³= Z⁴= CH₃ | (-)-Form |
| CH₃ | O | | Z³= Z⁵= CH₃ | Racemat |
| CH₃ | O | | Z³= Z⁵= CH₃ | (+)-Form |
| CH₃ | O | | Z³= Z⁵= CH₃ | (-)-Form |
| CH₃ | O | | Z³/Z⁴ = (CH₂)₃ | |
| CH₃ | O | | Z³/Z⁴=-CH=CH-CH=CH- | |
| CH₃ | O | | Z⁴= F | |
| CH₃ | O | | Z⁴= Cl | |
| CH₃ | O | | Z⁴= Br | |
| CH₃ | O | | Z²= OCH₃ | Racemat |
| CH₃ | O | | Z⁴=OCH₃ | |
| CH₃ | O | | Z²= Z⁵= OCH₃ | |
| CH₃ | O | | Z²= OCH₃, Z⁵= CH₃ | Racemat |
| CH₃ | O | | Z²= OCH₃, Z⁵= CH₃ | (+)-Form |
| CH₃ | O | | Z²= OCH₃, Z⁵= CH₃ | (-)-Form |
| CH₃ | O | | Z²= OCH₃, Z⁴= F | |
| CH₃ | O | | Z²=OCH₃,Z⁵=F | |
| CH₃ | O | | Z⁴= OCH₃, Z²= F | |
| CH₃ | O | | Z⁴= OCH₃, Z³= F | |
| CH₃ | O | | Z²= OCH₃, Z⁵= Cl | Racemat |
| CH₃ | O | | Z²= OCH₃, Z⁵= Cl | (+)-Form |
| **R**^{**2**} | **W** | **X**^{**n**} (≠H) | **Z**^{**n**} (≠H) | **Isomerie** |
|---|---|---|---|---|
| CH₃ | O | | Z²= OCH₃, Z⁵= Cl | (-)-Form |
| H | S | | | |
| CH₃ | S | | | |
| H | CH₂ | | | |
| H | O-CH₂ (3) | | | |
| **R**^{**2**} | **W** | **X**^{**n**} (≠H) | **Z**^{**n**} (≠H) | **Isomerie** |
|---|---|---|---|---|
| CH₃ | O | | Z⁴ = CH=CH₂ | Racemat |
| CH₃ | O | | Z⁴ = CN | Racemat |
| CH₃ | O | | Z⁴ = COCH₃ | Racemat |
| CH₃ | O | | Z⁴ = CONH₂ | Racemat |
| CH₃ | O | | Z² = OCH₃, Z⁴ = Br | Racemat |
| CH₃ | O | | Z²= OCH₃, Z⁴ = Br | (+)-Enantiomer |
| CH₃ | O | | Z² = OCH₃, Z⁴ = Br | (-)-Form |
| CH₃ | O | | Z² = Br, Z⁴ = OCH₃ | Racemat |
| CH₃ | O | | Z² = OCH₃, Z⁴ = CN | Racemat |
| CH₃ | O | | Z³ = NO₂, Z⁴ = OCH₃ | Racemat |
| CH₃ | O | | Z² = COCH₃, Z⁴ = CH(CH₃)₂ | Racemat |
| CH₃ | O | | Z³ = COCH₃, Z⁴ = OCH₃ | Racemat |

22. Verbindungen der allgemeinen Formel nach Anspruch 1, nämlich die Verbindungen 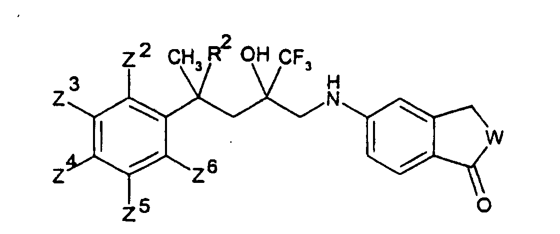
| **R**^{**2**} | **W** | **Z**^{**n**} (≠H) | **Isomerie** |
|---|---|---|---|
| H | O | | Racemat |
| H | O | | (+)-Form |
| H | O | | (-)-Form |
| CH₃ | O | | Racemat |
| CH₃ | O | | (+)-Form |
| CH₃ | O | | (-)-Form |
| CH₃ | O | Z⁴= F | Racemat |
| CH₃ | O | Z⁴= F | (+)-Form |
| CH₃ | O | Z⁴= F | (-)-Form |
| CH₃ | O | Z²= OCH₃, Z⁵= F | |
| H | CH₂ | | |
| H | OCH₂ (3) | | |

23. Verbindungen der allgemeinen Formel nach Anspruch 1, nämlich die Verbindungen

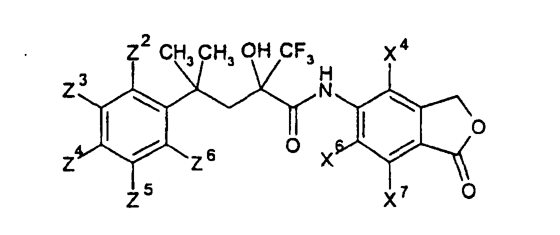

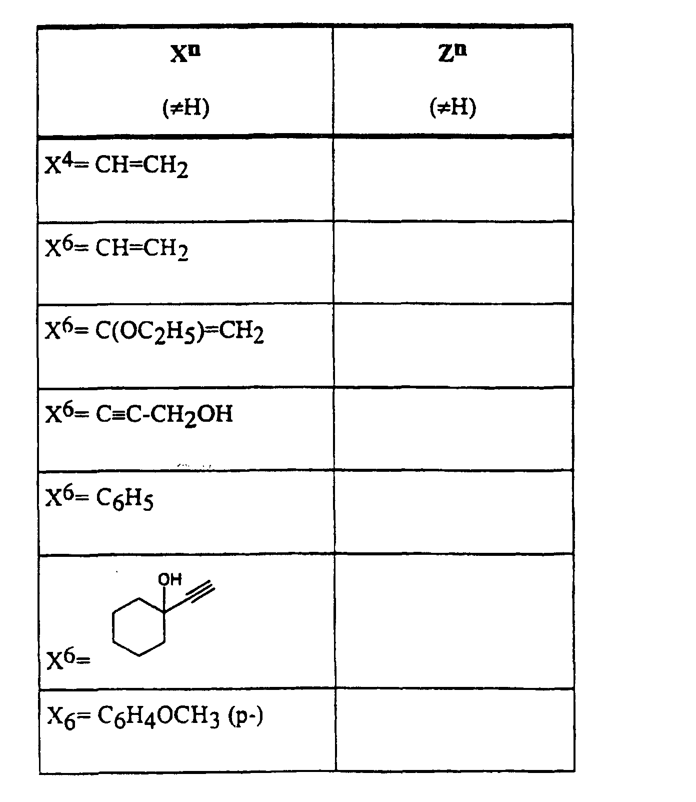

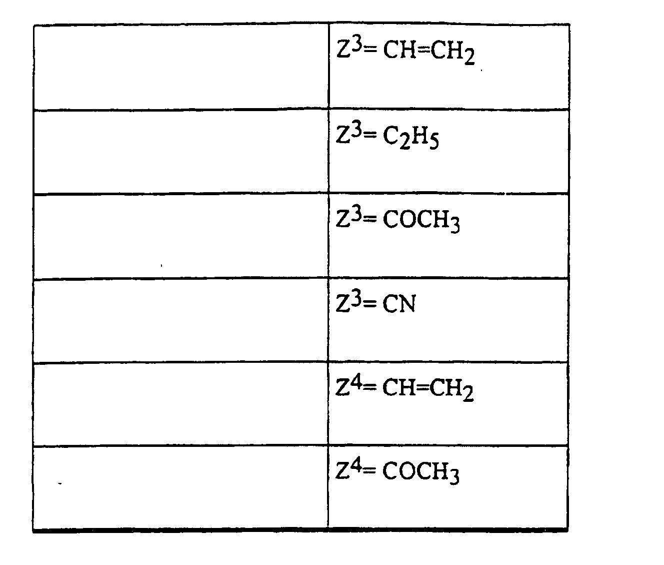

24. Verbindungen der allgemeinen Formel nach Anspruch 1, nämlich die Verbindungen 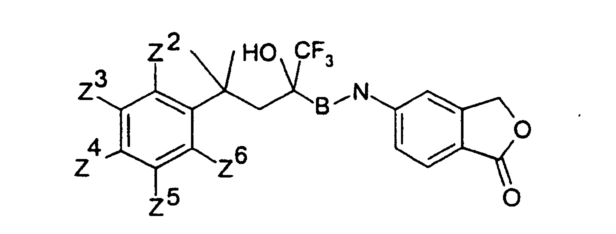
| **B** | **Z**^{**n**} (≠H) | **Isomerie** |
|---|---|---|
| C=O | Z²= OH | |
| C=O | Z⁴= OH | |
| C=O | Z²= Z⁵= OH | |
| C=O | Z²= OH, Z⁵= CH₃ | Racemat |
| C=O | Z²= OH, Z⁵= CH₃ | (+)-Form |
| C=O | Z²= OH, Z⁵= CH₃ | (-)-Form |
| C=O | Z²= OH, Z⁴= F | |
| C=O | Z²= OH, Z⁵= F | |
| C=O | Z⁴= OH, Z²= F | |
| C=O | Z²= OH, Z⁵= Cl | |
| CH₂ | Z²= OH, Z⁵= F | Racemat |
| CH₂ | Z²= OH, Z⁵= F | (+)-Form |
| CH₂ | Z²= OH, Z⁵= F | (-)-Form |
| C=O | Z² = OH, Z⁴ = Br | Racemat |
| C=O | Z³ = NO₂, Z⁴= OH | Racemat |
| C=O | Z³ = Cl, Z⁴= OH | Racemat |
| C=O | Z³ = Br, Z⁴= OH | Racemat |

25. Verbindungen der allgemeinen Formel nach Anspruch 1, nämlich die Verbindungen 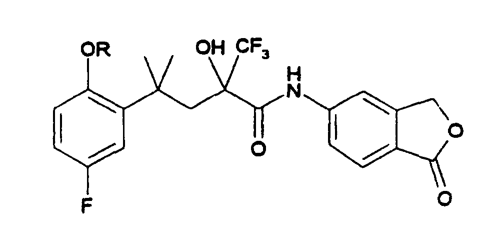
| **R** | **Isomerie** |
|---|---|
| CH(CH₃)₂ | Racemat |
| CH₂CH=CH₂ | Racemat |
| CH₂CH≡=CH₂ | Racemat |
| CH₂CN | Racemat |
| CH₂COOC(CH₃)₃ | Racemat |
| CH₂COOC(CH₃)₃ | (-)-Form |
| CH₂COOC(CH₃)₃ | (+)-Form |

26. Verbindungen der allgemeinen Formel nach Anspruch 1, nämlich die Verbindungen 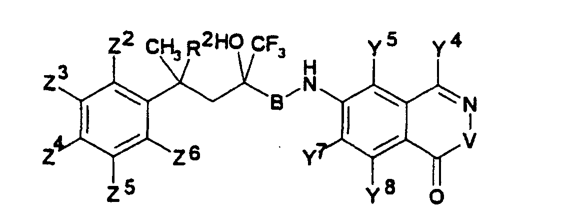
| **R**^{**2**} | **V** | **Z**^{**n**} (≠H) | **B** | **Y**^{**n**} (≠H) | **Isomerie** |
|---|---|---|---|---|---|
| H | O | | C=O | Y⁴= CH₃ | Racemat |
| H | O | | C=O | Y⁴= C₂H₅ | Racemat |
| CH₃ | O | | C=O | Y⁴= CH₃ | (+)-Form |
| CH₃ | O | | C=O | Y⁴= CH₃ | (-)-Form |
| CH₃ | O | | C=O | Y⁴= C₂H₅ | Racemat |
| CH₃ | O | | C=O | Y⁴= C₂H₅ | (+)-Form |
| CH₃ | O | | C=O | Y⁴= C₂H₅ | (-)-Form |
| **R**^{**2**} | **V** | **Z**^{**n**} (≠H) | **B** | **Y**^{**n**} (≠H) | **Isomerie** |
|---|---|---|---|---|---|
| CH₃ | O | Z²= OCH₃ | C=O | Y⁴= CH₃ | Racemat |
| CH₃ | O | Z²= OCH₃ | C=O | Y⁴= CH₃ | (-)-Form |
| CH₃ | O | Z²= OCH₃ | C=O | Y⁴= CH₃ | (+)-Form |
| CH₃ | O | Z²= OCH₃ | C=O | Y⁴= C₂H₅ | Racemat |
| CH₃ | O | Z²= OCH₃ | C=O | Y⁴= C₂H₅ | (+)-Form |
| CH₃ | O | Z²= OCH₃ | C=O | Y⁴= C₂H₅ | (-)-Form |
| CH₃ | O | Z²= OCH₃, Z⁵= F | C=O | Y⁴= CH₃ | Racemat |
| CH₃ | O | Z²= OCH₃, Z⁵= F | C=O | Y⁴= CH₃ | (+)-Form |
| CH₃ | O | Z²= OCH₃, Z⁵= F | C=O | Y⁴= CH₃ | (-)-Form |
| CH₃ | O | Z²= OCH₃, Z⁵= F | C=O | Y⁴= C₂H₅ | Racemat |
| CH₃ | O | Z²= OCH₃, Z⁵= F | C=O | Y⁴= C₂H₅ | (-)-Form |
| CH₃ | O | Z²= OCH₃, Z⁵= F | C=O | Y⁴= C₂H₅ | (+)-Form |
| CH₃ | O | Z²= OCH₃, Z⁵= Cl | C=O | Y⁴= CH₃ | Racemat |
| CH₃ | O | Z²= OCH₃, Z⁵= Cl | C=O | Y⁴= CH₃ | (+)-Form |
| CH₃ | O | Z²= OCH₃, Z⁵= Cl | C=O | Y⁴= CH₃ | (-)-Form |
| CH₃ | O | Z²= OCH₃, Z⁴= Br | C=O | Y⁴= CH₃ | Racemat |
| CH₃ | O | Z²= OCH₃, Z⁴= Br | C=O | Y⁴= CH₃ | (+)-Form |
| CH₃ | O | Z²= OCH₃, Z⁴= Br | C=O | Y⁴= CH₃ | (-)-Form |
| CH₃ | O | Z⁴= CH₃ | C=O | Y⁴= CH₃ | Racemat |
| CH₃ | O | Z⁴= CH₃ | C=O | Y⁴= C₂H₅ | Racemat |
| CH₃ | O | Z⁴= CH₃ | C=O | Y⁴= C₂H₅ | (-)-Form |
| CH₃ | O | Z⁴= CH₃ | C=O | Y⁴= C₂H₅ | (+)-Form |
| CH₃ | O | Z⁴= F | C=O | Y⁴= CH₃ | Racemat |
| CH₃ | O | Z⁴= Br | C=O | Y⁴= CH₃ | Racemat |
| CH₃ | O | Z⁴= Br | C=O | Y⁴= CH₃ | (-)-Form |
| CH₃ | O | Z⁴= Br | C=O | Y⁴= CH₃ | (+)-Form |
| CH₃ | O | | C=O | Y⁴= CF₃ | |
| CH₃ | NH | | C=O | Y⁴= CH₃ | |
| CH₃ | NCH₃ | | C=O | Y⁴= CH₃ | |
| CH₃ | O | Z² = OH, Z⁵ = F | C=O | Y⁴= CH₃ | Racemat |
| CH₃ | O | Z² = OH, Z⁵ = F | C=O | Y⁴= CH₃ | (+)-Form |
| CH₃ | O | Z² = OH, Z⁵ = F | C=O | Y⁴= CH₃ | (-)-Form |
| CH₃ | O | Z² = OH, Z⁴ = Br | C=O | Y⁴= CH₃ | Racemat |
| CH₃ | O | Z³ = NO₂, Z⁴ = OCH₃ | C=O | Y⁴= CH₃ | Racemat |
| H | O | | CH₂ | Y⁴= CH₃ | Racemat |
| CH₃ | O | | CH₂ | Y⁴= CH₃ | Racemat |
| CH₃ | O | | CH₂ | Y⁴= C₂H₅ | Racemat |

27. Pharmazeutische Präparate, enthaltend mindestens eine Verbindung der allgemeinen Formel I gemäß Anspruch 1 sowie einen pharmazeutisch verträglichen Träger.

28. Verwendung der Verbindungen der allgemeinen Formel I gemäß Anspruch 1 zur Herstellung von pharmazeutischen Präparaten.

29. Verfahren zur Herstellung von Verbindungen der allgemeinen Formel I 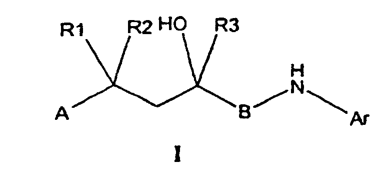 worin
A, B, Ar, R¹, R² sowie R³ die in Anspruch 1 angegebene Bedeutung haben, **dadurch gekennzeichnet, daß** eine Carbonylverbindung der allgemeinen Formel II 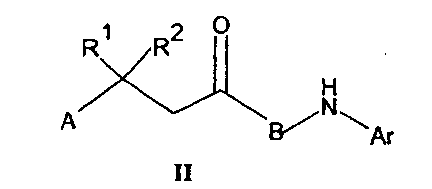 worin A, B, Ar, R¹ und R² die in Formel I angegebene Bedeutung haben, mit einer Verbindung der allgemeinen Formel R³-SiMe₃, worin R³ die in der allgemeinen Formel I angegebene Bedeutung hat, in Gegenwart eines Katalysators oder mit einer Alkylmetallverbindung, beispielsweise einem Grignard-Reagenz oder einem Lithiumalkyl, zu einer Verbindung der allgemeinen Formel I umgesetzt wird.

30. Verfahren nach Anspruch 29, **dadurch gekennzeichnet, daß** der Katalysator ein Fluorid-Salz oder ein Alkalicarbonat ist.

31. Verfahren zur Herstellung von Verbindungen der allgemeinen Formel I 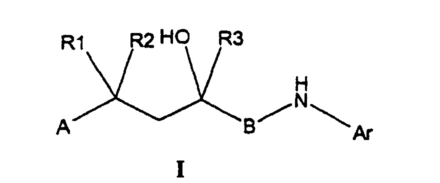 worin
A, B, Ar, R¹, R² sowie R³ die in Anspruch 1 angegebene Bedeutung haben, **dadurch gekennzeichnet, daß** eine Verbindung der allgemeinen Formel III 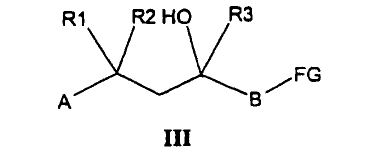 worin A, B, R¹,R² und R³ die in der allgemeinen Formel I angegebene Bedeutung haben und FG eine Fluchtgruppe bedeutet, mit einer Verbindung der Formel Ar-NH-R¹¹, wobei R¹¹ ein Wasserstoffatom oder eine C₁-C₅ Alkanoylgruppe bedeutet und Ar die in der allgemeinen Formel I angegebene Bedeutung hat, umgesetzt und gegebenenfalls anschließend der Rest R¹¹ abgespalten wird.

32. Verfahren nach Anspruch 31, **dadurch gekennzeichnet, daß** die Fluchtgruppe FG in der Verbindung der allgemeinen Formel III ein Chlor-, Brom- oder Iodatom, ein Tosylat- oder Mesylatrest oder ein C₁-C₄-Perfluoralkylsulfonyloxyrest ist.

33. Verfahren nach Anspruch 32, **dadurch gekennzeichnet, daß** die Verbindung der allgemeinen Formel III ein intermediär aus der entsprechenden Carbonsäure gebildetes Säurechlorid ist.

34. Verfahren zur Herstellung von Verbindungen der allgemeinen Formel I 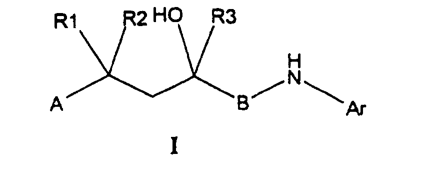 worin
A, Ar, R¹, R² sowie R³ die in Anspruch 1 angegebene Bedeutung haben und B eine -CH₂-Gruppe bedeutet, **dadurch gekennzeichnet, daß** eine Verbindung der allgemeinen Formel IV 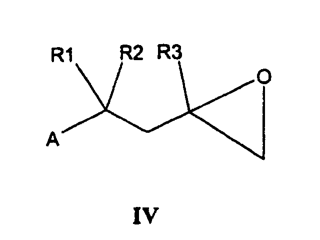 worin A, R¹,R² und R³ die in Formel I angegebene Bedeutung haben, mit einer Verbindung der Formel Ar-NH-R¹¹, wobei R¹¹ und Ar die in Anspruch 23 angegebenen Bedeutungen haben, umgesetzt und anschließend der Rest R¹¹ gegebenenfalls abgespalten wird.

## Claims

1. Compounds of the general formula I 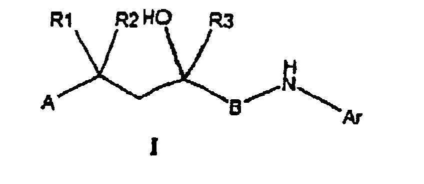 where
R¹ and R² are the same or different and are each a hydrogen atom, a C₁-C₅-alkyl group or a halogen atom, or else, together with the carbon atom of the chain, are a ring having a total of 3-7 members,
R³ is a C₁-C₅-alkyl group or a partly or fully fluorinated C₁-C₅-alkyl group,
A is a mono- or bicyclic, carbocyclic or heterocyclic, aromatic ring which is optionally substituted by one or more radicals selected from halogen atoms, C₁-C₅-alkyl groups, C₂-C₅-alkenyl groups, -CR⁵=CR⁶R⁷ where R⁵, R⁶ and R⁷ are the same or different and are each independently hydrogen atoms or C₁-C₅-alkyl groups, hydroxyl groups, hydroxyl groups which bear a C₁-C₁₀-acyl group, a C₃-C₁₀-carbalkoxyalkyl group, a C₂-C₅-cyanoalkyl group, a C₃-C₁₀ unsubstituted or substituted allyl group, a C₃-C₁₀ unsubstituted or substituted propargyl group, a C₂-C₅-alkoxyalkyl group or a C₁-C₅-alkyl group substituted fully or partly by fluorine atoms, or is selected from the cyano or nitro group, C₁-C₅-alkoxy groups, C₁-C₅-alkylthio groups, mono- or disubstituted C₁-C₁₀ amino groups or partly or fully fluorinated C₁-C₅-alkyl groups,
B is a carbonyl or a CH₂ group and
Ar is a ring system selected from the group of the general subformulae **2-11**: 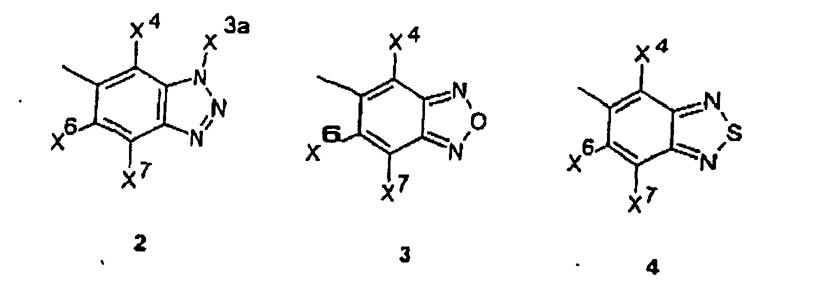 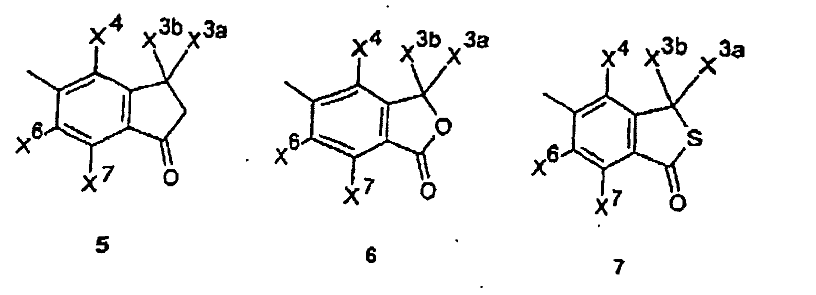 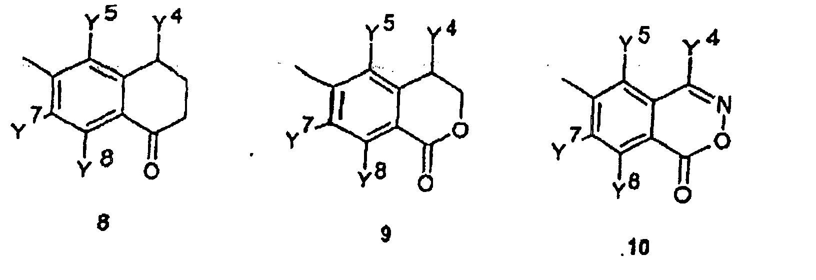 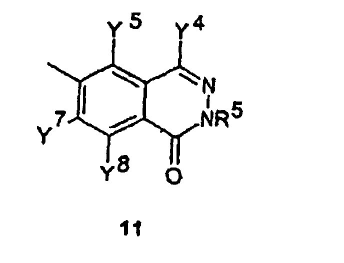
where
the X^{3a} X⁴, X⁶, X⁷ radicals (in the subformula **2**), the X⁴, X⁶, X⁷ radicals (in the subformulae **3** and **4**), the X^{3a}, X^{3b}, X⁴, X⁶, X⁷ radicals (in the subformulae **5**, **6** and **7**) or the Y⁴, Y⁵, Y⁷, Y⁸ radicals (in the subformulae **8**, **9**, **10** and **11**) are the same or different and are selected from hydrogen atoms, C₁-C₅-alkyl groups which may additionally contain a hydroxyl group optionally etherified with a C₁-C₅-alkyl group or esterified with a C₁-C₅-alkanoyl group, partly or fully fluorinated C₁-C₅-alkyl groups, C₂-C₅-alkenyl groups, -CR⁵=CR⁶R⁷ where R⁵, R⁶ and R⁷ are each as defined above, alkynyl groups - C≡CR⁵ where R⁵ is as defined above,
the X^{3a} and X^{3b} radicals, together with the carbon atom of the benzofused ring system **5**, **6** or **7**, may also form a ring having a total of 3-7 members,
and additionally the X⁴, X⁶, X⁷ radicals (in the subformulae **2**, **3**, **4**, **5**, **6** and **7**) or the Y⁴, Y⁵, Y⁷, Y⁸ radicals (in the subformulae **8**, **9**, **10** and **11**) are selected from halogen atoms, hydroxyl groups, C₁-C₅-alkoxy groups or C₁-C₅-alkanoyl groups,
and also, in the case that B is a CH₂ group, the physiologically tolerated salts of the compounds of the general formula I with acids.

2. Compounds of the general formula I according to Claim 1 in the form of the racemate or diastereomer mixture.

3. Compounds of the general formula I according to Claim 1 in the form of the separate optical isomers.

4. Compounds of the general formula I according to Claim 1, **characterized in that** R¹ and R² are the same or different and are each a hydrogen atom, a methyl or ethyl group, or else, together with the carbon atom of the chain, are a cyclopropyl ring.

5. Compounds of the general formula I according to Claim 1, **characterized in that** R³ is a C₁-C₅-perfluoroalkyl group.

6. Compounds of the general formula I according to Claim 1, **characterized in that** A is a benzene, naphthalene or thiophene ring optionally substituted by one or more radicals selected from fluorine atoms, chlorine atoms, bromine atoms, methyl groups, ethyl groups, vinyl groups, hydroxyl groups, methoxy groups, ethoxy groups.

7. Compounds of the general formula I according to Claim 1, **characterized in that** X^{3a} is a hydrogen atom or a C₁-C₅-alkyl group.

8. Compounds of the general formula I according to Claim 1, **characterized in that** X^{3a} and X^{3b} are the same or different and are each a hydrogen atom or a C₁-C₅-alkyl group.

9. Compounds of the general formula I according to Claim 1, **characterized in that** X⁴, X⁶ and X⁷ are the same or different and are each independently a hydrogen atom or a halogen atom.

10. Compounds of the general formula I according to Claim 1, **characterized in that** Y⁴ is a C₁-C₅-alkyl group or a C₁-C₅-perfluoroalkyl group.

11. Compounds of the general formula I according to Claim 1, **characterized in that** Y⁵, Y⁷ and Y⁸ are the same or different and are each independently a hydrogen atom or a halogen atom.

12. Compounds of the general formula I according to Claim 1, **characterized in that** R¹ and R² are the same or different and are each a hydrogen atom, a methyl or ethyl group, or else, together with the carbon atom of the chain, are a cyclopropyl ring, and R³ is a C₁-C₅-perfluoroalkyl group, A is a benzene, naphthalene or thiophene ring optionally substituted by one or more radicals selected from fluorine atoms, chlorine atoms, bromine atoms, methyl groups, ethyl groups, vinyl groups, hydroxyl groups, methoxy groups, ethoxy groups, and either X^{3a} is a hydrogen atom or a C₁-C₅-alkyl group, or X^{3a} and X^{3b} are the same or different and are each a hydrogen atom or a C₁-C₅-alkyl group, and X⁴, X⁶ and X⁷ are the same or different and are each independently a hydrogen atom or a halogen atom, or Y⁴ is a C₁-C₅-alkyl group or a C₁-C₅-perfluoroalkyl group, and Y⁵, Y⁷ and Y⁸ are the same or different and are each independently a hydrogen atom or a halogen atom, and the other substituents are each as defined in the formula I.

13. Compounds of the general formula I according to Claim 1, wherein Ar is a ring system of the subformula **6**.

14. Compounds of the general formula I according to Claim 1, wherein Ar is a ring system of the subformula **7**.

15. Compounds of the general formula I according to Claim 1, wherein Ar is a ring system of the subformula **10**.

16. Compounds of the general formula I according to Claim 1, wherein Ar is a ring system of the subformula **11**.

17. Compounds of the general formula I according to Claim 1, specifically
4-bromo-5-(2-hydroxy-4-methyl-4-phenyl-2-trifluoromethylvaleroylamino)phthalide,
6-bromo-5-(2-hydroxy-4-methyl-4-phenyl-2-trifluoromethylvaleroylamino)phthalide,
5-(2-hydroxy-4-methyl-2-pentafluoroethyl-4-phenyl-valeroylamino)phthalide,
5-[2-hydroxy-4-(3-methoxyphenyl)-4-methyl-2-trifluoromethylvaleroylamino]phthalide,
5-[2-hydroxy-4-(4-methoxyphenyl)-4-methyl-2-trifluoromethylvaleroylamino]phthalide,
5-[2-hydroxy-4-(2-hydroxyphenyl)-4-methyl-2-trifluoromethylvaleroylamino]phthalide,
5-[4-(2-fluorophenyl)-2-hydroxy-4-methyl-2-trifluoromethylvaleroylamino]phthalide,
5-[4-(4-fluorophenyl)-2-hydroxy-4-methyl-2-trifluoromethylvaleroylamino]phthalide,
5-[4-(4-chlorophenyl)-2-hydroxy-4-methyl-2-trifluoromethylvaleroylamino]phthalide,
5-[4-(4-bromophenyl)-2-hydroxy-4-methyl-2-trifluoromethylvaleroylamino]phthalide,
5-[2-hydroxy-4-methyl-4-(4-tolyl)-2-trifluoromethylvaleroylamino]phthalide,
5-[2-hydroxy-4-methyl-4-(3-tolyl)-2-trifluoromethylvaleroylamino]phthalide,
5-[4-(4-cyanophenyl-)-2-hydroxy-4-methyl-2-trifluoromethylvaleroylamino]phthalide,
5-[4-(3,4-dimethylphenyl)-2-hydroxy-4-methyl-2-trifluoromethylvaleroylamino]phthalide,
5-[4-(3,5-dimethylphenyl)-2-hydroxy-4-methyl-2-trifluoromethylvaleroylamino]phthalide,
5-[2-hydroxy-4-(2-methoxy-5-methylphenyl)-4-methyl-2-trifluoromethylvaleroylamino]phthalide,
5-[4-(5-chloro-2-hydroxyphenyl)-2-hydroxy-4-methyl-2-trifluoromethylvaleroylamino]phthalide,
5-[4-(5-fluoro-2-hydroxyphenyl)-2-hydroxy-4-methyl-2-trifluoromethylvaleroylamino]phthalide,
5-[2-hydroxy-4-(2-hydroxy-5-methylphenyl)-4-methyl-2-trifluoromethylvaleroylamino]phthalide,
5-[4-(5-fluoro-2-methoxyphenyl)-2-hydroxy-4-methyl-2-trifluoromethylvaleroylamino]phthalide,
5-[4-(2-fluoro-4-methoxyphenyl)-2-hydroxy-4-methyl-2-trifluoromethylvaleroylamino]phthalide,
5-[4-(3-fluoro-4-methoxyphenyl)-2-hydroxy-4-methyl-2-trifluoromethylvaleroylamino]phthalide,
5-(2-hydroxy-4-phenyl-2-trifluoromethylvaleroylamino)phthalide,
5-[2-hydroxy-4-(2-methoxyphenyl)-4-methyl-2-trifluoromethylvaleroylamino]phthalide,
5-[4-(5-chloro-2-methoxyphenyl)-2-hydroxy-4-methyl-2-trifluoromethylvaleroylamino]phthalide,
5-(2-hydroxy-4-phenyl-2-trifluoromethylpentylamino)phthalide,
5-(2-hydroxy-4-methyl-4-phenyl-2-trifluoromethylpentylamino)phthalide,
5-[4-(4-fluorophenyl)-2-hydroxy-4-methyl-2-trifluoromethylpentylamino]phthalide,
5-[4-(5-fluoro-2-hydroxyphenyl)-2-hydroxy-4-methyl-2-trifluoromethylpentylamino]phthalide,
6-acetyl-5-(2-hydroxy-4-methyl-4-phenyl-2-trifluoromethylvaleroylamino)phthalide,
5-[4-(3-fluoro-4-hydroxyphenyl)-2-hydroxy-4-methyl-2-trifluoromethylvaleroylamino]phthalide,
5-[4-(3-fluorophenyl)-2-hydroxy-4-methyl-2-trifluoromethylvaleroylamino]phthalide,
6-(3-hydroxy-3-methyl-1-butynyl)-5-(2-hydroxy-4-methyl-4-phenyl-2-trifluoromethylvaleroylamino)phthalide,
6-(2-hydroxy-4-methyl-4-phenyl-2-trifluoromethylvaleroylamino)-4-methyl-2,3-benzoxazin-1-one,
6-(2-hydroxy-4-methyl-4-phenyl-2-trifluoromethylvaleroylamino)-4-trifluoromethyl-2,3-benzoxazin-1-one,
4-ethyl-6-(2-hydroxy-4-phenyl-2-trifluoromethylpentylamino)-2,3-benzoxazin-1-one,
4-ethyl-6-[2-hydroxy-4-(2-methoxyphenyl)-4-methyl-2-trifluoromethylvaleroylamino]-2,3-benzoxazin-1-one,
6-[2-hydroxy-4-(2-methoxyphenyl)-4-methyl-2-trifluoromethylvaleroylamino]-4-methyl-2,3-benzoxazin-1-one,
4-ethyl-6-[2-hydroxy-4-methyl-4-(4-methylphenyl)-2-trifluoromethylvaleroylamino]-2,3-benzoxazin-1-one,
6-[4-(4-bromophenyl)-2-hydroxy-4-methyl-2-trifluoromethylvaleroylamino]-4-ethyl-2,3-benzoxazin-1-one,
4-ethyl-6-[4-(5-fluoro-2-methoxyphenyl)-2-hydroxy-4-methyl-2-trifluoromethylvaleroylamino]-2,3-benzoxazin-1-one,
6-[4-(5-fluoro-2-methoxyphenyl)-2-hydroxy-4-methyl-2-trifluoromethylvaleroylamino]-4-methyl-2,3-benzoxazin-1-one,
1-(4-nitro-3-trifluoromethylanilino)-4-phenyl-2-trifluoromethyl-2-pentanol,
5-(2-hydroxy-4,4-dimethyl-2-trifluoromethyl-5-hexenoylamino)phthalide,
5-[2-hydroxy-3-(1-phenylcyclopropyl)-2-trifluoromethylpropionylamino]phthalide,
5-[2-hydroxy-3-(1-phenylcyclobutyl)-2-trifluoromethylpropionylamino]phthalide,
5-[2-hydroxy-3-(1-phenylcyclohexyl)-2-trifluoromethylpropionylamino]phthalide,
6-(2-hydroxy-2,4-dimethyl-4-phenylvaleroylamino)-4-methyl-2,3-benzoxazin-1-one,
5-[4-(3-chloro-4-methoxyphenyl)-2-hydroxy-4-methyl-2-trifluoromethylvaleroylamino]phthalide.

18. Compounds of the general formula I according to Claim 1, specifically the compounds 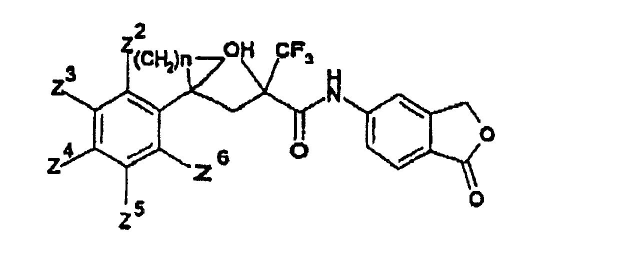
| **n** | **Z**^{**n**} **(≠H)** | **Isomerism** |
|---|---|---|
| 1 | | racemate |
| 1 | | (+)-enantiomer |
| 1 | | (-)-enantiomer |
| 1 | 3-F | racemate |
| 1 | 2-Cl | racemate |
| 1 | 4-Cl | racemate |
| 1 | 4-Cl | (+)-enantiomer |
| 1 | 4-Cl | (-)-enantiomer |
| 1 | 2-Br | racemate |
| 1 | 3-Br | racemate |
| 1 | 2,4-Cl₂ | racemate |
| 1 | 2-OCH₃ | (+)-enantiomer |
| 1 | 2-OCH₃ | (-)-enantiomer |
| 1 | 3-OCH₃ | racemate |
| 1 | 3-CF₃ | racemate |
| 2 | | racemate |
| 2 | | (+)-enantiomer |
| 2 | | (-)-enantiomer |
| 3 | | (+)-enantiomer |
| 3 | 4-CH₃ | racemate |
| 4 | | racemate |
| 4 | | (+)-enantiomer |
| 4 | | (-)-enantiomer |

19. Compounds of the general formula according to Claim 1, specifically the compounds 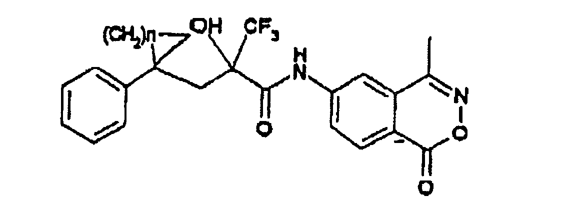
| **N** | **Isomerism** |
|---|---|
| 1 | Racemate |
| 1 | (+)-enantiomer |
| | (-)-enantiomer |
| 2 | Racemate |
| 4 | Racemate |
| 4 | (+)-enantiomer |
| 4 | (-)-enantiomer |

20. Compounds of the general formula according to Claim 1, specifically the compounds 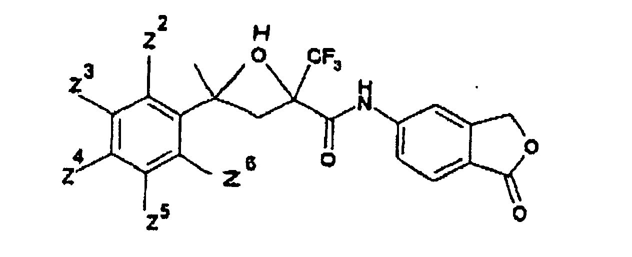
| **Z**^{**n**} **(≠H)** |
|---|
| Z² = I |
| Z³ = Cl |
| Z³= Br |
| Z³ = I |

21. Compounds of the general formula according to Claim 1, specifically the compounds 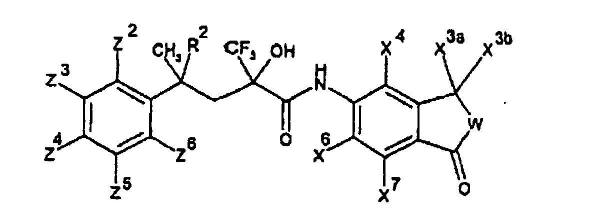
| **R**^{**2**} | **W** | **X**^{**n**} **(≠H)** | **Z**^{**n**} **(≠H)** | **Isomerism** |
|---|---|---|---|---|
| H | O | X^{3a} /X^{3b}=H/CH₃ | | Diast. mixt. |
| H | O | X^{3a}=H, X^{3b}=CH₃ | | (+)-form |
| H | O | X^{3a}=H, X^{3b}=CH₃ | | (-)-form |
| H | O | X^{3a}=CH₃, X^{3b}=H | | (+)-form |
| H | O | X^{3a}=CH₃, X^{3b}=H | | (-)-form |
| H | O | X^{3a=}C₂H₅ | | |
| H | O | X^{3a}=CH=CH₂ | | |
| H | O | X^{3a}=CH=CH₂-CH₃ | | |
| H | O | X^{3a}=CF₃ | | |
| H | O | X^{3a}=X^{3b}=CH₃ | | |
| H | O | X^{3a}=X^{3b}=C₂H₅ | | |
| H | O | X^{a}=X^{3b}=(CH₂)₄ | | |
| H | O | X⁴=Br | | |
| CH₃ | O | | | |
| CH₃ | O | X⁴=Br | | |
| CH₃ | O | | Z^{Z}=CH₃ | racemate |
| CH₃ | O | | Z²=CH₃ | (+)-form |
| CH₃ | O | | Z²=CH₃ | (-)-form |
| CH₃ | O | | Z⁴=CH₃ | |
| **R**^{**2**} | **W** | **X**^{**n**} **(≠H)** | **Z**^{**n**} **(≠H)** | **Isomerism** |
|---|---|---|---|---|
| CH₃ | O | | Z³=Z⁴=CH₃ | racemate |
| CH₃ | O | | Z³=Z⁴=CH₃ | (+)-form |
| CH₃ | O | | Z³=Z⁴=CH₃ | (-)-form |
| CH₃ | O | | Z³=Z⁵=CH₃ | racemate |
| CH₃ | O | | Z³=Z⁵=CH₃ | (+)-form |
| CH₃ | O | | Z³=Z⁵=CH₃ | (-)-form |
| CH₃ | O | | Z³/Z⁴=(CH₂)₃ | |
| CH₃ | O | | Z³/Z⁴=-CH=CH-CH=CH- | |
| CH₃ | O | | Z⁴=F | |
| CH₃ | O | | Z⁴=Cl | |
| CH₃ | O | | Z⁴-Br | |
| CH₃ | O | | Z²=OCH₃ | racemate |
| CH₃ | O | | Z⁴=OCH₃ | |
| CH₃ | O | | Z²=Z⁵=OCH₃ | |
| CH₃ | O | | Z²=OCH₃, Z⁵=CH₃ | racemate |
| CH₃ | O | | Z²=OCH₃, Z⁵=CH₃ | (+)-form |
| CH₃ | O | | Z²=OCH₃, Z⁵=CH₃ | (-)-form |
| CH₃ | O | | Z²=OCH₃, Z⁴=F | |
| CH₃ | O | | Z²=OCH₃, Z⁵=F | |
| CH₃ | O | | Z⁴=OCH₃, Z²=F | |
| CH₃ | O | | Z⁴=OCH₃, Z³=F | |
| CH₃ | O | | Z²=OCH₃, Z⁵=Cl | racemate |
| CH₃ | O | | Z²=OCH₃, Z⁵=Cl | (+)-form |
| **R**^{**2**} | **W** | **X**^{**n**} **(≠H)** | **Z**^{**n**} **(≠H)** | **Isomerism** |
|---|---|---|---|---|
| CH₃ | O | | Z²=OCH₃, Z⁵=Cl | (-)-form |
| H | S | | | |
| CH₃ | S | | | |
| H | CH₂ | | | |
| H | O-CH₂(3) | | | |
| **R**^{**2**} | **W** | **X**^{**n**} **(≠H)** | **Z**^{**n**} **(≠H)** | **Isomerism** |
|---|---|---|---|---|
| CH₃ | O | | Z⁴=CH=CH₂ | racemate |
| CH₃ | O | | Z⁴=CN | racemate |
| CH₃ | O | | Z⁴=COCH₃ | racemate |
| CH₃ | O | | Z⁴=CONH₂ | racemate |
| CH₃ | O | | Z²=OCH₃, Z⁴=Br | racemate |
| CH₃ | O | | Z²=OCH₃, Z⁴=Br | (+)-enantiomer |
| CH₃ | O | | Z²=OCH₃, Z⁴=Br | (-)-form |
| CH₃ | O | | Z²=Br, Z⁴=OCH₃ | Racemate |
| CH₃ | O | | Z²=OCH₃, Z⁴=CN | Racemate |
| CH₃ | O | | Z³=NO₂, Z⁴=OCH₃ | Racemate |
| CH₃ | O | | Z²=COCH₃, Z⁴=CH (CH₃)₂ | Racemate |
| CH₃ | O | | Z³=COCH₃, Z⁴=OCH₃ | Racemate |

22. Compounds of the general formula according to Claim 1, specifically the compounds 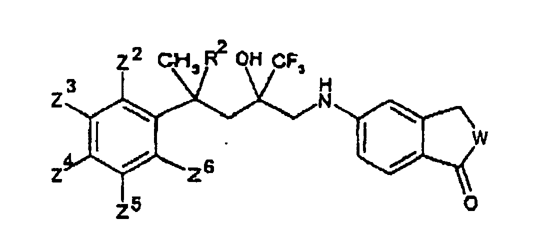
| **R**^{**2**} | **W** | **Z**^{**n**} **(#H)** | **Isomerism** |
|---|---|---|---|
| H | O | | racemate |
| H | O | | (+)-form |
| H | O | | (-)-form |
| CH₃ | O | | racemate |
| CH₃ | O | | (+)-form |
| CH₃ | O | | (-)-form |
| CH₃ | O | Z⁴=F | racemate |
| CH₃ | O | Z⁴=F | (+)-form |
| CH₃ | O | Z⁴=F | (-)-form |
| CH₃ | O | Z²=OCH₃, Z⁵=F | |
| H | CH₂ | | |
| H | OCH₂(3) | | |

23. Compounds of the general formula according to Claim 1, specifically the compounds

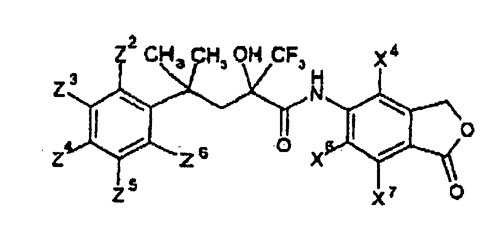

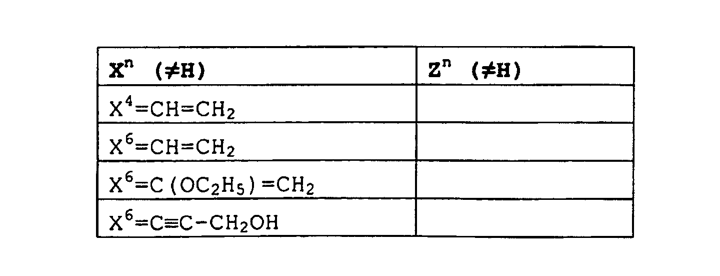

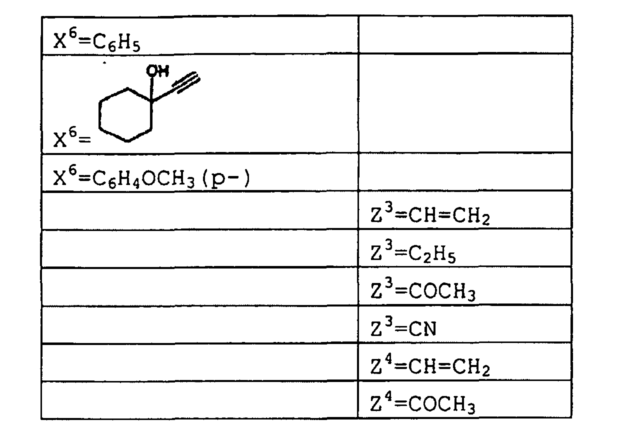

24. Compounds of the general formula according to Claim 1, specifically the compounds 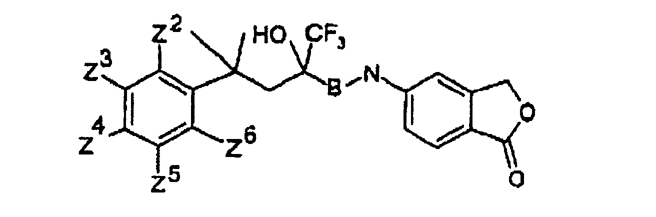
| **B** | **Z**^{**n**} **(≠H)** | **Isomerism** |
|---|---|---|
| C=O | Z²=OH | |
| C=O | Z⁴=OH | |
| C=O | Z²=Z⁵=OH | |
| C=O | Z²=OH, Z⁵=CH₃ | Racemate |
| C=O | Z²=OH, Z⁵=CH₃ | (+) -form |
| C=O | Z²=OH, Z⁵=CH₃ | (-) -form |
| C=O | Z²=OH, Z⁴=F | |
| C=O | Z²=OH, Z⁵=F | |
| C=O, | Z⁴=OH, Z²= F | |
| C=O | Z²=OH, Z⁵=Cl | |
| CH₂ | Z²=OH, Z⁵=F | racemate |
| CH₂ | Z²=OH, Z⁵=F | (+) -form |
| CH₂ | Z²=OH, Z⁵=F | (-) -form |
| C=O | Z²=OH, Z⁴=Br | Racemate |
| C=O | Z³=NO₂, Z⁴=OH | Racemate |
| C=O | Z³=Cl, Z⁴=OH | racemate |
| C=O | Z³=Br, Z⁴=OH | Racemate |

25. Compounds of the general formula according to Claim 1, specifically the compounds 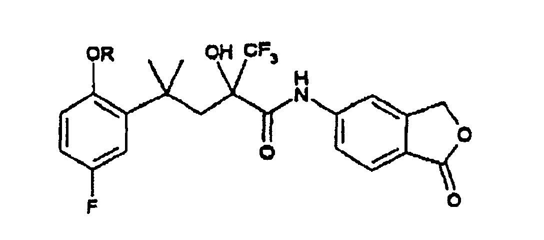
| **R** | **Isomerism** |
|---|---|
| CH (CH₃) ₂ | Racemate |
| CH₂CH=CH₂ | Racemate |
| CH₂CH=CH₂ | Racemate |
| CH₂CN | Racemate |
| CH₂COOC(CH₃)₃ | Racemate |
| CH₂COOC(CH₃)₃ | (-)-form |
| CH₂COOC(CH₃)₃ | (+)-form |

26. Compounds of the general formula according to Claim 1, specifically the compounds 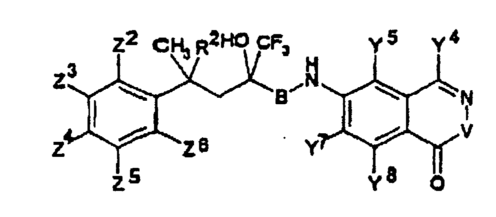
| **R**^{**2**} | **V** | **Z**^{**n**} **(≠H)** | **B** | **Y**^{**n**} **(≠H)** | **Isomerism** |
|---|---|---|---|---|---|
| H | O | | C=O | Y⁴=CH₃ | racemate |
| H | O | | C=O | Y⁴=C₂H₅ | racemate |
| CH₃ | O | | C=O | Y⁴=CH₃ | (+)-form |
| CH₃ | O | | C=O | Y⁴=CH₃ | (-)-form |
| CH₃ | O | | C=O | Y⁴=C₂H₅ | racemate |
| CH₃ | O | | C=O | Y⁴=C₂H₅ | (+)-form |
| CH₃ | O | | C=O | Y⁴=C₂H₅ | (-)-form |
| CH₃ | O | Z²=OCH₃ | C=O | Y⁴=CH₃ | racemate |
| CH₃ | O | Z²=OCH₃ | C=O | Y⁴=CH₃ | (-)-form |
| CH₃ | O | Z²=OCH₃ | C=O | Y⁴=CH₃ | (+)-form |
| CH₃ | O | Z²=OCH₃ | C=O | Y⁴=C₂H₅ | racemate |
| CH₃ | O | Z²=OCH₃ | C=O | Y⁴=C₂H₅ | (+)-form |
| CH₃ | O | Z²=OCH₃ | C=O | Y⁴=C₂H₅ | (-)-form |
| CH₃ | O | Z²=OCH₃, Z⁵=F | C=O | Y⁴=CH₃ | racemate |
| CH₃ | O | Z²=OCH₃, Z⁵=F | C=O | Y⁴=CH₃ | (+)-form |
| CH₃ | O | Z²=OCH₃, Z⁵=F | C=O | Y⁹=CH₃ | (-)-form |
| CH₃ | O | Z²=OCH₃, Z⁵=F | C=O | Y⁴=C₂H₅ | racemate |
| CH₃ | O | Z²=OCH₃, Z⁵=F | C=O | Y⁴=C₂H₅ | (-)-form |
| CH₃ | O | Z²=OCH₃, Z⁵=F | C=O | Y⁴=C₂H₅ | (+)-form |
| CH₃ | O | Z²=OCH₃, Z⁵=Cl | C=O | Y⁴=CH₃ | racemate |
| CH₃ | O | Z²=OCH₃, Z⁵=Cl | C=O | Y⁴=CH₃ | (+)-form |
| CH₃ | O | Z²=OCH₃, Z⁵=Cl | C=O | Y⁴=CH₃ | (-)-form |
| CH₃ | O | Z²=OCH₃, Z⁵=Br | C=O | Y⁴=CH₃ | racemate |
| CH₃ | O | Z²=OCH₃, Z⁵=Br | C=O | Y⁴=CH₃ | (+)-form |
| CH₃ | O | Z²=OCH₃, Z⁵=Br | C=O | Y⁴=CH₃ | (-)-form |
| CH₃ | O | Z⁴=CH₃ | C=O | Y⁴=CH₃ | racemate |
| CH₃ | O | Z⁴=CH₃ | C=O | Y⁴=C₂H₅ | racemate |
| CH₃ | O | Z⁴ =CH₃ | C=O | Y⁴=C₂H₅ | (-)-form |
| CH₃ | O | Z⁴=CH₃ | C=O | Y⁴=C₂H₅ | (+)-form |
| CH₃ | O | Z⁴=F | C=O | Y⁴=CH₃ | racemate |
| CH₃ | O | Z⁴=Br | C=O | Y⁴=CH₃ | racemate |
| CH₃ | O | Z⁴=Br | C=O | Y⁴=CH₃ | (-)-form |
| CH₃ | O | Z⁴=Br | C=O | Y⁴=CH₃ | (+)-form |
| CH₃ | O | | C=O | Y⁴=CH₃ | |
| CH₃ | NH | | C=O | Y⁴=CH₃ | |
| CH₃ | NCH₃ | | C=O | Y⁴=CH₃ | |
| CH₃ | O | Z²=OH, Z⁵=F | C=O | Y⁴=CH₃ | racemate |
| CH₃ | O | Z²=OH, Z⁵=F | C=O | Y⁴=CH₃ | (+)-form |
| CH₃ | O | Z²=OH, Z⁵=F | C=O | Y⁴=CH₃ | (-)-form |
| CH₃ | O | Z²=OH, Z⁴=Br | C=O | Y⁹=CH₃ | racemate |
| CH₃ | O | Z³=NO₂, Z⁴=OCH₃ | C=O | Y⁹=CH₃ | racemate |
| H | O | | CH₂ | Y⁴=CH₃ | racemate |
| CH₃ | O | | CH₂ | Y⁴=CH₃ | racemate |
| CH₃ | O | | CH₂ | Y⁹=C₂H₅ | racemate |

27. Pharmaceutical preparations comprising at least one compound of the general formula I according to Claim 1 and a pharmaceutically tolerated carrier.

28. Use of compounds of the general formula I according to Claim 1 for preparing pharmaceutical preparations.

29. Process for preparing compounds of the general formula I 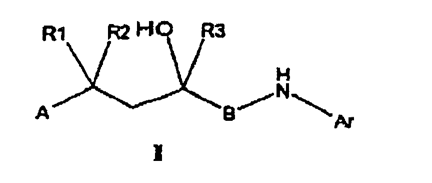 where
A, B, Ar, R¹, R² and R³ are each as defined in Claim 1, **characterized in that** a carbonyl compound of the general formula II 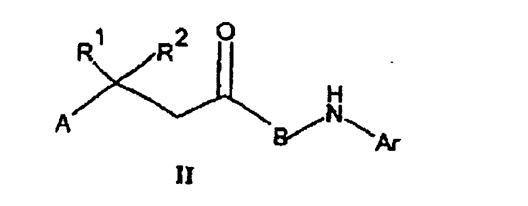 where A, B, Ar, R¹ and R² are each as defined in formula I is reacted with a compound of the general formula R³-SiMe₃ where R³ is as defined in the general formula I in the presence of a catalyst, or with an alkyl-metal compound, for example a Grignard reagent or a lithium alkyl, to give a compound of the general formula I.

30. The process according to Claim 29, **characterized in that** the catalyst is a fluoride salt or an alkali metal carbonate.

31. Process for preparing compounds of the general formula I 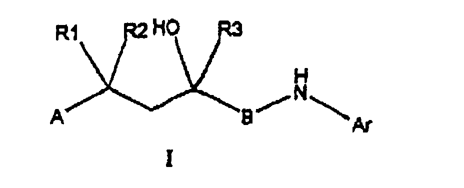 where
A, B, Ar, R¹, R² and R³ are each as defined in Claim 1, **characterized in that** a compound of the general formula III 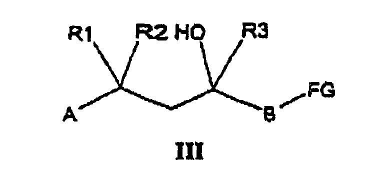 where A, B, R¹, R² and R³ are each as defined in the general formula I and FG is a leaving group is reacted with a compound of the formula Ar-NH-R¹¹ where R¹¹ is a hydrogen atom or a C₁-C₅-alkanoyl group and Ar is as defined in the general formula I, and the R¹¹ radical is subsequently, where appropriate, detached.

32. The process according to Claim 31, **characterized in that** the leaving group in the compound of the general formula III is a chlorine, bromine or iodine atom, a tosylate or mesylate radical or a C₁-C₄-perfluoroalkylsulphonyloxy radical.

33. The process according to Claim 32, **characterized in that** the compound of the general formula III is an acid chloride formed as an intermediate from the corresponding carboxylic acid.

34. Process for preparing compounds of the general formula I 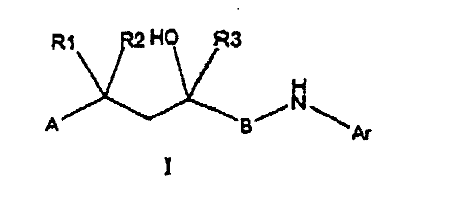 where
A, Ar, R¹, R² and R³ are each as defined in Claim 1, and B is a -CH₂- group, **characterized in that** a compound of the general formula IV 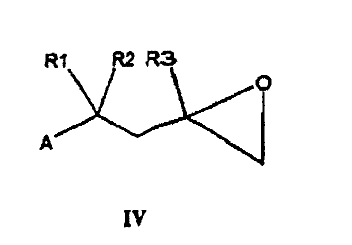 where A, R¹, R² and R³ are each as defined in formula I is reacted with a compound of the formula Ar-NH-R¹¹ where R¹¹ and Ar are each as defined in Claim 23, and the R¹¹ radical is subsequently, where appropriate, detached.

## Revendications

1. Composés de formule générale 1 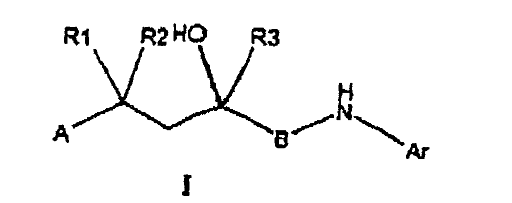 dans laquelle R¹ et R² sont identiques ou différents et représentent un atome d'hydrogène, un groupe alkyle en C₁-C₅ ou un atome d'halogène, et en outre, conjointement avec l'atome de carbone de la chaîne, un noyau à 3-7 chaînons au total,
R³ représente un groupe alkyle en C₁-C₅ ou un groupe alkyle en C₁-C₅ partiellement ou totalement fluoré,
A représente un noyau aromatique carbocyclique ou hétérocyclique, mono- ou bicyclique, éventuellement substitué par un ou plusieurs radicaux choisis parmi des atomes d'halogène, des groupes alkyle en C₁-C₅, des groupes alcényles en C₂-C₅, -CR⁵=CR⁶R⁷, où R⁵, R⁶ et R⁷ sont identiques ou différents et représentent, indépendamment les uns des autres, des atomes d'hydrogène ou des groupes alkyles en C₁-C₅, des groupes hydroxy, des groupes hydroxy portant un groupe acyle en C₁-C₁₀, un groupe C₃-C₁₀-carbalcoxyalkyle, un groupe C₂-C₅-cyanalkyle, un groupe alkyle en C₃-C₁₀ non substitué ou substitué, un groupe propargyle en C₃-C₁₀ non substitué ou substitué, un groupe C₂-C₅-alcoxyalkyle, un groupe alkyle en C₁-C₅ partiellement ou totalement substitué par des atomes de fluor, le groupe cyano ou nitro, des groupes alcoxy en C₁-C₅, des groupes C₁-C₅-alkylthio, des groupes amino en C₁-C₁₀ mono- ou bi-substitué ou des groupes alkyle en C₁-C₅ partiellement ou totalement fluorés,
B représente un groupe carbonyle ou CH₂, et
Ar représente un système cyclique choisi parmi le groupe des formules partielles générales 2 à 11 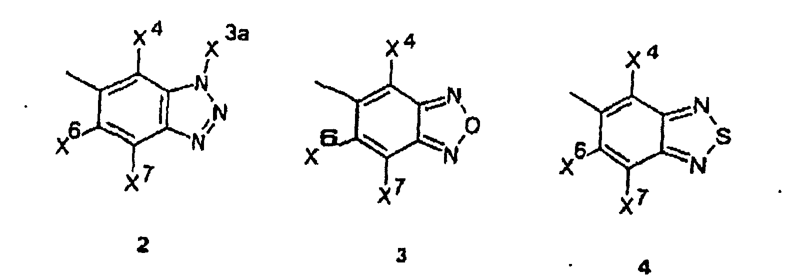 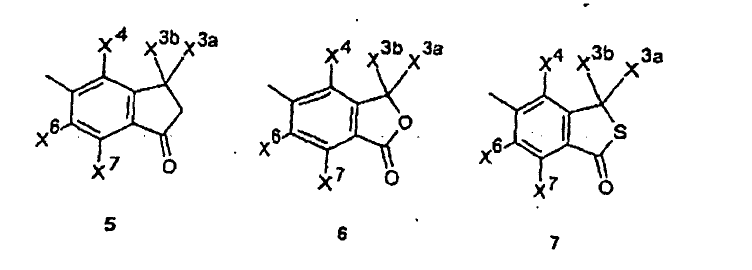 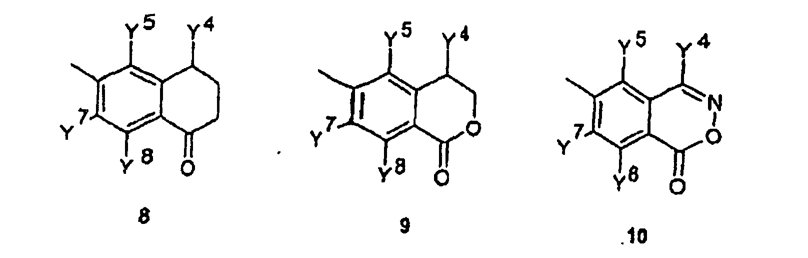 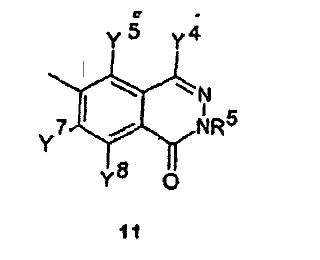 dans lesquelles
les radicaux X^{3a}, X⁴, X⁶, X⁷ (dans la formule partielle 2), X⁴, X⁶, X⁷(dans les formules partielles 3 et 4) X^{3a}, X^{3b}, X⁴, X⁶, X⁷ (dans les formules partielles 5, 6 et 7) ou Y⁴, Y⁵, Y⁷, Y⁸(dans les formules partielles 8, 9, 10 et 11) sont identiques ou différents et choisis parmi des atomes d'hydrogène, des groupes alkyle en C₁-C₅, qui peuvent en outre également éventuellement renfermer un groupe hydroxy éthérifié par un groupe alkyle en C₁-C₅ ou estérifié par un groupe alcanoyle en C₁-C₅, des groupes alkyle en C₁-C₅ partiellement ou totalement fluorés, des groupes alcényle en C₂-C₅ -CR⁵=CR⁶R⁷, où R⁵, R⁶ et R⁷ présentent la signification susmentionnée, des groupes alcynyle -C≡CR⁵, où R⁵ présente la signification susmentionnée,
les radicaux X^{3a} et X^{3b} peuvent en outre former, conjointement avec l'atome de carbone du système cyclique benzo-condensé 5, 6 ou 7, un noyau à 3-7 chaînons au total,
et par ailleurs les radicaux X⁴, X⁶, X⁷ (dans les formules partielles 2, 3, 4, 5, 6 et 7) ou Y⁴, Y⁵, Y⁷, Y⁸ (dans les formules partielles 8, 9, 10 et 11) sont choisis parmi les atomes d'halogène, des groupes hydroxy, des groupes alcoxy en C₁-C₅ ou des groupes alcanoyle en C₁-C₅,
et dans le cas où B représente un groupe CH₂, les sels physiologiquement acceptables des composés de formule générale 1 avec des acides.

2. Composés de formule générale 1 selon la revendication 1 sous forme du racémate ou du mélange de diastéréoisomères.

3. Composés de formule générale 1 selon la revendication 1 sous forme des isomères optiques séparés.

4. Composés de formule générale 1 selon la revendication 1, **caractérisés en ce que** R¹ et R² sont identiques ou différents et représentent un atome d'hydrogène, un groupe méthyle ou éthyle, et en outre un noyau cyclopropyle, conjointement avec l'atome de carbone de la chaîne.

5. Composés de formule générale 1 selon la revendication 1 **caractérisés en ce que** R³ représente un groupe perfluoroalkyle en C₁-C₅.

6. Composés de formule générale 1 selon la revendication 1, **caractérisés en ce que** A représente un noyau benzène, naphtalène ou thiophène éventuellement substitué par un ou plusieurs radicaux choisis parmi des atomes de fluor, des atomes de chlore, des atomes de brome, des groupes méthyle, des groupes éthyle, des groupes vinyle, des groupes hydroxy, des groupes méthoxy, des groupes éthoxy.

7. Composés de formule générale 1 selon la revendication 1 **caractérisés en ce que** X^{3a} représente un atome d'hydrogène, un groupe alkyle en C₁-C₅.

8. Composés de formule générale 1 selon la revendication 1 **caractérisés en ce que** X^{3a} etX^{3b} sont identiques ou différents et représentent un atome d'hydrogène ou un groupe alkyle en C₁-C₅.

9. Composés de formule générale 1 selon la revendication 1, **caractérisés en ce que** X⁴, X⁶ et X⁷ sont identiques ou différents et représentent, indépendamment les uns des autres, un atome d'hydrogène ou un atome d'halogène.

10. Composés de formule générale 1 selon la revendication 1, **caractérisés en ce que** Y⁴ représente un groupe alkyle en C₁-C₅ ou un groupe perfluoroalkyle en C₁-C₅.

11. Composés de formule générale 1 selon la revendication 1 **caractérisés en ce que** Y⁵, Y⁷ et Y⁸ sont identiques ou différents et représentent, indépendamment les uns des autres, un atome d'hydrogène ou un atome d'halogène.

12. Composés de formule générale 1 selon la revendication 1 **caractérisés en ce que** R¹ et R² sont identiques ou différents et représentent un atome d'hydrogène, un groupe méthyle ou éthyle et en outre un noyau cyclopropyle conjointement avec l'atome de carbone de la chaîne et R³ représente un groupe perfluoroalkyle en C₁-C₅, A représente un noyau benzène, naphtalène ou thiophène éventuellement substitué par un ou plusieurs radicaux choisis parmi des atomes de fluor, des atomes de chlore, des atomes de brome, des groupes méthyle, des groupes éthyle, des groupes vinyle, des groupes hydroxy, des groupes méthoxy, des groupes éthoxy, et soit X^{3a} représente un atome d'hydrogène, un groupe alkyle en C₁-C₅, soit X^{3a} et X^{3b} sont identiques ou différents et représentent un atome d'hydrogène ou un groupe alkyle en C₁-C₅, et X⁴, X⁶ et X⁷ sont identiques ou différents et représentent, indépendamment les uns des autres, un atome d'hydrogène ou un atome d'halogène, ou Y⁴ représente un groupe alkyle en C₁-C₅ ou un groupe perfluoroalkyle en C₁-C₅ et Y⁵, Y⁷ et Y⁸ sont identiques ou différents et représentent, indépendamment les uns des autres, un atome d'hydrogène ou un atome d'halogène, et les autres substituants présentent toutes les significations indiquées dans la formule 1.

13. Composés de formule générale 1 selon la revendication 1 dans laquelle Ar représente un système cyclique de formule partielle 6.

14. Composés de formule générale 1 selon la revendication 1 dans laquelle Ar représente un système cyclique de formule partielle 7.

15. Composés de formule générale 1 selon la revendication 1 dans laquelle Ar représente un système cyclique de formule partielle 10.

16. Composés de formule générale 1 selon la revendication 1 dans laquelle Ar représente un système cyclique de formule partielle 11.

17. Composés de formule générale 1 selon la revendication 1 à savoir
le 4-bromo-5-(2-hydroxy-4-méthyl-4-phényl-2-trifluorométhyl-valéroylamino)-phtalide,
le 6-bromo-5-(2-hydroxy-4-méthyl-4-phényl-2-trifluorométhyl-valéroylamino)-phtalide,
le 5-(2-hydroxy-4-méthyl-2-pentafluoroéthyl-4-phényl-valéroylamino)-phtalide,
le 5-[2-hydroxy-4-(3-méthoxyphényl)-4-méthyl-2-trifluorométhyl-valéroylamino]-phtalide,
le 5-[2-hydroxy-4-(4-méthoxyphényl)-4-méthyl-2-trifluorométhyl-valéroylamino]-phtalide,
le 5-[2-hydroxy-4-(2-hydroxyphényl)-4-méthyl-2-trifluorométhyl-valéroylamino]-phtalide,
le 5-[4-(2-fluorophényl)-2-hydroxy-4-méthyl-2-trifluorométhyl-valéroylamino]-phtalide,
le 5-[4-(4-fluorophényl)-2-hydroxy-4-méthyl-2-trifluorométhyl-valéroylamino]-phtalide,
le 5-[4-(4-chlorophényl)-2-hydroxy-4-méthyl-2-trifluorométhyl-valéroylamino]-phtalide,
le 5-[4-(4-bromophényl)-2-hydroxy-4-méthyl-2-trifluorométhyl-valéroylamino]-phtalide,
le 5-[2-hydroxy-4-méthyl-4-(4-tolyl)-2-trifluorométhyl-valéroylamino]-phtalide,
le 5-[2-hydroxy-4-méthyl-4-(3-tolyl)-2-trifluorométhyl-valéroylamino]-phtalide,
le 5-[4-(4-cyanophényl)-2-hydroxy-4-méthyl-2-trifluorométhyl-valéroylamino]-phtalide,
le 5-[4-(3,4-diméthylphényl)-2-hydroxy-4-méthyl-2-trifluorométhyl-valéroylamino]-phtalide,
le 5-[4-(3,5-diméthylphényl)-2-hydroxy-4-méthyl-2-trifluorométhyl-valéroylamino]-phtalide,
le 5-[2-hydroxy-4-(2-méthoxy-5-méthylyphényl)-4-méthyl-2-trifluorométhyl-valéroylamino]-phtalide,
le 5-[4-(5-chloro-2-hydroxyphényl)-2-hydroxy-4-méthyl-2-trifluorométhyl-valéroylamino]-phtalide,
le 5-[4-(5-fluoro-2-hydroxyphényl)-2-hydroxy-4-méthyl-2-trifluorométhyl-valéroylamino]-phtalide,
le 5-[2-hydroxy-4-(2-hydroxy-5-méthylphényl)-4-méthyl-2-trifluorométhyl-valéroylamino]-phtalide,
le 5-[4-(5-fluoro-2-méthoxyphényl)-2-hydroxy-4-méthyl-2-trifluorométhyl-valéroylamino]-phtalide,
le 5-[4-(2-fluoro-4-méthoxyphényl)-2-hydroxy-4-méthyl-2-trifluorométhyl-valéroylamino]-phtalide,
le 5-[4-(3-fluoro-4-méthoxyphényl)-2-hydroxy-4-méthyl-2-trifluorométhyl-valéroylamino]-phtalide,
le 5-(2-hydroxy-4-phényl)-2-trifluorométhylvaléroylamino)-phtalide,
le 5-[2-hydroxy-4-(2-méthoxyphényl)-4-méthyl-2-trifluorométhyl-valéroylamino]-phtalide,
le 5-[4-(5-chloro-2-méthoxyphényl)-2-hydroxy-4-méthyl-2-trifluorométhyl-valéroylamino]-phtalide,
le 5-(2-hydroxy-4-méthyl-4-phényl-2-trifluorométhyl-pentylamino)-phtalide,
le 5-[4-(4-fluorophényl)-2-hydroxy-4-méthyl-2-trifluorométhyl-pentylamino]-phtalide,
le 5-[4-(5-fluoro-2-hydroxyphényl)-2-hydroxy-4-méthyl-2-trifluorométhyl-pentylamino]-phtalide,
le 6-acétyl-5-(2-hydroxy-4-méthyl-4-phényl-2-trifluorométhyl-valéroylamino)-phtalide,
le 5-[4-(3-fluoro-4-hydroxyphényl)-2-hydroxy-4-méthyl-2-trifluorométhyl-valéroylamino]-phtalide,
le 5-[4-(3-fluorophényl)-2-hydroxy-4-méthyl-2-trifluorométhyl-valéroylamino]-phtalide,
le 6-(3-hydroxy-3-méthyl-1-butinyl)-5(2-hydroxy-4-méthyl-4-phényl-2-trifluorométhylvaléroylamino)phtalide,
la 6-(2-hydroxy-4-méthyl-4-phényl-2-trifluorométhyl-valéroylamino)-4-méthyl-2,3-benzoxazin-1-one,
la 6-(2-hydroxy-4-méthyl-4-phényl-2-trifluorométhyl-valéroylamino)-4-trifluorométhyl-2,3-benzoxazin-1-one,
la 4-éthyl-6-(2-hydroxy-4-phényl-2-trifluorométhyl-pentylamino)-2,3-benzoxazin-1-one,
la 4-éthyl-6-[2-hydroxy-4-(2-méthoxyphényl)-4-méthyl-2-trifluorométhyl-valéroylamino]-2,3-benzoxazin-1-one,
la 6-[2-hydroxy-4-(2-méthoxyphényl)-4-méthyl-2-trifluorométhyl-valéroylamino]-4-méthyl-2,3-benzoxazin-1-one,
la 4-éthyl-6-[2-hydroxy-4-méthyl-4-(4-méthylphényl)-2-trifluorométhyl-valéroylamino]-2,3-benzoxazin-1-one,
la 6-[4-(4-bromophényl)-2-hydroxy-4-méthyl-2-trifluorométhyl-valéroylamino]-4-éthyl-2,3-benzoxazin-1-one,
la 4-éthyl-6-[4-(5-fluoro-2-méthoxyphényl)-2-hydroxy-4-méthyl-2-trifluorométhyl-valéroylamino]-2,3-benzoxazin-1-one,
la 6-[4-(5-fluoro-2-méthoxyphényl)-2-hydroxy-4-méthyl-2-trifluorométhyl-valéroylamino]-4-méthyl-2,3-benzoxazin-1-one,
le 1-(4-nitro-3-trifluorométhylanilino)-4-phényl-2-trifluorométhyl-2-pentanol,
le 1-(4-nitro-3-trifluorométhylanilino)-4-phényl-2-trifluorométhyl-2-pentanol,
le 5-(2-hydroxy-4,4-diméthyl-2-trifluorométhyl-5-hexénoylamino)-phtalide
le 5-[2-hydroxy-3-(1-phényl-cyclopropyl)-2-trifluorométhyl-propionylamino]phtalide,
le 5-[2-hydroxy-3-(1-phényl-cyclopropyl)-2-trifluorométhyl-propionyl-amino]-phtalide,
le 5-[2-hydroxy-3-(1-phényl-cyclobutyl)-2-trifluorométhyl-propionylamino]phtalide,
le 5-[2-hydroxy-3-(1-phényl-cyclohexyl)-2-trifluorométhyl-propionylamino]phtalide,
la 6-(2-hydroxy-2,4-diméthyl-4-phényl-valéroylamino)-4-méthyl-2,3-benzoxazin-1-one,
le 5-[4-(3-chloro-4-méthoxyphényl)-2-hydroxy-4-méthyl-2-trifluorométhyl-valéroylamino]-phtalide,

18. Composés de formule générale 1 selon la revendication 1, à savoir les composés 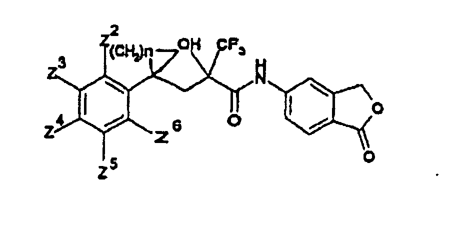
| **n** | **Z**^{**n**} **(≠H)** | **Isomérie** |
|---|---|---|
| 1 | | Racémate |
| 1 | | -Enantiomère(+) |
| 1 | | -Enantiomére(-) |
| 1 | 3-F | Racémate |
| 1 | 2-Cl | Racémate |
| 1 | 4-Cl | Racémate |
| 1 | 4-Cl | -Enantiomère(+) |
| 1 | 4-Cl | -Enantiomère(-) |
| 1 | 2-Br | Racémate |
| 1 | 3-Br | Racémate |
| 1 | 2,4-Cl₂ | Racémate |
| 1 | 2-OCH₃ | -Enantiomère(+) |
| 1 | 2-OCH₃ | -Enantiomère(-) |
| 1 | 3-OCH₃ | Racémate |
| 1 | 3-CF₃ | Racémate |
| 2 | | Racémate |
| 2 | | -Enantiomère(+) |
| 2 | | -Enantiomère(-) |
| 3 | | -Enantiomère(+) |
| 3 | 4-CH₃ | Racémate |
| 4 | | Racémate |
| 4 | | -Enantiomère(+) |
| 4 | | -Enantiomère(-) |

19. Composés de formule générale selon la revendication 1, à savoir les composés 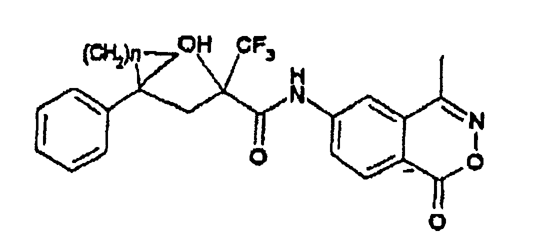
| **n** | **Isomérie** |
|---|---|
| 1 | Racémate |
| 1 | -Enantiomère(+) |
| | -Enantiomère(-) |
| 2 | Racémate |
| 4 | Racémate |
| 4 | -Enantiomère(+) |
| 4 | -Enantiomère(-) |

20. Composés de formule générale 1 selon la revendication 1, à savoir les composés 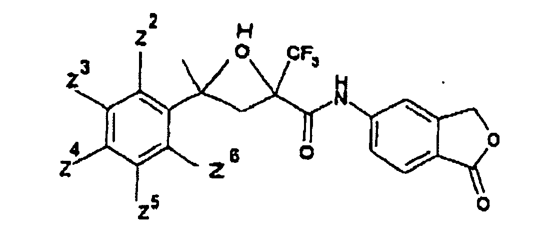
| **Z**ⁿ **(≠H)** |
|---|
| Z² =I |
| Z³ = Cl |
| Z³ =Br |
| Z³=I |

21. Composés de formule générale 1 selon la revendication 1, à savoir les composés 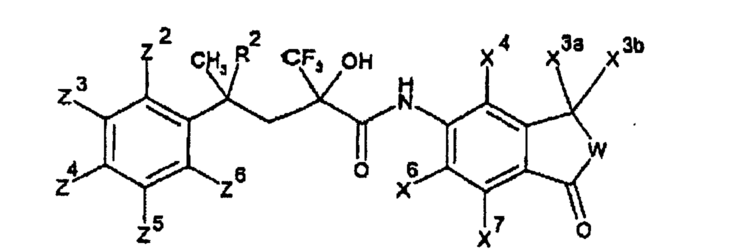
| **R**^{**2**} | **W** | **X**^{**n**} **(≠H)** | **Z**^{**n**} **(≠H)** | **Isomérie** |
|---|---|---|---|---|
| H | O | X^{3a}/X^{3b}= H/CH₃ | | mélange de Diast |
| H | O | X^{3a}= H, X^{3b}=CH₃ | | -Forme (+) |
| H | O | X^{3a}= H, X^{3b}= CH₃ | | -Forme(-) |
| H | O | X^{3a}=CH₃,X^{3b}=H | | -Forme(+) |
| H | O | X^{3a}= CH₃, X^{3b}=H | | -Forme(-) |
| H | O | X^{3a}=C₂H₅ | | |
| H | O | X^{3a}=CH=CH₂ | | |
| H | O | X^{3a}= CH=CH₂-CH₃ | | |
| | | | | |
|---|---|---|---|---|
| H | O | X^{3a}= CF₃ | | |
| H | O | X^{3a}=X^{3b}= ^{CH}₃ | | |
| H | O | X^{3a}= X^{3b}= C₂H₅ | | |
| H | O | X^{3a}+X^{3b}=(CH₂)⁴ | | |
| H | O | X⁴ = Br | | |
| CH₃ | O | | | |
| CH₃ | O | X⁴ = Br | | |
| CH₃ | O | | Z² CH₃ | Racémate |
| CH₃ | O | | Z²=CH₃ | -Forme(+) |
| CH₃ | O | | Z²= CH₃ | -Forme(-) |
| CH₃ | O | | Z⁴=CH₃ | |
| **R**^{**2**} | **W** | **X**^{**n**} **(≠H)** | **Z**^{**n**} **(≠H)** | **Isomérie** |
|---|---|---|---|---|
| CH₃ | O | | Z³=Z⁴=CH₃ | Racémate |
| CH₃ | O | | Z³= Z⁴ =CH₃ | -Form (+) |
| CH₃ | O | | Z³= Z⁴= CH₃ | -Form(-) |
| CH₃ | O | | Z³= Z⁵= CH₃ | Racémate |
| CH₃ | O | | Z³= Z⁵=CH₃ | -Form(+) |
| CH₃ | O | | Z³=Z⁵=CH₃ | -Form(-) |
| CH₃ | O | | Z³/Z⁴ = (CH₂)₃ | |
| CH₃ | O | | Z³/Z⁴ =-CH=CH-CH=CH- | |
| CH₃ | O | | Z⁴=F | |
| CH₃ | O | | Z⁴=Cl | |
| CH₃ | O | | Z⁴=Br | |
| CH₃ | O | | Z²=OCH₃ | Racémate |
| | | | | |
|---|---|---|---|---|
| CH₃ | O | | Z⁴- OCH₃ | |
| CH₃ | O | | Z²=Z⁵= OCH₃ | |
| CH₃ | O | | Z²= OCH₃, Z⁵ =CH₃ | Racémate |
| CH₃ | O | | Z²= OCH₃, Z⁵=CH₃ | -forme(+) |
| CH₃ | O | | Z²=OCH₃, Z⁵=CH₃ | -Forme(-) |
| CH₃ | O | | Z²= OCH₃, Z⁴=F | |
| CH₃ | O | | Z² =OCH₃, Z⁵= F | |
| CH₃ | O | | Z⁴=OCH₃,Z²= F | |
| CH₃ | O | | Z⁴= OCH₃, Z³= F | |
| CH₃ | O | | Z²= OCH₃, Z⁵= Cl | Racémate |
| **R**^{**2**} | **W** | **X**^{**n**} **(≠H)** | **Z**^{**n**} **(≠H)** | **Isomérie** |
|---|---|---|---|---|
| CH₃ | O | | Z²= OCH₃, Z⁵= Cl | -Forme(-) |
| H | S | | | |
| CH₃ | S | | | |
| H | CH₂ | | | |
| H | O-CH₂ (3) | | | |
| **R**^{**2**} | **W** | **X**^{**n**}**(≠H)** | **Z**^{**n**} **(≠H)** | **Isomérie** |
|---|---|---|---|---|
| CH₃ | O | | Z⁴ = CH=CH₂ | Racémate |
| CH₃ | O | | Z⁴=CN | Racémate |
| CH₃ | O | | Z⁴=COCH₃ | Racémate |
| CH₃ | O | | Z⁴=CONH₂ | Racémate |
| CH₃ | O | | Z² = OCH₃, Z⁴ = Br | Racémate |
| | | | | |
|---|---|---|---|---|
| CH₃ | O | | Z² = OCH₃, Z⁴ =Br | -Enantiomère(+) |
| CH₃ | O | | Z² = OCH₃, Z⁴ = Br | -Forme(-) |
| CH₃ | O | | Z² - Br, Z⁴ = OCH₃ | Racémate |
| CH₃ | O | | Z² = OCH₃, Z⁴ = CN | Racémate |
| CH₃ | O | | Z³ = NO₂, Z⁴ *=* OCH₃ | Racémate |
| CH₃ | O | | Z² = COCH₃, Z⁴ = CH(CH₃)₂ | Racémate |
| CH₃ | O | | Z³ = COCH₃, Z⁴ = OCH₃ | Racémate |

22. Composés de formule générale 1 selon la revendication 1, à savoir les composés 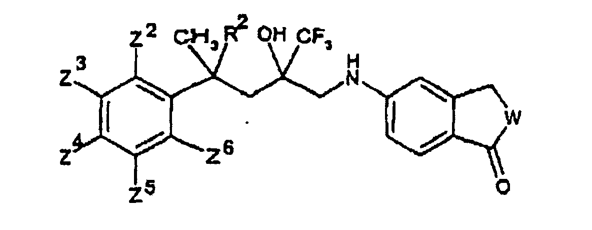
| **R**^{**2**} | **W** | **Z**^{**n**} **(≠H)** | **Isomérie** |
|---|---|---|---|
| H | O | | Racémate |
| H | O | | -Forme(+) |
| H | O | | -Forme(+) |
| H | O | | -Forme(-) |
| CH₃ | O | | Racémate |
| CH₃ | O | | -Forme(+) |
| CH₃ | O | | -Forme(-) |
| CH₃ | O | Z⁴= F | Racémate |
| CH₃ | O | Z⁴=F | -Forme(+) |
| CH₃ | O | Z⁴= F | -Forme(-) |
| | | | |
|---|---|---|---|
| CH₃ | O | Z²= OCH₃, Z⁵= F | |
| H | CH₂ | | |
| H | OCH₂ (3) | | |

23. Composés de formule générale 1 selon la revendication 1, à savoir les composés

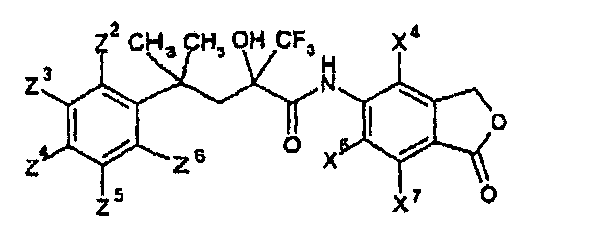

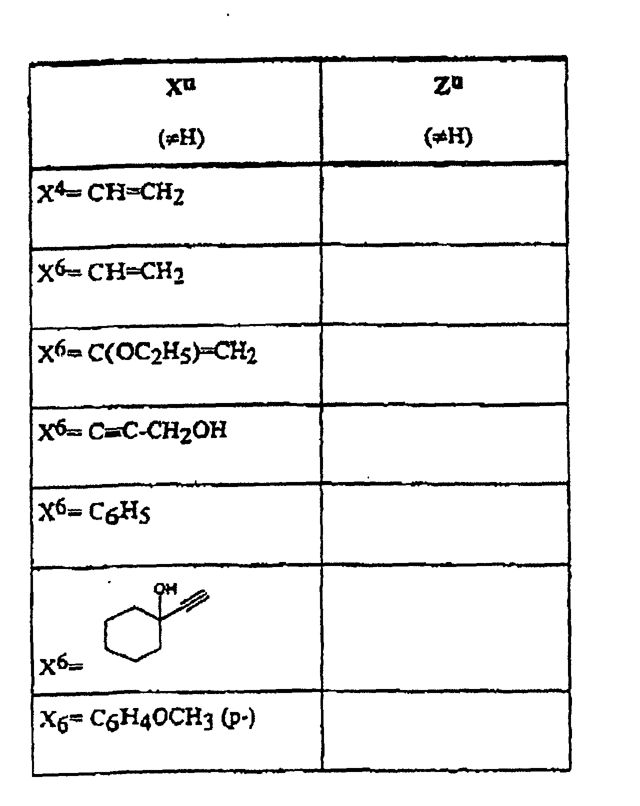

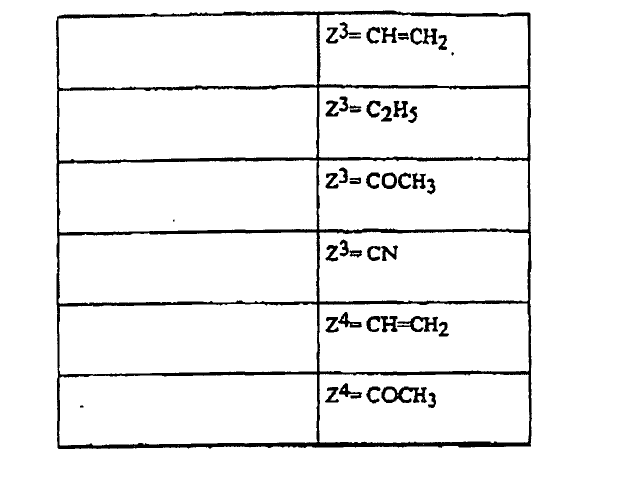

24. Composés de formule générale 1 selon la revendication 1, à savoir les composés 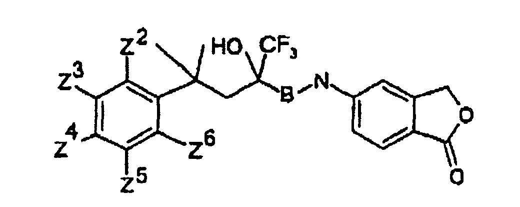
| **B** | **Z**^{**n**}(≠H) | **Isomérie** |
|---|---|---|
| C=O | Z²= OH | |
| C=O | Z⁴= OH | |
| C=O | Z²= Z⁵= OH | |
| C=O | Z²= OH, Z⁵= CH₃ | Racémate |
| C =O | Z²= OH, Z⁵= CH₃ | -Forme(+) |
| C=O | Z²= OH,Z⁵= CH₃ | -Forme(-) |
| C=O | Z²=OH,Z⁴=F | |
| C=O | Z²= OH, Z⁵= F | |
| C=O | Z⁴=OH, Z²=F | |
| C=O | Z²=OH, Z⁵=Cl | |
| CH₂ | Z²=OH, Z⁵=F | Racémate |
| CH₂ | Z²= OH, Z⁵= F | -Forme(+) |
| CH₂ | Z²= OH, Z⁵= F | -Forme(-) |
| C=O | Z² =OH, Z⁴ = Br | Racémate |
| C=O | Z³ =NO₂, Z⁴= OH | Racémate |
| C=O | Z³=Cl, Z⁴=OH | Racémate |
| C=O | Z³ = Br, Z⁴= OH | Racémate |

25. Composés de formule générale 1 selon la revendication 1, à savoir les composés 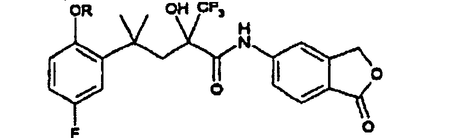
| **R** | **Isomérie** |
|---|---|
| CH(CH₃)₂ | Racémate |
| CH₂CH=CH₂ | Racémate |
| CH₂CH=CH₂ | Racémate |
| CH₂CN | Racémate |
| CH₂COOC(CH₃)₃ | Racémate |
| CH₂COOC(CH₃)₃ | -Forme(-) |
| CH₂COOC(CH₃)₃ | -Forme(+) |

26. Composés de formule générale 1 selon la revendication 1, à savoir les composés 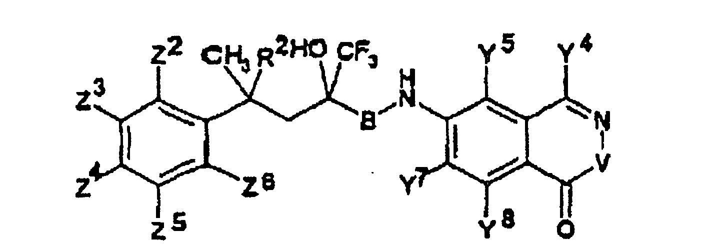
| **R**^{**2**} | **V** | **Z**^{**n**} **(≠H)** | **B** | **Y**^{**n**} **(≠H)** | **Isomérie** |
|---|---|---|---|---|---|
| H | O | | C=O | Y⁴=CH₃ | Racémate |
| H | O | | C=O | Y⁴= C₂H₅ | Racémate |
| CH₃ | O | | C=O | Y⁴ =CH₃ | -Forme(+) |
| CH₃ | O | | C=O | Y⁴=CH₃ | -Forme(-) |
| CH₃ | O | | C=O | Y⁴= C₂H₅ | Racémate |
| CH₃ | O | | C=O | Y⁴=C₂H₅ | -Forme(+) |
| CH₃ | O | | C=O | Y⁴-C₂H₅ | -Forme(-) |
| **R**^{**2**} | **V** | **Z**^{**n**} **(≠H)** | **B** | **Y**^{**n**} **Isomérie (≠H)** | |
|---|---|---|---|---|---|
| CH₃ | O | Z²⁼ OCH₃ | C=O | Y⁴= CH₃ | Racémate |
| CH₃ | O | Z²= OCH₃ | C=O | Y⁴= CH₃ | (-)-Form |
| CH₃ | O | Z²= OCH₃ | C=O | Y⁴= CH₃ | (+)-Form |
| CH₃ | O | Z²= OCH₃ | C=O | Y⁴= C₂H₅ | Racémate |
| CH₃ | O | Z²= OCH₃ | C=O | Y⁴= C₂H₅ | (+)-Form |
| CH₃ | O | Z²= OCH₃ | C=O | Y⁴= C₂H₅ | (-)-Form |
| CH₃ | O | Z²= OCH₃, Z⁵= F | C=O | Y⁴=CH₃ | -Forme(-) |
| CH₃ | O | Z²= OCH₃, Z⁵⁼ F | C=O | Y⁴= C₂H₅ | Racémate |
| CH₃ | O | Z²= OCH₃, Z⁵= F | C=O | Y⁴=C₂H₅ | -Forme(-) |
| CH₃ | O | Z²= OCH₃, Z⁵= F | C=O | Y⁴= C₂H₅ | -Forme(+) |
| CH₃ | O | Z²= OCH₃, Z⁵ =Cl | C=O | Y⁴= CH₃ | Racémate |
| CH₃ | O | Z²= OCH₃, Z⁵=Cl | C=O | Y⁴=CH₃ | -Forme(+) |
| CH₃ | O | Z²= OCH₃, Z⁵=Cl | C=O | Y⁴= CH₃ | -Forme(-) |
| CH₃ | O | Z²= OCH₃, Z⁴= Br | C=O | Y⁴= CH₃ | Racémate |
| CH₃ | O | Z²= OCH₃, Z⁴= Br | C=O | Y⁴= CH₃ | -Forme(+) |
| CH₃ | O | Z²= OCH₃, Z⁴= Br | C=O | Y⁴= CH₃ | -Forme(-) |
| CH₃ | O | Z⁴=CH₃ | C=O | Y⁴=CH₃ | Racémate |
| CH₃ | O | Z⁴= CH₃ | C=O | Y⁴= C₂H⁵ | Racémate |
| CH₃ | O | Z⁴= CH₃ | C=O | Y⁴= C₂H₅ | -Forme(-) |
| CH₃ | O | Z⁴= CH₃ | C=O | Y⁴= C₂H₅ | -Forme(+) |
| CH₃ | O | Z⁴= F | C=O | Y⁴=CH₃ | Racémate |
| CH₃ | O | Z⁴= Br | C=O | Y⁴= CH₃ | Racémate |
| CH₃ | O | Z⁴= Br | C=O | Y⁴= CH₃ | -Forme(-) |
| CH₃ | O | Z⁴= Br | C=O | Y⁴= CH₃ | -Forme(+) |
| CH₃ | O | | C=O | Y⁴= CF₃ | |
| CH₃ | NH | | C=O | Y⁴ CH3 | |
| CH₃ | NCH₃ | | C=O | Y⁴= CH₃ | |
| CH₃ | O | Z²= OH, Z⁵ =F | C=O | Y⁴= CH₃ | Racémate |
| CH₃ | O | Z² = OH, Z⁵ = F | C=O | Y⁴= CH₃ | -Forme(+) |
| CH₃ | O | Z²=OH,Z⁵=F | C=O | Y⁴=CH₃ | -Forme(-) |
| CH₃ | O | Z² = OH, Z⁴ = Br | C=O | Y⁴= CH₃ | Racémate |
| CH₃ | O | Z³=NO₂, Z⁴= OCH₃ | C=O | Y⁴= CH₃ | Racémate |
| H | 0 | | CH₂ | Y⁴= CH₃ | Racémate |
| CH₃ | O | | CH₂ | Y⁴=CH₃ | Racémate |
| CH₃ O | | | CH₂ | Y⁴=C₂H₃ | Racémate |

27. Préparation pharmaceutique comprenant au moins un composé de formule générale 1 selon la revendication 1 et un support pharmaceutiquement acceptable.

28. Utilisation des composés de formule générale 1 selon la revendication 1 pour la production de préparations pharmaceutiques.

29. Procédé de préparation de composés de formule générale 1 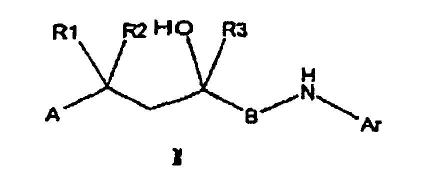 dans laquelle A, B, Ar, R¹, R² et R³ présentent la signification indiquée dans la revendication 1, **caractérisé en ce qu'**un composé carbonyle de formule générale 2 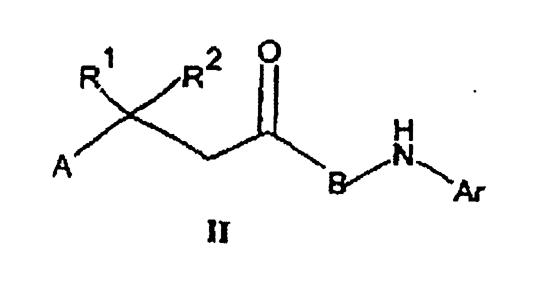 dans laquelle A, B, Ar, R¹ et R² présentent la signification indiquée dans la formule 1, est mis à réagir avec un composé de formule générale R³-SiMe₃, dans laquelle R³ présente la signification indiquée dans la formule générale 1, en présence d'un catalyseur, ou avec un composé alkyle-métal, par exemple un réactif de Grignard ou un lithium-alkyle, pour donner lieu à un composé de formule générale 1.

30. Procédé selon la revendication 29, **caractérisé en ce que** le catalyseur est un sel de fluorure ou un carbonate de métal alcalin.

31. Procédé de préparation de composés de formule générale 1 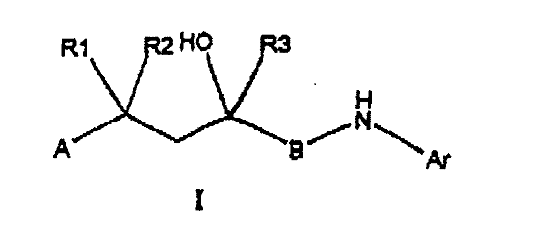 dans laquelle A, B, Ar, R¹, R² et R³ présentent la signification indiquée dans la revendication 1, **caractérisé en ce qu'**un composé de formule générale 3 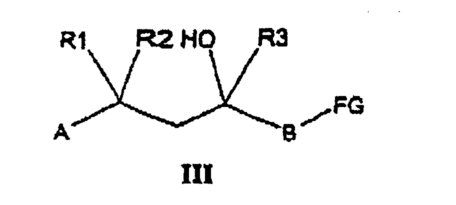 dans laquelle A, B, R¹, R² et R³ présentent la signification indiquée dans la formule générale 1 et FG représente un groupe partant, est mis à réagir avec un composé de formule Ar-NH-R¹¹ dans laquelle R¹¹ représente un atome d'hydrogène ou un groupe alcanoyle en C₁-C₅, et Ar présente la signification indiquée dans la formule générale 1, et le radical R¹¹ est ensuite éventuellement éliminé.

32. Procédé selon la revendication 31, **caractérisé en ce que** le groupe partant FG dans le composé de formule générale 3 représente un atome de chlore, de brome ou d'iode, un radical tosilate ou mésilate ou un radical C₁-C₄-perfluoroalkylsulfonyloxy.

33. Procédé selon la revendication 32, **caractérisé en ce que** le composé de formule générale 3 est un chlorure d'acide formé de façon intermédiaire à partir de l'acide carboxylique correspondant.

34. Procédé de préparation de composés de formule générale 1 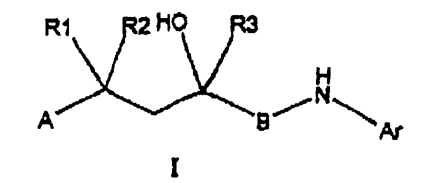 dans laquelle
A, Ar, R¹, R² et R³ présentent la signification indiquée dans la revendication 1 et B représente un groupe -CH₂- **caractérisé en ce qu'**un composé de formule générale 4 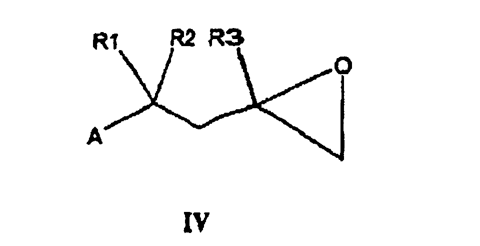 dans laquelle A, R¹, R² et R³ présentent la signification indiquée dans la formule 1, est mis à réagir avec un composé de formule Ar-NH-R¹¹, dans laquelle R¹¹ et Ar présentent la signification indiquée dans la revendication 23, et le radical R¹¹ est ensuite éventuellement éliminé.
